# EUROPEAN PATENT SPECIFICATION

(11) **EP 3 280 420 B1**
(45) Date of publication and mention of the grant of the patent: **27.03.2024**
(21) Application number: 16777382.9
(22) Date of filing: 08.04.2016
(51) Int. Cl.: A61K 31/57, A61K 45/06, A61P 25/00, C07J 7/00, A61P 25/24

(54) **COMPOSITIONS AND METHODS FOR TREATING CNS DISORDERS**
ZUSAMMENSETZUNGEN UND VERFAHREN ZUR BEHANDLUNG VON STÖRUNGEN DES ZENTRALEN NERVENSYSTEMS
COMPOSITIONS ET MÉTHODES PERMETTANT DE TRAITER DES TROUBLES DU SNC

(30) Priority: 10.04.2015 US 201562146034 P; 24.07.2015 US 201562196591 P; 20.01.2016 US 201662280825 P
(43) Date of publication of application: 14.02.2018
(73) Proprietor: Sage Therapeutics, Inc., Cambridge, MA 02142 (US)
(72) Inventor: ROBICHAUD, Albert, Jean, Cambridge, MA 02141 (US); SALITURO, Francesco, G., Marlborough, MA 01752 (US); MARTINEZ BOTELLA, Gabriel, Wayland, MA 01778 (US); HARRISON, Boyd, L., Princeton Junction, NJ 08550 (US); JONAS, Jeffrey, Cambridge, MA (US)
(74) Representative: Coles, Andrea Birgit
(86) International application number: PCT/US2016/026705
(87) International publication number: WO 2016/164763

(56) References cited:
- WO-A1-2013/056181
- WO-A1-2014/085668
- WO-A1-2015/134670
- WO-A1-2016/040322
- WO-A2-2013/112605
- WO-A2-2015/010054
- US-A- 4 061 661
- J Vine ET AL: "2H-labelled 3[alpha]-hydroxy-5[alpha]-pregnane-11,20-d ione and 3[alpha],21-dihydroxy-5[alpha]-pregnane-11 ,20-dione 21-acetate", Journal of Labelled Compounds and Radiopharmaceuticals, 1 April 1982 (1982-04-01), pages 597-604, XP055516145, DOI: 10.1002/jlcr.2580190414 Retrieved from the Internet: URL:https://onlinelibrary.wiley.com/doi/pd f/10.1002/jlcr.2580190414
- JOHNSON D W ET AL: "Deuterium labelled steroid hormones: Syntheses and applications in quantitation and endocrinology", JOURNAL OF STEROID BIOCHEMISTRY, PERGAMON PRESS PLC, GB, vol. 14, no. 8, 1 August 1981 (1981-08-01) , pages 793-800, XP023415677, ISSN: 0022-4731, DOI: 10.1016/0022-4731(81)90017-0 [retrieved on 1981-08-01]
- MAREK ET AL.: 'A stereospecific pathway for the introduction of deuterium on the brassinosteroid skeleton by reductive dechlorination of chlorocarbonates.' TETRAHEDRON LETTERS vol. 53, no. 16, 03 February 2012, pages 2048 - 2050, XP028468897

## Description

### Background of the Invention

Brain excitability is defined as the level of arousal of an animal, a continuum that ranges from coma to convulsions, and is regulated by various neurotransmitters. In general, neurotransmitters are responsible for regulating the conductance of ions across neuronal membranes. At rest, the neuronal membrane possesses a potential (or membrane voltage) of approximately -70 mV, the cell interior being negative with respect to the cell exterior. The potential (voltage) is the result of ion (K⁺, Na⁺, Cl⁻, organic anions) balance across the neuronal semipermeable membrane. Neurotransmitters are stored in presynaptic vesicles and are released under the influence of neuronal action potentials. When released into the synaptic cleft, an S2170a change of potential from -70 mV to -50 mV. This effect is mediated by postsynaptic nicotinic receptors which are stimulated by acetylcholine to increase membrane permeability to Na⁺ ions. The reduced membrane potential stimulates neuronal excitability in the form of a postsynaptic action potential.

In the case of the GABA receptor complex (GRC), the effect on brain excitability is mediated by γ-aminobutyric acid (GABA), a neurotransmitter. GABA has a profound influence on overall brain excitability because up to 40% of the neurons in the brain utilize GABA as a neurotransmitter. GABA regulates the excitability of individual neurons by regulating the conductance of chloride ions across the neuronal membrane. GABA interacts with its recognition site on the GRC to facilitate the flow of chloride ions down an electrochemical gradient of the GRC into the cell. An intracellular increase in the levels of this anion causes hyperpolarization of the transmembrane potential, rendering the neuron less susceptible to excitatory inputs, *i.e.*, reduced neuron excitability. In other words, the higher the chloride ion concentration in the neuron, the lower the brain excitability and level of arousal.

It is well-documented that the GRC is responsible for the mediation of anxiety, seizure activity, and sedation. Thus, GABA and drugs that act like GABA or facilitate the effects of GABA (e.g., the therapeutically useful barbiturates and benzodiazepines (BZs), such as Valium^{®}) produce their therapeutically useful effects by interacting with specific regulatory sites on the GRC. Accumulated evidence has now indicated that in addition to the benzodiazepine and barbiturate binding site, the GRC contains a distinct site for neuroactive steroids. See, *e.g.,* Lan, N. C. et al., Neurochem. Res. (1991) 16:347-356.

Neuroactive steroids can occur endogenously. The most potent endogenous neuroactive steroids are 3α-hydroxy-5-reduced pregnan-20-one and 3α-21-dihydroxy-5-reduced pregnan-20-one, metabolites of hormonal steroids progesterone and deoxycorticosterone, respectively. The ability of these steroid metabolites to alter brain excitability was recognized in 1986 (Majewska, M. D. et al., Science 232:1004-1007 (1986); Harrison, N. L. et al., J Pharmacol. Exp. Ther. 241:346-353 (1987)).
J. Vine et al., Journal of Labelled Compounds and Radiopharmaceuticals, 1 April 1982, pages 597-604 discloses 2H-labelled 3[alpha]-hydroxy-5[alpha]-pregnane-11,20-dione and 3[alpha],21-dihydroxy-5 [alpha]-pregnane-11,20-dione 21-acetate.
WO 2014/085668 discloses anticonvulsant activity of steroids.
WO 2013/112605 discloses neuroactive steroid formulations and methods of treating CNS disorders.
D. W. Johnson et al., Journal of Steroid Biochemistry, 1 August 1981, pages 793-800 discloses deuterium labelled steroid hormones, their synthesis and applications in quantitation and endocrinology.
WO 2016/040322 discloses neuroactive steroids, and compositions thereof, for use in methods of treating tremor.

New and improved neuroactive steroids are needed that act as modulating agents for brain excitability, as well as agents for the prevention and treatment of CNS-related diseases. The compounds, compositions, and methods described herein are directed toward this end.

### Summary of the Invention

Provided herein are deuterium-enriched neuroactive steroids designed, for example, to act as GABA modulators. The deuterium-enriched compounds described herein comprise compounds that have deuterium levels that are above the naturally occurring levels.

In some embodiments, such compounds are envisioned to be useful as therapeutic agents for treating a CNS-related disorder (*e.g.,* sleep disorder, a mood disorder (e.g., depression, such as post-partum depression (PPD)), a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, or tinnitus) in a subject in need (*e.g.,* a subject with Rett syndrome, Fragile X syndrome, or Angelman syndrome). In certain embodiments, such compounds are envisioned to be useful as therapeutic agents for the inducement of anesthesia and/or sedation in a subject.

In one aspect, provided is a deuterium-enriched compound or a pharmaceutically acceptable salt thereof, for use in a method of treating a mood disorder (e.g., depression, for example post-partum depression; or anxiety disorder) in a subject, comprising administering to the subject an effective amount of the deuterium-enriched compound selected from the group consisting of: 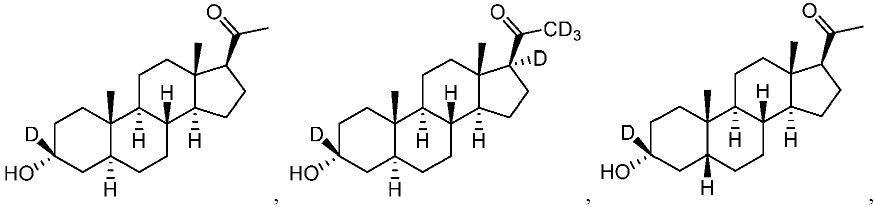 and 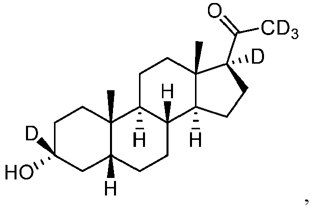 or a pharmaceutically acceptable salt thereof.

In some embodiments, the mood disorder is depression. In some embodiments, the depression is is postpartum depression.

In some embodiments, the administering is performed orally.

In some embodiments, the administering is performed parenterally. In some embodiments, the administering is performed intravenously. In some embodiments, the administering occurs by continuous intravenous infusion.

In some embodiments, the subject is a mammal. In some embodiments, the subject is a human. In some embodiments, the subject is a female. In some embodiments, the subject is an adult. In some embodiments, the subject is from 18 to 45 years of age.

In some embodiments, the subject is suffering from (*e.g.,* has been diagnosed with) postpartum depression (*e.g.,* severe postpartum depression). In some embodiments, the subject has experienced a Major Depressive Episode in the postpartum period. In some embodiments, the period begins within the first 4 weeks following delivery of a baby.

In one aspect, provided is a compound or a pharmaceutically acceptable salt thereof, for use in a method of inducing sedation and/or anesthesia in a subject, comprising administering to the subject an effective amount of the compound selected from the group consisting of: 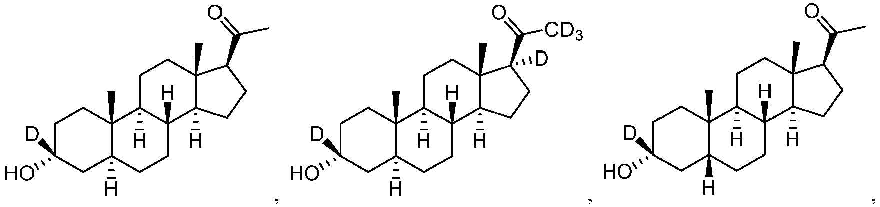 and 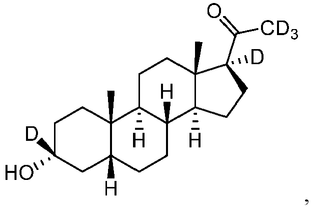 or a pharmaceutically acceptable salt thereof.

In one aspect, provided is a compound or a pharmaceutically acceptable salt thereof, for use in a method of administering to a subject in need thereof an effective amount of the compound, the pharmaceutically acceptable salt thereof, or pharmaceutical composition of the compound or the pharmaceutically acceptable salt thereof, selected from the group consisting of: 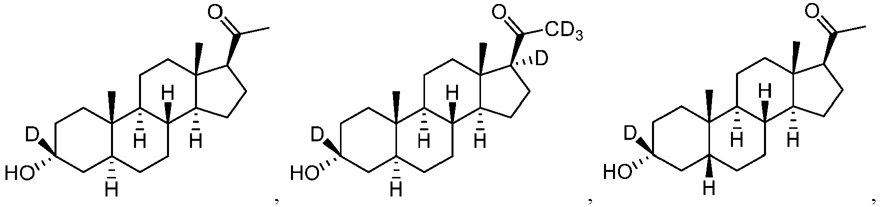 and 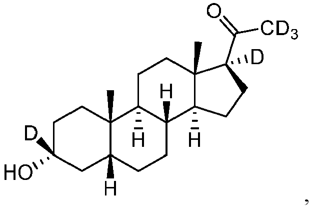 or a pharmaceutically acceptable salt thereof.

In some embodiments, the subject experiences sedation and/or anesthesia within one hour of administration. In some embodiments, the subject experiences sedation and/or anesthesia instantaneously.

In some embodiments, the compound is administered by intravenous administration. In some embodiments, the compound is administered chronically.

In some embodiments, the subject is a mammal. In some embodiments, the subject is a human.

In some embodiments, the compound is administered in combination with another therapeutic agent.

In one aspect, provided is a compound or a pharmaceutically acceptable salt thereof, for use in a method for treating seizure in a subject, comprising administering to the subject an effective amount of the compound selected from the group consisting of: 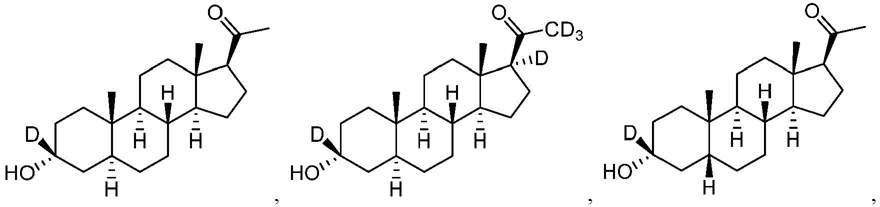 and 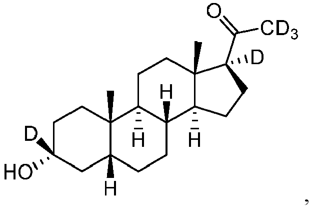 or a pharmaceutically acceptable salt thereof.

In one aspect, provided is a compound or pharmaceutically acceptable salt thereof, for use in a method for treating epilepsy in a subject, the method comprising administering to the subject an effective amount of the compound selected from the group consisting of: 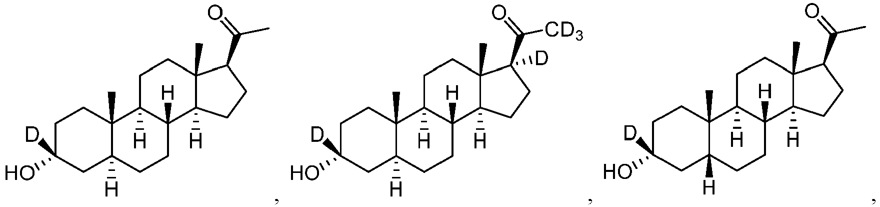 and 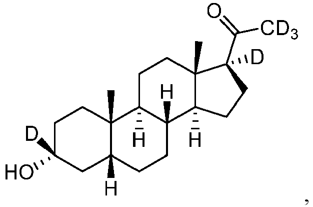 or a pharmaceutically acceptable salt thereof.

In one aspect, provided is a compound or pharmaceutically acceptable salt thereof, for use in a method for treating status epilepticus (SE) in a subject, the method comprising administering to the subject an effective amount of the compound selected from the group consisting of: 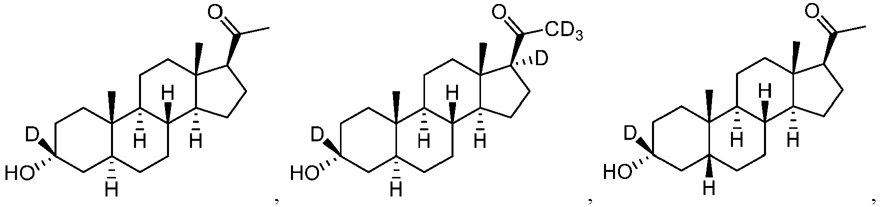 and 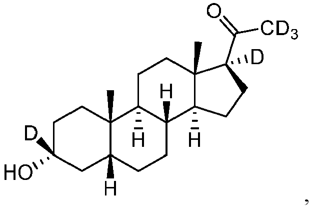 or a pharmaceutically acceptable salt thereof.

In some embodiments, the status epilepticus is convulsive status epilepticus (e.g., early status epilepticus, established status epilepticus, refractory status epilepticus, super-refractory status epilepticus) or non-convulsive status epilepticus, (*e.g.,* generalized status epilepticus, complex partial status epilepticus).

In one aspect, provided is a compound or pharmaceutically acceptable salt thereof, for use in a method of treating a human subject suffering from tremor, the method comprising administering a therapeutically effective amount of the compound selected from the group consisting of: 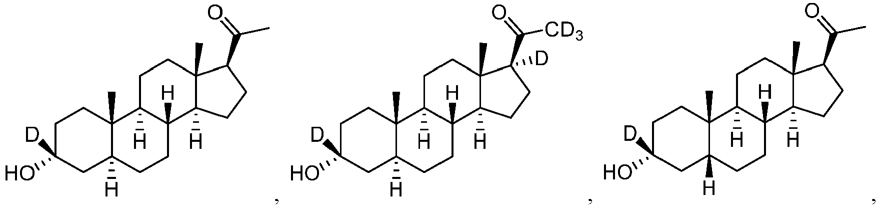 and 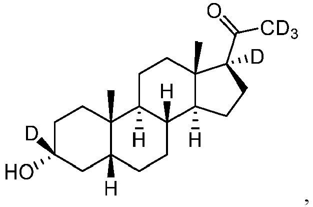 or a pharmaceutically acceptable salt thereof.

In some embodiments, the tremor is essential tremor.

In some embodiments, the administering is performed parenterally. In some embodiments, the administering is performed intravenously.

In some embodiments, the administering is performed orally.

In one aspect, provided is a deuterium-enriched compound or a pharmaceutically acceptable salt thereof, for use in a method for treating a CNS-related disorder in a subject in need thereof, comprising administering to the subject an effective amount of the deuterium-enriched compound selected from the group consisting of: 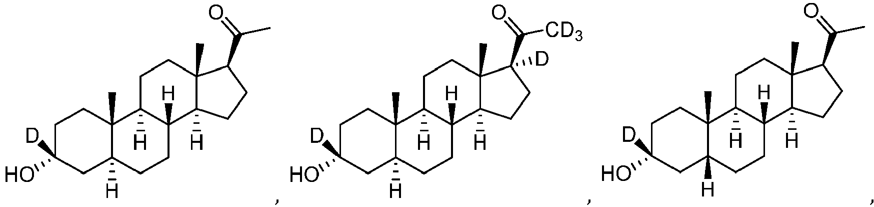 and 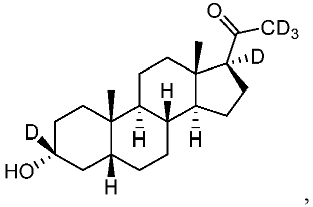 or a pharmaceutically acceptable salt thereof.

In some embodiments, the compound is administered orally.

In some embodiments, the compound is administered intramuscularly.

In some embodiments, the CNS-related disorder is a sleep disorder, a mood disorder, a schizophrenia spectrum disorder, a convulsive disorder, a disorder of memory and/or cognition, a movement disorder (e.g., tremor, for example essential tremor), a personality disorder, autism spectrum disorder, pain, traumatic brain injury, a vascular disease, a substance abuse disorder and/or withdrawal syndrome, or tinnitus.

In some embodiments, the subject is a subject with Rett syndrome, Fragile X syndrome, or Angelman syndrome.

In some embodiments, the subject is a mammal. In some embodiments, the subject is a human.

In some embodiments, the compound is administered in combination with another therapeutic agent.

In one aspect, provided is a pharmaceutical composition comprising a deuterium-enriched compound selected from the group consisting of: 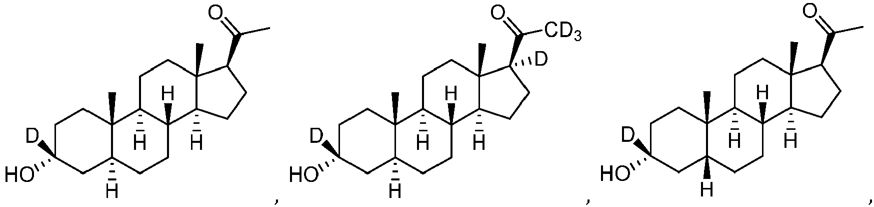 and 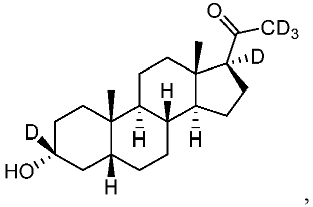 or a pharmaceutically acceptable salt thereof.

In some embodiments, the pharmaceutical composition comprises a pharmaceutically acceptable excipient.

In one aspect, provided is a deuterium-enriched compound selected from the group consisting of: 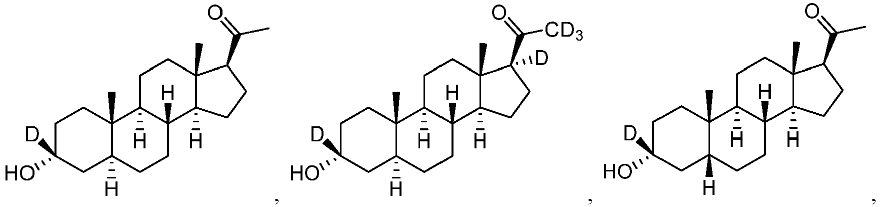 and 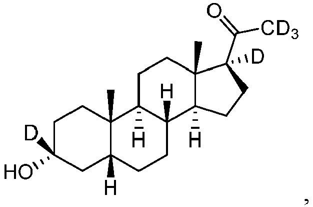 or a pharmaceutically acceptable salt thereof.

Also described herein are compositions comprising deuterium-enriched compounds of the Formula **(I).** The compositions require the presence of deuterium-enriched compounds of the Formula **(I)** that are greater than its natural abundance.

The present invention also provides pharmaceutical compositions comprising a deuterium-enriched compound of the present invention and deuterium-enriched compounds for use in methods of treatment, e.g., such as for inducing sedation and/or anesthesia, for treating a CNS-related disorder.
The compounds 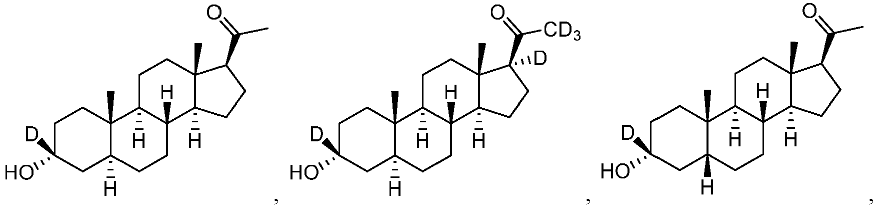 and 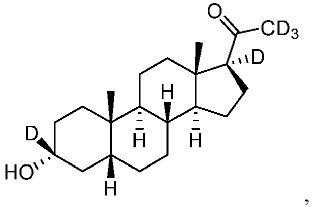 and pharmaceutically acceptable salts thereof are collectively referred to herein as "compounds of the present invention."

In another aspect, provided is a pharmaceutical composition comprising a deuterium-enriched compound of the present invention and a pharmaceutically acceptable excipient. In certain embodiments, the deuterium-enriched compound of the present invention is provided in an effective amount in the pharmaceutical composition. In certain embodiments, the deuterium-enriched compound of the present invention is provided in a therapeutically effective amount. In certain embodiments, the deuterium-enriched compound of the present invention is provided in a prophylactically effective amount.

Compounds of the present invention as described herein, act, in certain embodiments, as GABA modulators, e.g., effecting the GABA_{A} receptor in either a positive or negative manner. As modulators of the excitability of the central nervous system (CNS), as mediated by their ability to modulate GABA_{A} receptor, such compounds are expected to have CNS-activity.

Thus, in another aspect, compounds of the present invention are for use in methods of treating a CNS-related disorder in a subject in need thereof. In certain embodiments, the CNS-related disorder is selected from the group consisting of a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, and tinnitus. In certain embodiments, the compound is administered orally, subcutaneously, intravenously, or intramuscularly. In certain embodiments, the compound is administered chronically. In certain embodiments, the compound is administered continuously, *e.g.,* by continuous intravenous infusion.

Other objects and advantages will become apparent to those skilled in the art from a consideration of the ensuing **Detailed Description,** and **Claims.**

### Definitions

### Chemical definitions

Definitions of specific functional groups and chemical terms are described in more detail below. The chemical elements are identified in accordance with the Periodic Table of the

Elements, CAS version, *Handbook of Chemistry and Physics,* 75^{th} Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Compounds described herein (e.g., a compound of Formula (**II**), e.g., a compound of Formula **(I)**) are deuterium-enriched.

Deuterium (D or ²H) is a stable, non-radioactive isotope of hydrogen and has an atomic weight of 2.0144. Hydrogen naturally occurs as a mixture of the isotopes ¹H (hydrogen or protium), D (²H or deuterium), and T (³H or tritium). The natural abundance of deuterium is 0.015%. One of ordinary skill in the art recognizes that in all chemical compounds with a H atom, the H atom actually represents a mixture of H and D, with about 0.015% being D. Thus, compounds with a level of deuterium that has been enriched to be greater than its natural abundance of 0.015% should be considered unnatural and, as a result, novel over their non-enriched counterparts.

The effects of deuterium modification on a compound's metabolic properties are not predictable, even when deuterium atoms are incorporated at known sites of metabolism. Only by actually preparing and testing a deuterated compound can one determine if and how the rate of metabolism will differ from that of its non-deuterated counterpart. See, for example, Fukuto et al. (J. Med. Chem. 1991, 34, 2871-76). Many compounds have multiple sites where metabolism is possible. The site(s) where deuterium substitution is required and the extent of deuteration necessary to see an effect on metabolism, if any, will be different for each compound.

Unless otherwise stated, when a position is designated specifically as "H" or "hydrogen," the position is understood to have hydrogen at its natural abundance isotopic composition. Also unless otherwise stated, when a position is designated specifically as "D" or "deuterium," the position is understood to have deuterium at an abundance that is at least 3000 times greater than the natural abundance of deuterium, which is 0.015% (i.e., the term "D" or "deuterium" indicates at least 45% incorporation of deuterium).

The term "isotopic enrichment factor" as used herein means the ratio between the isotopic abundance of D at the specified position in a compound of this invention and the naturally occurring abundance of that isotope.

Increasing the amount of deuterium present in a compound (e.g., a compound of Formula (I) is called "deuterium-enrichment," and such compounds are referred to as "deuterium-enriched" compounds. If not specifically noted, the percentage of enrichment refers to the percentage of deuterium present in the compound.

In other embodiments, a compound of this invention has an isotopic enrichment factor for each deuterium present at a site designated at a potential site of deuteration on the compound of at least 3500 (52.5.% deuterium incorporation), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6466.7 (97% deuterium incorporation), at least 6633.3 (99.5% deuterium incorporation). It is understood that the isotopic enrichment factor of each deuterium present at a site designated as a site of deuteration is independent of other deuterated sites. For example, if there are two sites of deuteration on a compound one site could be deuterated at 52.5% while the other could be deuterated at 75%. The resulting compound would be considered to be a compound wherein the isotopic enrichment factor is at least 3500 (52.5%).

Because the natural abundance of deuterium is about 0.015%, approximately one in every 6,667 naturally occurring compounds of Formula (**II**), e.g., a compounds of Formula **(I)** would be expected to have one naturally occurring compound of Formula (**II**), e.g., a compounds of Formula **(I)** with one deuterium present.

In some embodiments, the compounds of Formula (**II**), e.g., a compounds of Formula **(I)** comprise an amount of deuterium-enrichment that is more than the amount of deuterium-enrichment present in naturally occurring compounds of Formula (**II**), e.g., a compounds of Formula **(I).**

All percentages given for the amount of deuterium present are mole percentages.

It can be difficult in the laboratory to achieve 100% deuteration at any one site of a lab scale amount of compound (e.g., milligram or greater). When 100% deuteration is recited or a deuterium atom is specifically shown in a structure, it is assumed that a small percentage of hydrogen may still be present. Deuterium-enriched can be achieved by either exchanging protons with deuterium or by synthesizing the molecule with enriched starting materials.

Also described herein is the isolation or purification of deuterium-enriched compounds of Formula (**II**), e.g., a compounds of Formula **(I).** The isolated or purified deuterium-enriched compounds of Formula (**II**), e.g., a compounds of Formula **(I)** are above the naturally occurring levels.

Compounds described herein can comprise one or more asymmetric centers, and thus can exist in various isomeric forms, e.g., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The invention additionally encompasses compounds described herein as individual isomers substantially free of other isomers, and alternatively, as mixtures of various isomers.

As used herein a pure enantiomeric compound is substantially free from other enantiomers or stereoisomers of the compound (*i.e.,* in enantiomeric excess). In other words, an "S" form of the compound is substantially free from the "R" form of the compound and is, thus, in enantiomeric excess of the "R" form. The term "enantiomerically pure" or "pure enantiomer" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of the enantiomer. In certain embodiments, the weights are based upon total weight of all enantiomers or stereoisomers of the compound.

In the compositions provided herein, an enantiomerically pure compound can be present with other active or inactive ingredients. For example, a pharmaceutical composition comprising enantiomerically pure R-compound can comprise, for example, about 90% excipient and about 10% enantiomerically pure R-compound. In certain embodiments, the enantiomerically pure R-compound in such compositions can, for example, comprise, at least about 95% by weight R-compound and at most about 5% by weight S-compound, by total weight of the compound. For example, a pharmaceutical composition comprising enantiomerically pure S-compound can comprise, for example, about 90% excipient and about 10% enantiomerically pure S-compound. In certain embodiments, the enantiomerically pure S-compound in such compositions can, for example, comprise, at least about 95% by weight S-compound and at most about 5% by weight R-compound, by total weight of the compound. In certain embodiments, the active ingredient can be formulated with little or no excipient or carrier.

Compound described herein may also comprise one or more isotopic substitutions. For example, H may be in any isotopic form, including ¹H, ²H (D or deuterium), and ³H (T or tritium); C may be in any isotopic form, including ¹²C, ¹³C, and ¹⁴C; O may be in any isotopic form, including ¹⁶O and ¹⁸O; and the like.

The articles "a" and "an" may be used herein to refer to one or to more than one (*i.e.* at least one) of the grammatical objects of the article. By way of example "an analogue" means one analogue or more than one analogue.

When a range of values is listed, it is intended to encompass each value and sub-range within the range. For example "C₁₋₆ alkyl" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

The following terms are intended to have the meanings presented therewith below and are useful in understanding the description and intended scope of the present invention.

"Alkyl" refers to a radical of a straight-chain or branched saturated hydrocarbon group having 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). Unless otherwise specified, each instance of an alkyl group is independently optionally substituted, *i.e.,* unsubstituted (an "unsubstituted alkyl") or substituted (a "substituted alkyl") with one or more substituents; e.g., for instance from 1 to 3 substituents, or 1 substituent. In certain embodiments, the alkyl group is unsubstituted C₁₋₄ alkyl (*e.g.,* -CH₃). In certain embodiments, the alkyl group is substituted C₁₋₄ alkyl. Common alkyl abbreviations include Me (-CH₃), Et (-CH₂CH₃), or iPr (-CH(CH₃)₂).

A "counterion" or "anionic counterion" is a negatively charged group associated with a cationic quaternary amino group in order to maintain electronic neutrality. Exemplary counterions include halide ions (*e.g.,* F⁻, Cl⁻, Br⁻, I⁻), NO₃⁻, ClO₄⁻, OH , H₂PO4⁻, HSO₄⁻, sulfonate ions (*e.g.,* methansulfonate, trifluoromethanesulfonate, p-toluenesulfonate, benzenesulfonate, 10-camphor sulfonate, naphthalene-2-sulfonate, naphthalene-1-sulfonic acid-5-sulfonate, ethan-1-sulfonic acid-2-sulfonate, and the like), and carboxylate ions (*e.g.,* acetate, ethanoate, propanoate, benzoate, glycerate, lactate, tartrate, glycolate, and the like).

### Other definitions

As used herein, the term "modulation" refers to the inhibition or potentiation of GABA receptor function. A "modulator" (*e.g.,* a modulator compound) may be, for example, an agonist, partial agonist, antagonist, or partial antagonist of the GABA receptor.

"Pharmaceutically acceptable" means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

"Pharmaceutically acceptable salt" refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. The term "pharmaceutically acceptable cation" refers to an acceptable cationic counter-ion of an acidic functional group. Such cations are exemplified by sodium, potassium, calcium, magnesium, ammonium, tetraalkylammonium cations, and the like. See, *e.g.,* Berge, et al., J. Pharm. Sci. (1977)66(1): 1-79.

"Solvate" refers to forms of the compound that are associated with a solvent or water (also referred to as "hydrate"), usually by a solvolysis reaction. This physical association includes hydrogen bonding. Conventional solvents include water, ethanol, acetic acid, and the like. The compounds of the invention may be prepared *e.g.* in crystalline form and may be solvated or hydrated. Suitable solvates include pharmaceutically acceptable solvates, such as hydrates, and further include both stoichiometric solvates and non-stoichiometric solvates. In certain instances the solvate will be capable of isolation, for example when one or more solvent molecules are incorporated in the crystal lattice of the crystalline solid. "Solvate" encompasses both solution-phase and isolable solvates. Representative solvates include hydrates, ethanolates and methanolates.

"Stereoisomers": It is also to be understood that compounds that have the same molecular formula but differ in the nature or sequence of bonding of their atoms or the arrangement of their atoms in space are termed "isomers." Isomers that differ in the arrangement of their atoms in space are termed "stereoisomers." Stereoisomers that are not mirror images of one another are termed "diastereomers" and those that are non-superimposable mirror images of each other are termed "enantiomers." When a compound has an asymmetric center, for example, it is bonded to four different groups, a pair of enantiomers is possible. An enantiomer can be characterized by the absolute configuration of its asymmetric center and is described by the Rand S-sequencing rules of Cahn and Prelog, or by the manner in which the molecule rotates the plane of polarized light and designated as dextrorotatory or levorotatory (*i.e.,* as (+) or (-)-isomers respectively). A chiral compound can exist as either individual enantiomer or as a mixture thereof. A mixture containing equal proportions of the enantiomers is called a "racemic mixture".

"Tautomers" refer to compounds that are interchangeable forms of a particular compound structure, and that vary in the displacement of hydrogen atoms and electrons. Thus, two structures may be in equilibrium through the movement of π electrons and an atom (usually H). For example, enols and ketones are tautomers because they are rapidly interconverted by treatment with either acid or base. Another example of tautomerism is the aci- and nitro- forms of phenylnitromethane, that are likewise formed by treatment with acid or base. Tautomeric forms may be relevant to the attainment of the optimal chemical reactivity and biological activity of a compound of interest.

A "subject" to which administration is contemplated includes, but is not limited to, humans (*i.e.,* a male or female of any age group, *e.g.,* a pediatric subject (*e.g,* infant, child, adolescent) or adult subject (*e.g.,* young adult, middle-aged adult or senior adult)) and/or a non-human animal, *e.g.,* a mammal such as primates (*e.g.,* cynomolgus monkeys, rhesus monkeys), cattle, pigs, horses, sheep, goats, rodents, cats, and/or dogs. In certain embodiments, the subject is a human. In certain embodiments, the subject is a non-human animal. The terms "human," "patient," and "subject" are used interchangeably herein.

Disease, disorder, and condition are used interchangeably herein.

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition ("therapeutic treatment"), and also contemplates an action that occurs before a subject begins to suffer from the specified disease, disorder or condition ("prophylactic treatment").

In general, the "effective amount" of a compound refers to an amount sufficient to elicit the desired biological response, *e.g.,* to treat a CNS-related disorder, is sufficient to induce anesthesia or sedation. As will be appreciated by those of ordinary skill in this art, the effective amount of a compound of the invention may vary depending on such factors as the desired biological endpoint, the pharmacokinetics of the compound, the disease being treated, the mode of administration, and the age, weight, health, and condition of the subject. An effective amount encompasses therapeutic and prophylactic treatment.

As used herein, and unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

As used herein, and unless otherwise specified, a "prophylactically effective amount" of a compound is an amount sufficient to prevent a disease, disorder or condition, or one or more symptoms associated with the disease, disorder or condition, or prevent its recurrence. A prophylactically effective amount of a compound means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

### Detailed Description of Certain Embodiments of the Invention

The present invention, as defined in the appended claims, relates to compounds selected from the group consisting of: 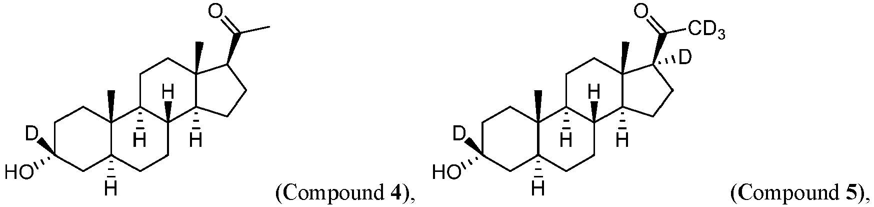 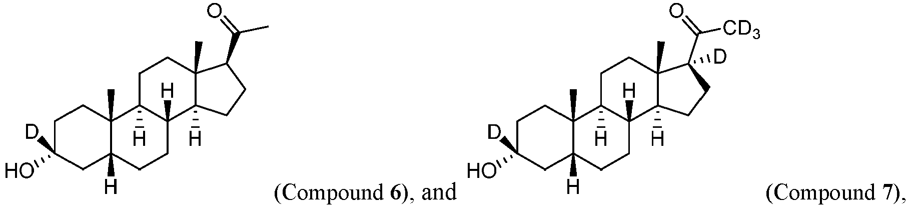 or pharmaceutically acceptable salts thereof, as set out in the claims and which are collectively referred to herein as "compounds of the present invention".

The technical information set out below may in some respects go beyond the scope of the presently claimed invention, which is defined by the appended claims. With the exception of Compounds **4, 5, 6,** and **7,** and their pharmaceutically acceptable salts, compounds of Formulae **(II), (II-a), (II-b), (II-c), (I), (I-a), (I-b), (I-c), (I-a-1), (I-a-2), (I-a-3)** and their pharmaceutically acceptable salts as described herein are not encompassed by the wording of the claims but are considered useful for understanding the invention, and are provided to place the invention in a broader technical context.

With the exception of Compounds **4, 5, 6,** and **7,** and their pharmaceutically acceptable salts, compounds of Formulae **(II), (II-a), (II-b), (II-c), (I), (I-a), (I-b), (I-c), (I-a-1), (I-a-2), (I-a-3),** and their pharmaceutically acceptable salts, which do not fall within the scope of the appended claims are not part of the presently claimed invention.

As generally described herein, the present invention provides deuterium-enriched neuroactive steroids designed, for example, to act as GABA modulators. The compounds of the present invention are envisioned to be useful as therapeutic agents for treating a CNS-related disorder (e.g., a disorder as described herein, for example depression, such as post-partum depression). The compounds of the present invention are envisioned to be useful as therapeutic agents for the inducement of anesthesia and/or sedation in a subject.

### Compounds

The present invention, as defined in the appended claims, relates to compounds selected from the group consisting of: 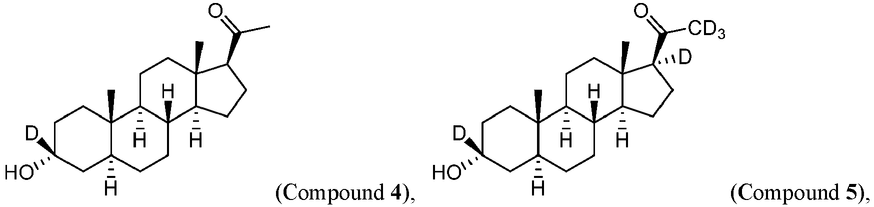 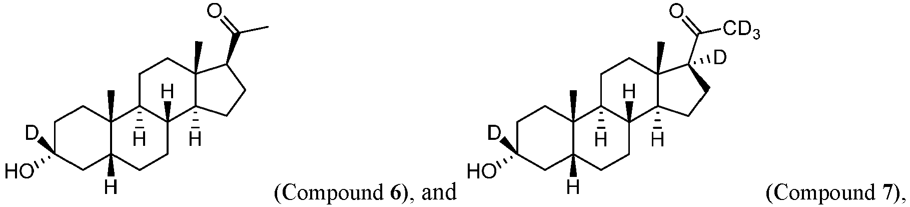 or pharmaceutically acceptable salts thereof, as set out in the claims and which are collectively referred to herein as "compounds of the present invention".

Described generally below are compounds of Formulae **(II), (II-a), (II-b), (II-c), (I), (I-a), (I-b), (I-c), (I-a-1), (I-a-2), (I-a-3):**
In one aspect, provided is a deuterium-enriched compound of the Formula **(II):** 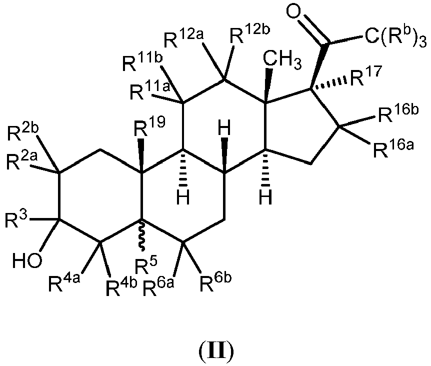 or a pharmaceutically acceptable salt thereof wherein: each of R^{2a}, R^{2b}, R^{4a}, R^{4b}, R⁵, R^{6a}, R^{6b}, R^{12a}, R^{12b,} R^{16a}, R^{16b}, R¹⁷, and R^{b} is independently hydrogen or deuterium; R³ is hydrogen, deuterium, or -C(R^{c})₃, wherein each R^{c} is independently hydrogen or deuterium; each of R^{11a} and R^{11b} is independently hydrogen or deuterium; or R^{11a} and R^{11b} are taken together to form an oxo (=O) group; and R¹⁹ is hydrogen, deuterium, or -C(R^{a})₃, wherein each R^{a} is independently hydrogen or deuterium; wherein at least one of R^{2a}, R^{2b}, R³, R^{4a}, R^{4b}, R⁵, R^{6a}, R^{6b}, R^{11a}, R^{11b}, R^{12a,} R^{12b}, R^{16a}, R^{16b}, R¹⁷, R¹⁹, R^{a}, R^{b} and R^{c} is deuterium.

In some aspects, R⁵ is deuterium.

In some aspects, R¹⁹ is deuterium or -CD₃.

In some aspects, at least one of R³, R^{4a}, R^{4b}, R⁵, R^{6a}, R^{6b}, R^{11a}, R^{11b}, R^{12a}, R^{12b,} R^{16a}, R^{16b}, R¹⁷, R^{a}, R^{b} and R^{c} is deuterium.

In some aspects, at least one of R^{2a}, R^{2b}, R³, R⁵, R^{6a}, R^{6b}, R^{11a}, R^{11b}, R^{12a}, R^{12b,} R^{16a}, R^{16b}, R¹⁷, R^{a} , R^{b} and R^{c} is deuterium.

In some aspects, at least one of R^{2a}, R^{2b}, R³, R^{4a}, R^{4b}, R⁵, R^{11a}, R^{11b}, R^{12a}, R^{12b,} R^{16a}, R^{16b}, R¹⁷, R^{a} , R^{b} and R^{c} is deuterium.

In some aspects, at least one of R^{2a}, R^{2b}, R³, R^{4a}, R^{4b}, R⁵, R^{6a}, R^{6b}, R^{12a}, R^{12b,} R^{16a}, R^{16b}, R¹⁷, R^{a}, R^{b} and R^{c} is deuterium.

In some aspects, at least one of R^{2a}, R^{2b}, R³, R^{4a}, R^{4b}, R⁵, R^{6a}, R^{6b}, R^{11a}, R^{11b}, R^{16a}, R^{16b}, R¹⁷, R^{a}, R^{b} and R^{c} is deuterium.

In some aspects, at least one of R^{2a}, R^{2b}, R³, R^{4a}, R^{4b}, R⁵, R^{6a}, R^{6b}, R^{11a}, R^{11b}, R^{12a}, R^{12b,} R¹⁷, R^{a}, R^{b} and R^{c} is deuterium.

In some aspects, at least one of R³, R⁵, R¹⁷, R^{a}, R^{b} and R^{c} is deuterium.

In some aspects, the compound of Formula (**II**) is a compound of Formula (**II-a**):

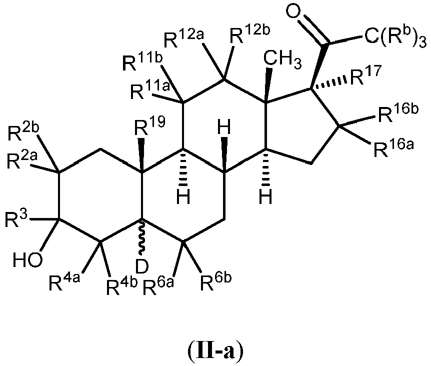

In some aspects, the compound of Formula (**II**) is a compound of Formula (**II-b**):

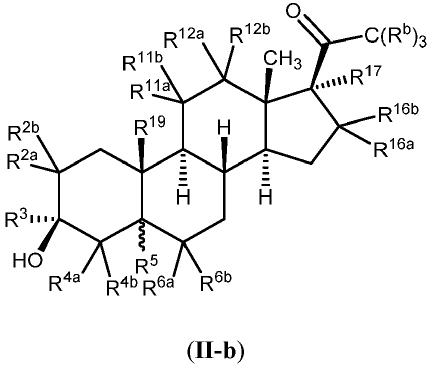

In some aspects, the compound of Formula (**II**) is a compound of Formula (**II-c**):

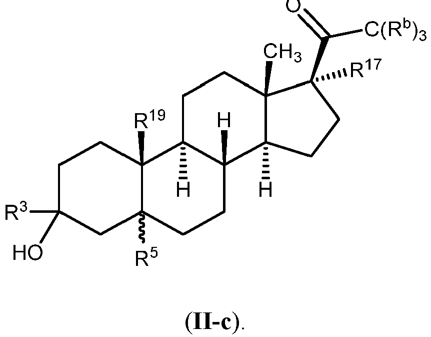

In some aspects, the compound of Formula (**II**) is a compound of Formula **(I):** 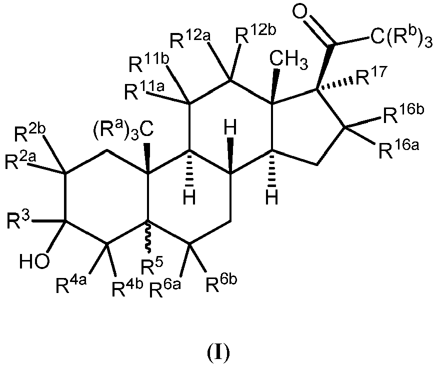 or a pharmaceutically acceptable salt thereof wherein: each of R^{2a}, R^{2b}, R^{4a}, R^{4b}, R⁵, R^{6a}, R^{6b}, R^{12a}, R^{12b,} R^{16a}, R^{16b}, R¹⁷, R^{a}, and R^{b} is independently hydrogen or deuterium; R³ is hydrogen, deuterium, or -C(R^{c})₃, wherein each R^{c} is independently hydrogen or deuterium; and each of R^{11a} and R^{11b} is independently hydrogen or deuterium; or R^{11a} and R^{11b} are taken together to form an oxo (=O) group; wherein at least one of R^{2a}, R^{2b}, R³, R^{4a}, R^{4b}, R⁵, R^{6a}, R^{6b}, R^{11a,} R^{11b}, R^{12a}, R^{12b,} R^{16a}, R^{16b}, R¹⁷, R^{a} , R^{b} and R^{c} is deuterium.

In some aspects, the deuterium-enriched compound is not:

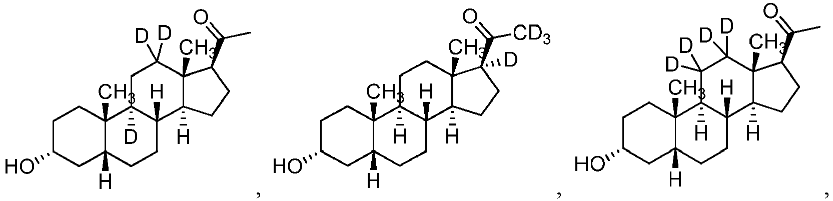

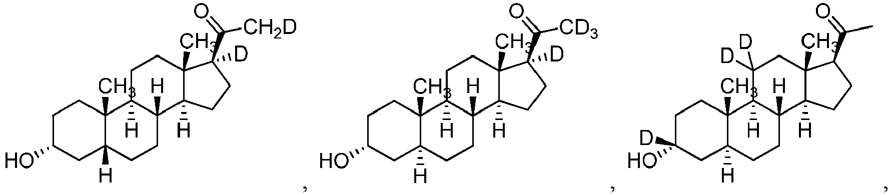

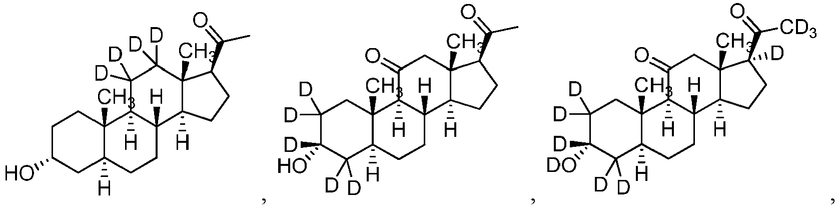

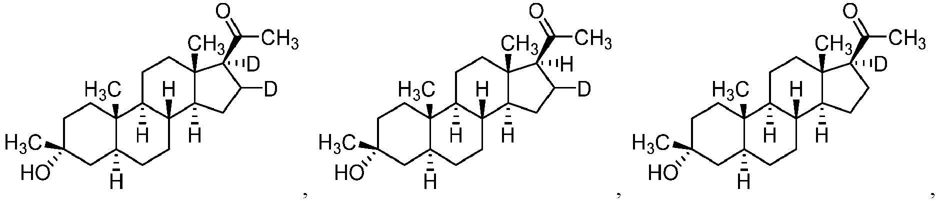

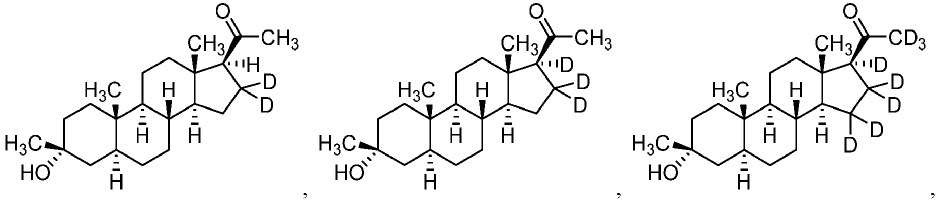

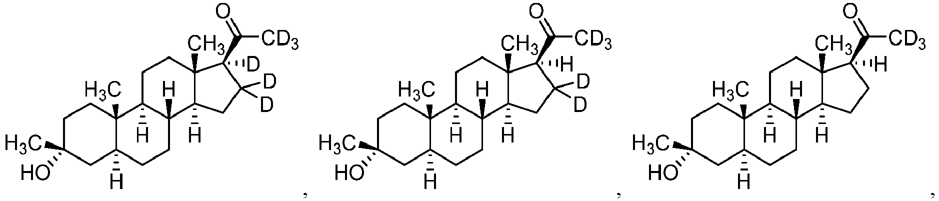

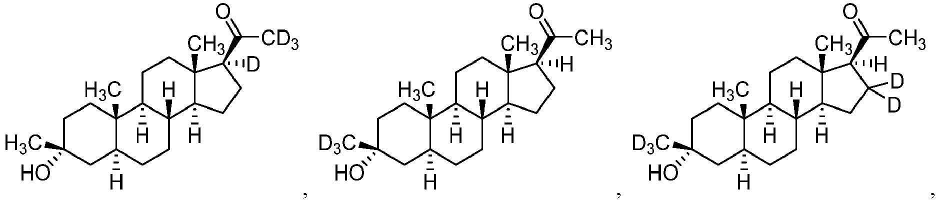

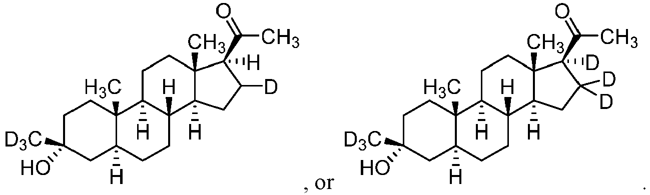

In some aspects, the compound is a compound of Formula **(I-a):** 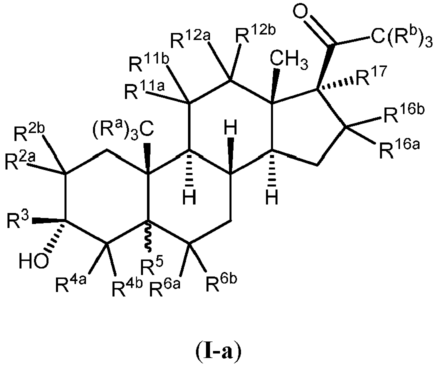 or a pharmaceutically acceptable salt thereof, wherein R^{2a}, R^{2b}, R³, R^{4a}, R^{4b}, R⁵, R^{6a}, R^{6b}, R^{11a}, R^{11b}, R^{12a}, R^{12b,} R^{16a}, R^{16b}, R¹⁷, R^{a}, R^{b} and R^{c} are as defined in Formula **(I).**

In some aspects, the compound is a compound of Formula **(I-b):** 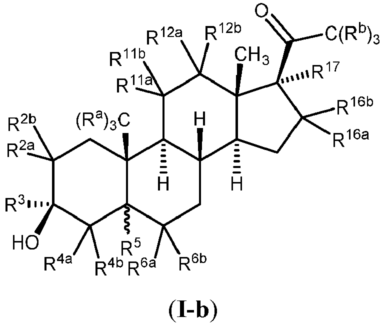 or a pharmaceutically acceptable salt thereof, wherein R^{2a}, R^{2b}, R³, R^{4a}, R^{4b}, R⁵, R^{6a}, R^{6b}, R^{11a}, R^{11b}, R^{12a}, R^{12b,} R^{16a}, R^{16b}, R¹⁷, R^{a}, R^{b} and R^{c} are as defined in Formula **(I).**

In some aspects, the compound is a compound of Formula (**I-a-1**): 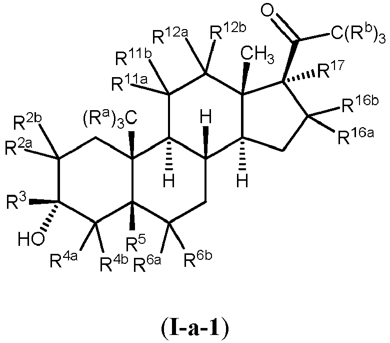 or a pharmaceutically acceptable salt thereof, wherein R^{2a}, R^{2b}, R³, R^{4a}, R^{4b}, R⁵, R^{6a}, R^{6b}, R^{11a}, R^{11b}, R^{12a}, R^{12b,} R^{16a}, R^{16b}, R¹⁷, R^{a}, R^{b} and R^{c} are as defined in Formula **(I).**

In some aspects, the compound is a compound of Formula (**I-a-2**): 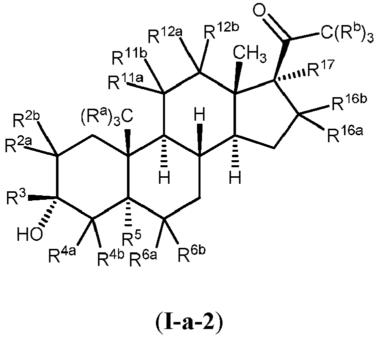 or a pharmaceutically acceptable salt thereof, wherein R^{2a}, R^{2b}, R³, R^{4a}, R^{4b}, R⁵, R^{6a}, R^{6b}, R^{11a}, R^{11b}, R^{12a}, R^{12b,} R^{16a}, R^{16b}, R¹⁷, R^{a}, R^{b} and R^{c} are as defined in Formula **(I).**

In some aspects, the compound is a compound of Formula (**I-a-3**): 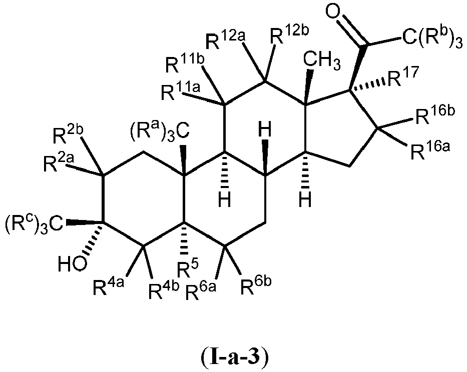 or a pharmaceutically acceptable salt thereof, wherein R^{2a}, R^{2b}, R^{4a}, R^{4b}, R⁵, R^{6a}, R^{6b}, R^{11a}, R^{11b}, R^{12a}, R^{12b,} R^{16a}, R^{16b}, R¹⁷, R^{a}, R^{b} and R^{c} are as defined in Formula **(I).**

In some aspects, the compound is a compound of Formula **(I-c):** 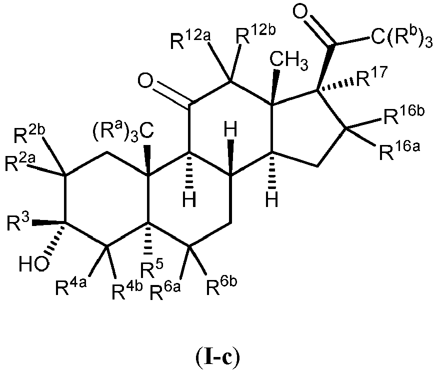 or a pharmaceutically acceptable salt thereof, wherein R^{2a}, R^{2b}, R³, R^{4a}, R^{4b}, R⁵, R^{6a}, R^{6b}, R^{12a}, R^{12b,} R^{16a}, R^{16b}, R¹⁷, R^{a}, R^{b} and R^{c} are as defined in Formula **(I).**

In some aspects, at least one of R^{2a}, R^{2b}, R^{4a}, R^{4b}, R⁵, R^{6a}, and R^{6b} is independently deuterium. In someaspects, at least one of R^{11a} , R^{11b}, R^{12a}, R^{12b,} R^{16a}, and R^{16b} is independently deuterium.

In some aspects, R¹⁷ is deuterium.

In some aspects, R³ is deuterium or C(R^{c})₃ (e.g., CDH₂, CD₂H, or CD₃). In someaspects, R^{a} or R^{b} is independently deuterium at each occurrence.

In some aspects, at least one of R^{2a}, R^{2b}, R^{4a}, R^{4b}, R⁵, R^{6a}, and R^{6b} is independently hydrogen. In some aspects, each of R^{2a}, R^{2b}, R^{4a}, R^{4b}, R⁵, R^{6a}, and R^{6b} is independently hydrogen. In some aspects, at least one of R^{11a}, R^{11b}, R^{12a}, R^{12b,} R^{16a}, and R^{16b} is independently hydrogen. In some aspects, R^{a} or R^{b} is independently hydrogen at each occurrence.

In someaspects, when R³ is deuterium, then at least one of R^{11a} and R^{11b} is hydrogen; when R¹⁷ is deuterium, then each R^{b} is hydrogen; and when R^{11a}, R^{11b}, R^{12a}, and R^{12b} are deuterium, then the compound comprises at least 5 deuterium atoms.

In some aspects, when R³ is deuterium, then either R^{2a} and R^{2b} or R^{11a} and R^{11b} are not deuterium.

In some aspects, when R¹⁷ is deuterium, then at least one of R⁵, R^{6a}, R^{6b}, R^{11a}, R^{11b}, R^{12a}, R^{12b,} R^{16a}, R^{16b}, and R^{a} is deuterium.

In some aspects, R⁵ is deuterium.

In some aspects, exactly one of R^{2a}, R^{2b}, R^{4a}, R^{4b}, R⁵, R^{6a}, R^{6b}, R^{11a}, R^{11b}, R^{12a}, R^{12b,} R^{16a}, and R^{16b} is deuterium.

### Pharmaceutical Compositions

The present invention, as defined in the appended claims, relates to a pharmaceutical composition comprising a deuterium-enriched compound selected from the group consisting of: 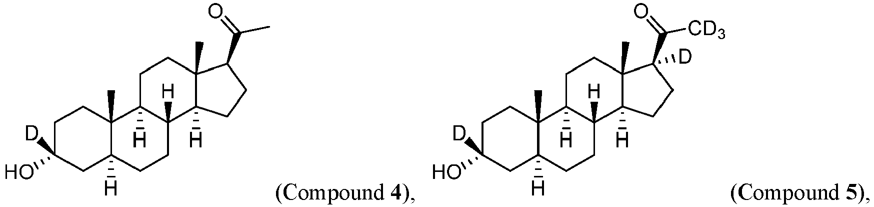 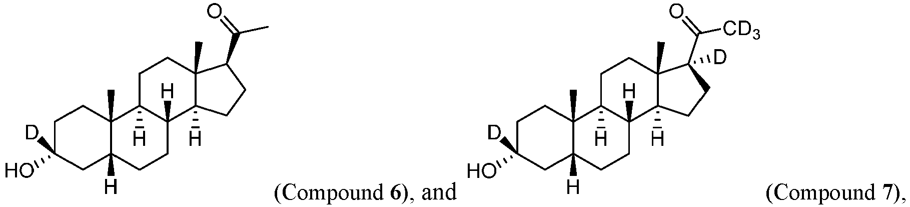 or a pharmaceutically acceptable salt thereof.

Described generally below are pharmaceutical compositions comprising a deuterium-enriched compound of Formulae **(II), (II-a), (II-b), (II-c), (I):**

In one aspect, provided is a pharmaceutical composition comprising a deuterium-enriched compound of the Formula (**II**): 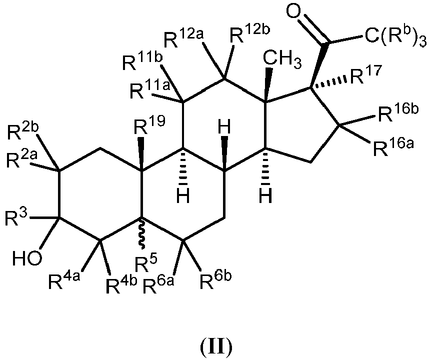 or a pharmaceutically acceptable salt thereof wherein: each of R^{2a}, R^{2b}, R^{4a}, R^{4b}, R⁵, R^{6a}, R^{6b}, R^{12a}, R^{12b,} R^{16a}, R^{16b}, R¹⁷, and R^{b} is independently hydrogen or deuterium; R³ is hydrogen, deuterium, or -C(R^{c})₃, wherein each R^{c} is independently hydrogen or deuterium; each of R^{11a} and R^{11b} is independently hydrogen or deuterium; or R^{11a} and R^{11b} are taken together to form an oxo (=O) group; and R¹⁹ is hydrogen, deuterium, or -C(R^{a})₃, wherein each R^{a} is independently hydrogen or deuterium; wherein at least one of R^{2a}, R^{2b}, R³, R^{4a}, R^{4b}, R⁵, R^{6a}, R^{6b}, R^{11a}, R^{11b}, R^{12a,} R^{12b}, R^{16a}, R^{16b}, R¹⁷, R¹⁹, R^{a}, R^{b} and R^{c} is deuterium.

In some aspects, R⁵ is deuterium.

In some aspects, R¹⁹ is deuterium or -CD₃.

In some aspects, at least one of R³, R^{4a}, R^{4b}, R⁵, R^{6a}, R^{6b}, R^{11a}, R^{11b}, R^{12a}, R^{12b,} R^{16a}, R^{16b}, R¹⁷, R^{a}, R^{b} and R^{c} is deuterium.

In some aspects, at least one of R^{2a}, R^{2b}, R³, R⁵, R^{6a}, R^{6b}, R^{11a}, R^{11b}, R^{12a}, R^{12b,} R^{16a}, R^{16b}, R¹⁷, R^{a}, R^{b} and R^{c} is deuterium.

In some aspects, at least one of R^{2a}, R^{2b}, R³, R^{4a}, R^{4b}, R⁵, R^{11a}, R^{11b}, R^{12a}, R^{12b,} R^{16a}, R^{16b}, R¹⁷, R^{a}, R^{b} and R^{c} is deuterium.

In some aspects, at least one of R^{2a}, R^{2b}, R³, R^{4a}, R^{4b}, R⁵, R^{6a}, R^{6b}, R^{12a}, R^{12b,} R^{16a}, R^{16b}, R¹⁷, R^{a}, R^{b} and R^{c} is deuterium.

In some aspects, at least one of R^{2a}, R^{2b}, R³, R^{4a}, R^{4b}, R⁵, R^{6a}, R^{6b}, R^{11a}, R^{11b}, R^{16a}, R^{16b}, R¹⁷, R^{a}, R^{b} and R^{c} is deuterium.

In some aspects, at least one of R^{2a}, R^{2b}, R³, R^{4a}, R^{4b}, R⁵, R^{6a}, R^{6b}, R^{11a}, R^{11b}, R^{12a}, R^{12b,} R¹⁷, R^{a}, R^{b} and R^{c} is deuterium.

In some aspects, at least one of R³, R⁵, R¹⁷, R^{a}, R^{b} and R^{c} is deuterium.

In some aspects, the compound of Formula (**II**) is a compound of Formula (**II-a**):

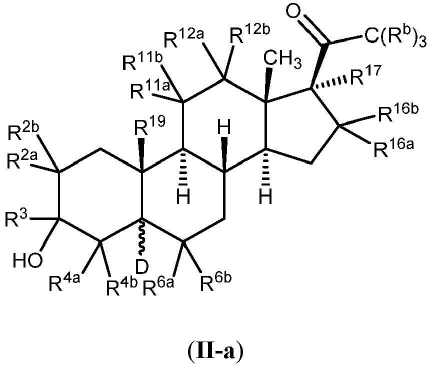

In some aspects, the compound of Formula (**II**) is a compound of Formula (**II-b**):

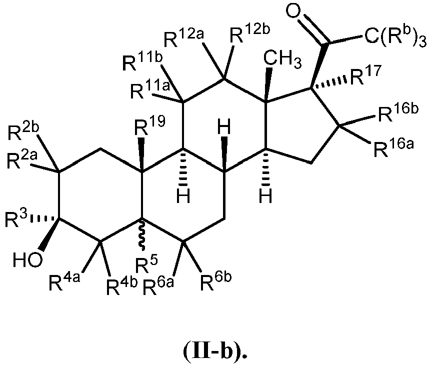

In some aspects, the compound of Formula (**II**) is a compound of Formula (**II-c**):

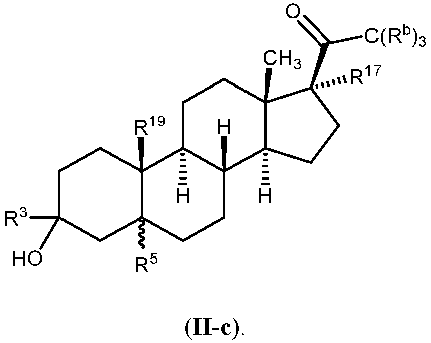

In some aspects, the deuterium-enriched compound of the Formula (**II**) is a compound of the Formula **(I):** 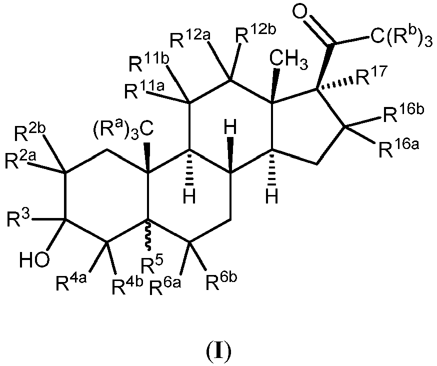 or a pharmaceutically acceptable salt thereof wherein: each of R^{2a}, R^{2b}, R^{4a}, R^{4b}, R⁵, R^{6a}, R^{6b}, R^{12a}, R^{12b}, R^{16a}, R^{16b}, R¹⁷, R^{a}, and R^{b} is independently hydrogen or deuterium; R³ is hydrogen, deuterium, or -C(R^{c})₃, wherein each R^{c} is independently hydrogen or deuterium; and each of R^{11a} and R^{11b} is independently hydrogen or deuterium; or R^{11a} and R^{11b} are taken together to form an oxo (=O) group; wherein at least one of R^{2a}, R^{2b}, R³, R^{4a}, R^{4b}, R⁵, R^{6a}, R^{6b}, R^{11a}, R^{11b}, R^{12a,} R^{12b}, R^{16a}, R^{16b}, R¹⁷, R^{a}, R^{b} and R^{c} is deuterium.

In some embodiments, the pharmaceutical composition comprises a pharmaceutically acceptable excipient. In certain embodiments, the pharmaceutical composition comprises an effective amount of the active ingredient. In certain embodiments, the pharmaceutical composition comprises a therapeutically effective amount of the active ingredient. In certain embodiments, the pharmaceutical composition comprises a prophylactically effective amount of the active ingredient.

The pharmaceutical compositions provided herein can be administered by a variety of routes including, but not limited to, oral (enteral) administration, parenteral (by injection) administration, rectal administration, transdermal administration, intradermal administration, intrathecal administration, subcutaneous (SC) administration, intravenous (IV) administration, intramuscular (IM) administration, and intranasal administration.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the onset of a CNS-disorder, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, *e.g.,* for example, over 3 months, 6 months, 1 year, 2 years, 3 years, 5 years, *etc,* or may be continued indefinitely, for example, for the rest of the subject's life. In certain embodiments, the chronic administration is intended to provide a constant level of the compound in the blood, *e.g.,* within the therapeutic window over the extended period of time.

The pharmaceutical compositions of the present invention may be further delivered using a variety of dosing methods. For example, in certain embodiments, the pharmaceutical composition may be given as a bolus, *e.g.,* in order to raise the concentration of the compound in the blood to an effective level. The placement of the bolus dose depends on the systemic levels of the active ingredient desired throughout the body, *e.g.,* an intramuscular or subcutaneous bolus dose allows a slow release of the active ingredient, while a bolus delivered directly to the veins (*e.g.,* through an IV drip) allows a much faster delivery which quickly raises the concentration of the active ingredient in the blood to an effective level. In other embodiments, the pharmaceutical composition may be administered as a continuous infusion, *e.g.,* by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or preferably from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with preferred doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, preferably from about 0.1 to about 20% by weight, preferably from about 0.1 to about 10% by weight, and more preferably from about 0.5 to about 15% by weight.

Injection dose levels range from about 0.1 mg/kg/hour to at least 20 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 5 g/day for a 40 to 80 kg human patient.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as a ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or Formulation. All such known transdermal formulations and ingredients are included within the scope provided herein.

The compounds provided herein can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 *of* Remington 's Pharmaceutical Sciences, 17th edition, 1985, Mack Publishing Company, Easton, Pennsylvania.

The compounds of the present invention can also be administered in sustained release forms or from sustained release drug delivery systems. A description of representative sustained release materials can be found in *Remington's Pharmaceutical Sciences.*

The present invention also relates to the pharmaceutically acceptable acid addition salt of a compound of the present invention. The acid which may be used to prepare the pharmaceutically acceptable salt is that which forms a non-toxic acid addition salt, *i.e.,* a salt containing pharmacologically acceptable anions such as the hydrochloride, hydroiodide, hydrobromide, nitrate, sulfate, bisulfate, phosphate, acetate, lactate, citrate, tartrate, succinate, maleate, fumarate, benzoate, para-toluenesulfonate, and the like.

In another aspect, the invention provides a pharmaceutical composition comprising a compound of the present invention and a pharmaceutically acceptable excipient, *e.g.,* a composition suitable for injection, such as for intravenous (IV) administration.

Pharmaceutically acceptable excipients include any and all diluents or other liquid vehicles, dispersion or suspension aids, surface active agents, isotonic agents, preservatives, lubricants and the like, as suited to the particular dosage form desired, *e.g.,* injection. General considerations in the formulation and/or manufacture of pharmaceutical compositions agents can be found, for example, in Remington's Pharmaceutical Sciences, Sixteenth Edition, E. W. Martin (Mack Publishing Co., Easton, Pa., 1980), and Remington: The Science and Practice of Pharmacy, 21st Edition (Lippincott Williams & Wilkins, 2005).

For example, injectable preparations, such as sterile injectable aqueous suspensions, can be formulated according to the known art using suitable dispersing or wetting agents and suspending agents. Exemplary excipients that can be employed include, but are not limited to, water, sterile saline or phosphate-buffered saline, or Ringer's solution.

In certain embodiments, the pharmaceutical composition further comprise a cyclodextrin derivative. The most common cyclodextrins are α-, β- and γ- cyclodextrins consisting of 6, 7 and 8 α-l ,4-linked glucose units, respectively, optionally comprising one or more substituents on the linked sugar moieties, which include, but are not limited to, substituted or unsubstituted methylated, hydroxyalkylated, acylated, and sulfoalkylether substitution. In certain embodiments, the cyclodextrin is a sulfoalkyl ether β-cyclodextrin, e.g., for example, sulfobutyl ether β-cyclodextrin, also known as Captisol^{®}. See, *e.g.,* U.S. 5,376,645. In certain embodiments, the composition comprises hexapropyl-β-cyclodextrin. In a more particular embodiment, the composition comprises hexapropyl-β-cyclodextrin (10-50% in water).

The injectable composition can be sterilized, for example, by filtration through a bacterial-retaining filter, or by incorporating sterilizing agents in the form of sterile solid compositions which can be dissolved or dispersed in sterile water or other sterile injectable medium prior to use.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, response of the individual patient, the severity of the patient's symptoms, and the like.

The compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include pre-filled, pre-measured ampules or syringes of the liquid compositions. In such compositions, the compound is usually a minor component (from about 0.1% to about 50% by weight or preferably from about 1% to about 40% by weight) with the remainder being various vehicles or carriers and processing aids helpful for forming the desired dosing form.

The compounds provided herein can be administered as the sole active agent, or they can be administered in combination with other active agents. In one aspect, the present invention provides a combination of a compound of the present invention and another pharmacologically active agent. Administration in combination can proceed by any technique apparent to those of skill in the art including, for example, separate, sequential, concurrent, and alternating administration.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with ordinary experimentation. General considerations in the formulation and/or manufacture of pharmaceutical compositions can be found, for example, in Remington: The Science and Practice of Pharmacy 21st ed., Lippincott Williams & Wilkins, 2005.

Described generally below, but not claimed herein, is a kit comprising a composition (e.g., a solid composition) comprising a deuterium-enriched compound of Formula (**II**): 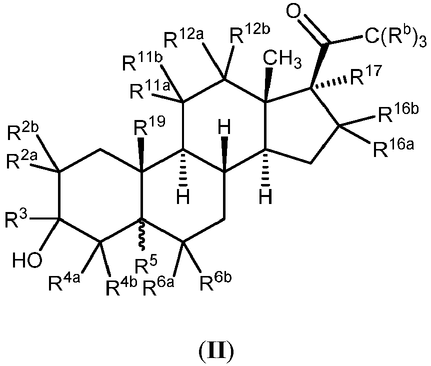 or a pharmaceutically acceptable salt thereof wherein: each of R^{2a}, R^{2b}, R^{4a}, R^{4b}, R⁵, R^{6a}, R^{6b}, R^{12a}, R^{12b,} R^{16a}, R^{16b}, R¹⁷, and R^{b} is independently hydrogen or deuterium; R³ is hydrogen, deuterium, or -C(R^{c})₃, wherein each R^{c} is independently hydrogen or deuterium; each of R^{11a} and R^{11b} is independently hydrogen or deuterium; or R^{11a} and R^{11b} are taken together to form an oxo (=O) group; and R¹⁹ is hydrogen, deuterium, or -C(R^{a})₃, wherein each R^{a} is independently hydrogen or deuterium; wherein at least one of R^{2a}, R^{2b}, R³, R^{4a}, R^{4b}, R⁵, R^{6a}, R^{6b}, R^{11a}, R^{11b}, R^{12a,} R^{12b,} R^{16a}, R^{16b}, R¹⁷, R¹⁹, R^{a}, R^{b} and R^{c} is deuterium.

In some aspects, R⁵ is deuterium.

In some aspects, R¹⁹ is deuterium or -CD₃.

In some aspects, at least one of R³, R^{4a}, R^{4b}, R⁵, R^{6a}, R^{6b}, R^{11a}, R^{11b}, R^{12a}, R^{12b,} R^{16a}, R^{16b}, R¹⁷, R^{a}, R^{b} and R^{c} is deuterium.

In some aspects, at least one of R^{2a}, R^{2b}, R³, R⁵, R^{6a}, R^{6b}, R^{11a}, R^{11b}, R^{12a}, R^{12b,} R^{16a}, R^{16b}, R¹⁷, R^{a}, R^{b} and R^{c} is deuterium.

In some aspects, at least one of R^{2a}, R^{2b}, R³, R^{4a}, R^{4b}, R⁵, R^{11a}, R^{11b}, R^{12a}, R^{12b,} R^{16a}, R^{16b}, R¹⁷, R^{a}, R^{b} and R^{c} is deuterium.

In some aspects, at least one of R^{2a}, R^{2b}, R³, R^{4a}, R^{4b}, R⁵, R^{6a}, R^{6b}, R^{12a}, R^{12b,} R^{16a}, R^{16b}, R¹⁷, R^{a}, R^{b} and R^{c} is deuterium.

In some aspects, at least one of R^{2a}, R^{2b}, R³, R^{4a}, R^{4b}, R⁵, R^{6a}, R^{6b}, R^{11a}, R^{11b}, R^{16a}, R^{16b}, R¹⁷, R^{a} , R^{b} and R^{c} is deuterium.

In some aspects, at least one of R^{2a}, R^{2b}, R³, R^{4a}, R^{4b}, R⁵, R^{6a}, R^{6b}, R^{11a}, R^{11b}, R^{12a}, R^{12b,} R¹⁷, R^{a} , R^{b} and R^{c} is deuterium.

In some aspects, at least one of R³, R⁵, R¹⁷, R^{a}, R^{b} and R^{c} is deuterium.

In some aspects, the compound of Formula (**II**) is a compound of Formula (**II-a**):

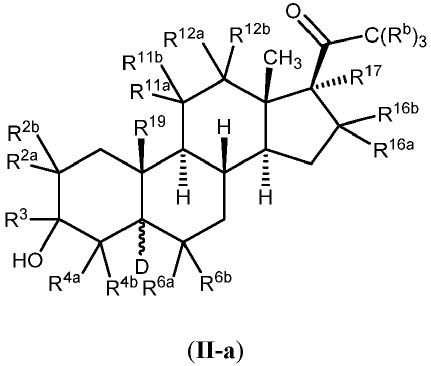

In some aspects, the compound of Formula (**II**) is a compound of Formula (**II-b**):

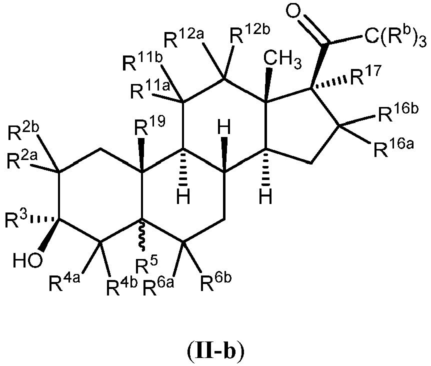

In some aspects, the compound of Formula (**II**) is a compound of Formula (**II-c**):

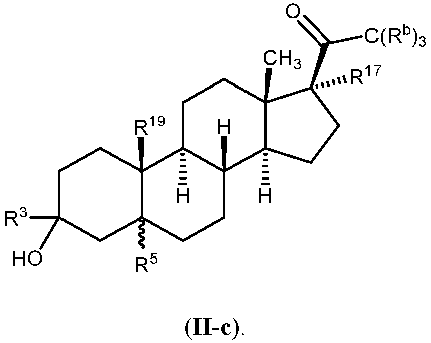

In some aspects, the compound of Formula **(II)** is a compound of Formula **(I):** 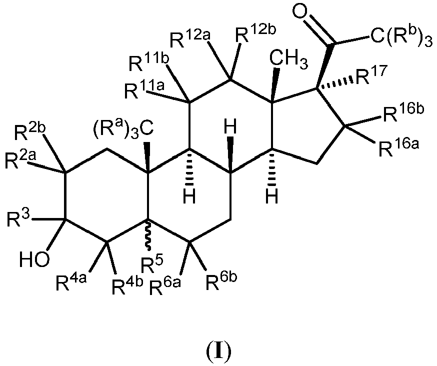 or a pharmaceutically acceptable salt thereof wherein: each of R^{2a}, R^{2b}, R^{4a}, R^{4b}, R⁵, R^{6a}, R^{6b}, R^{12a}, R^{12b,} R^{16a}, R^{16b}, R¹⁷, R^{a}, and R^{b} is independently hydrogen or deuterium; R³ is hydrogen, deuterium, or -C(R^{c})₃, wherein each R^{c} is independently hydrogen or deuterium; and each of R^{11a} and R^{11b} is independently hydrogen or deuterium; or R^{11a} and R^{11b} are taken together to form an oxo (=O) group; wherein at least one of R^{2a}, R^{2b}, R³, R^{4a}, R^{4b}, R⁵, R^{6a}, R^{6b}, R^{11a}, R^{11b}, R^{12a,} R^{12b}, R^{16a}, R^{16b}, R¹⁷, R^{a}, R^{b} and R^{c} is deuterium.

### Use in Methods of Treatment

The present invention, as defined in the appended claims, relates to a deuterium-enriched compound or a pharmaceutically-acceptable salt thereof, for use in methods of treatment, selected from the group consisting of: 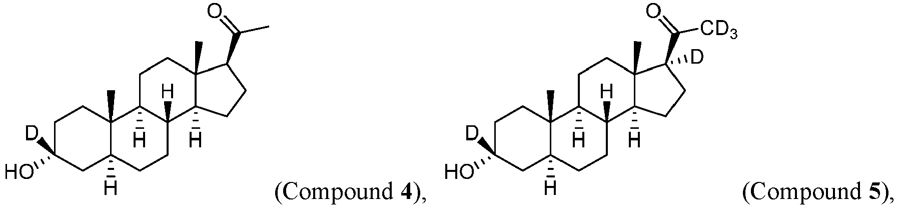 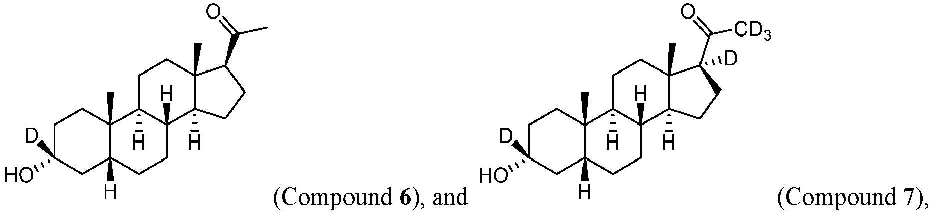 or a pharmaceutically acceptable salt thereof.

Generally described herein are deuterated neuroactive steroids designed, for example, to act as GABA modulators. The compounds of the present invention are envisioned to be useful as therapeutic agents for treating a CNS-related disorder (e.g., sleep disorder, a mood disorder (e.g., depression, for example post-partum depression), a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, or tinnitus) in a subject in need (*e.g.,* a subject with Rett syndrome, Fragile X syndrome, or Angelman syndrome). The compounds of the present invention are envisioned to be useful as therapeutic agents for the inducement of anesthesia and/or sedation in a subject.
The present invention, as defined in the appended claims, relates to a deuterium-enriched compound or a pharmaceutically-acceptable salt thereof, for use in a method of treating a mood disorder, depression, post-partum depression, or anxiety disorder, in a subject, comprising administering to the subject an effective amount of the compound selected from the group consisting of: 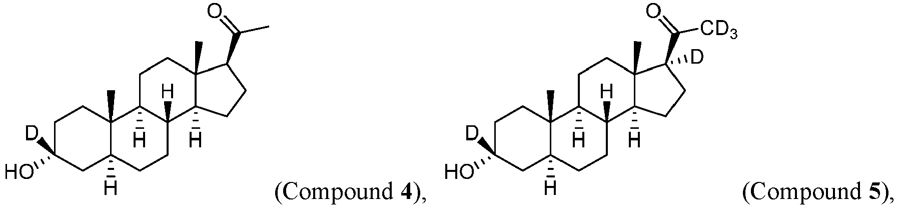 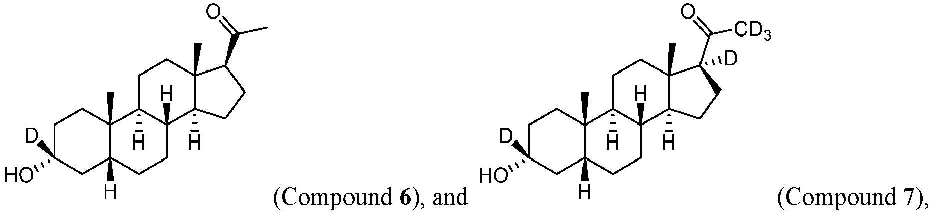 or a pharmaceutically acceptable salt thereof.

Described generally herein are deuterium-enriched compounds of Formulae (**II**), (**II-a**), (**II-b**), (**II-c**), **(I),** or a pharmaceutically-acceptable salt thereof, for use in a method of treating a mood disorder (e.g., depression, for example post-partum depression); or anxiety disorder in a subject, comprising administering to the subject an effective amount of the compound.

In some embodiments, the mood disorder is depression. In some embodiments, the depression is is postpartum depression.

In some embodiments, the administering is performed orally.

In some embodiments, the administering is performed parenterally. In some embodiments, the administering is performed intravenously. In some embodiments, the administering occurs by continuous intravenous infusion.

In some embodiments, the subject is a mammal. In some embodiments, the subject is a human. In some embodiments, the subject is a female. In some embodiments, the subject is an adult. In some embodiments, the subject is from 18 to 45 years of age.

In some embodiments, the subject is suffering from (*e.g.,* has been diagnosed with) postpartum depression (*e.g.,* severe postpartum depression). In some embodiments, the subject has experienced a Major Depressive Episode in the postpartum period.

In some embodiments, the period begins within the first 4 weeks following delivery of a baby.
The present invention, as defined in the appended claims, relates to a compound or a pharmaceutically-acceptable salt thereof, for use in a method of inducing sedation and/or anesthesia in a subject, comprising administering to the subject an effective amount of the compound selected from the group consisting of: 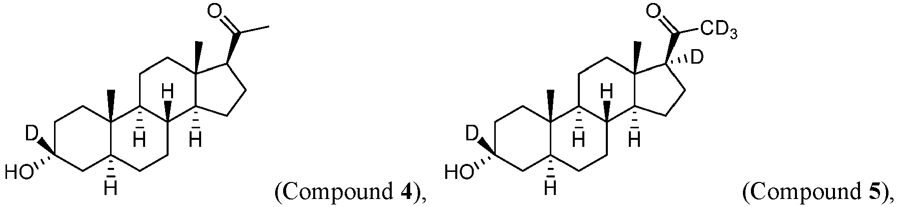 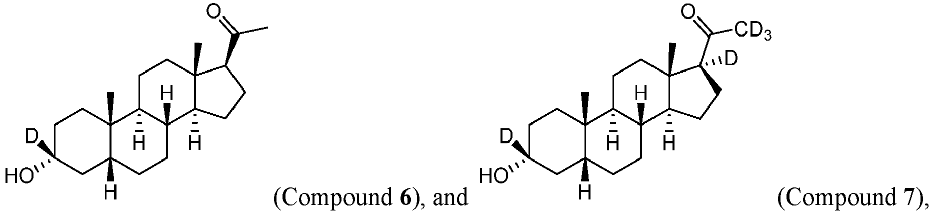 or a pharmaceutically acceptable salt thereof.

Described generally herein are compounds of Formulae **(II), (II-a), (11-b), (II-c), (I),** or a pharmaceutically-acceptable salt thereof, for use in a method of inducing sedation and/or anesthesia in a subject, comprising administering to the subject an effective amount of the compound.

.The present invention, as defined in the appended claims, relates to a compound or a pharmaceutically-acceptable salt thereof, or a pharmaceutical composition of the compound, for use as a medicament, the compound selected from the group consisting of: 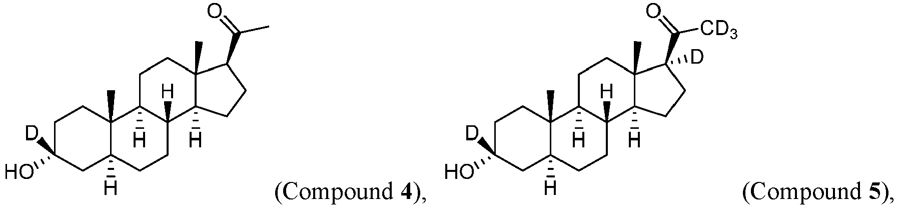 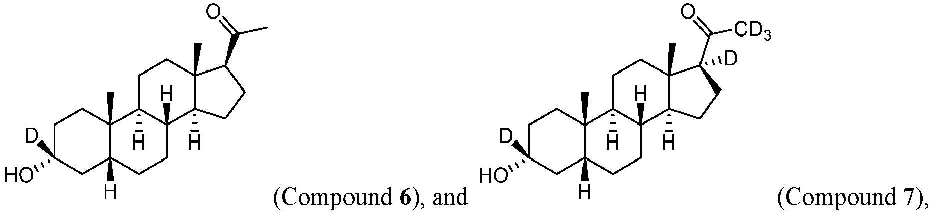 or a pharmaceutically acceptable salt thereof.

Described generally herein are compounds of Formulae **(II), (II-a), (II-b), (II-c), (I),** or a pharmaceutically-acceptable salt thereof, for use as a medicament.

The subject may experience sedation and/or anesthesia within one hour of administration. The subject may experience sedation and/or anesthesia instantaneously.

In some embodiments, the compound is administered by intravenous administration. In some embodiments, the compound is administered chronically. In some embodiments, the compound is administered in combination with another therapeutic agent.
The present invention, as defined in the appended claims, relates to a compound or a pharmaceutically-acceptable salt thereof, for use in a method for treating seizure in a subject, comprising administering to the subject an effective amount of the compound selected from the group consisting of: 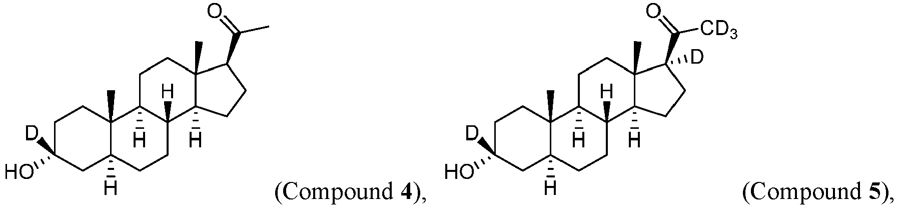 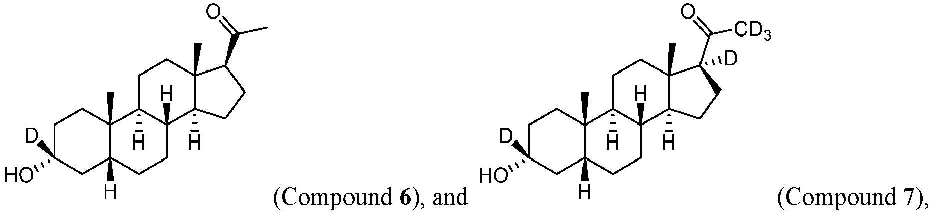 or a pharmaceutically acceptable salt thereof.
Described generally herein are compounds of Formulae **(II),** (**II-a**), **(II-b),** (**II-c**), **(I),** or a pharmaceutically-acceptable salt thereof, for use in a method for treating seizure in a subject, comprising administering to the subject an effective amount of the compound.

The present invention, as defined in the appended claims, relates to a compound or a pharmaceutically-acceptable salt thereof, for use in a method for treating epilepsy in a subject, the method comprising administering to the subject an effective amount of the compound selected from the group consisting of: 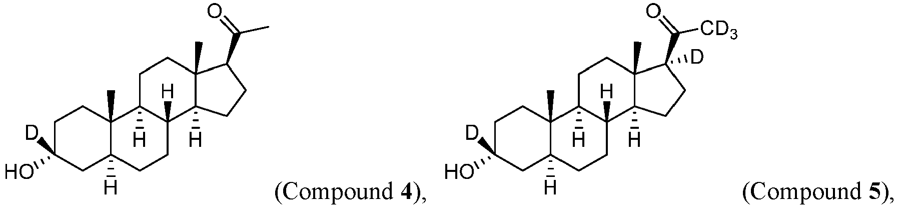 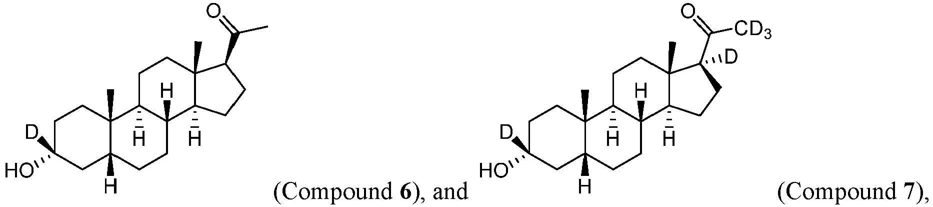 or a pharmaceutically acceptable salt thereof.
Described generally herein are compounds of Formulae **(II),** (**II-a**), **(II-b),** (**II-c**), **(I),** or a pharmaceutically-acceptable salt thereof, for use in a method for treating epilepsy in a subject, the method comprising administering to the subject an effective amount of the compound.

The present invention, as defined in the appended claims, relates to a compound or a pharmaceutically-acceptable salt thereof, for use in a method for treating status epilepticus (SE) in a subject, the method comprising administering to the subject an effective amount of the compound selected from the group consisting of: 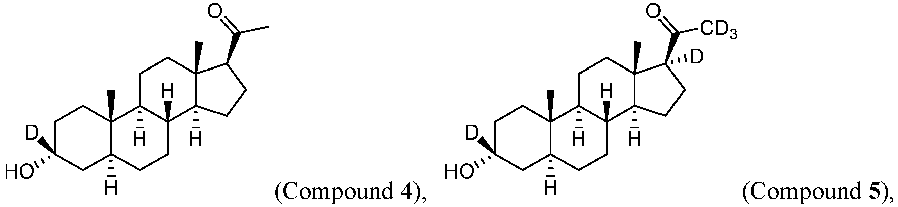 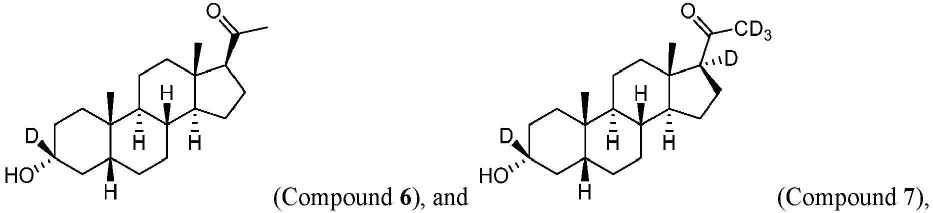 or a pharmaceutically acceptable salt thereof.
Described generally herein are compounds of Formulae **(II), (II-a), (II-b), (II-c), (I),** or a pharmaceutically-acceptable salt thereof, for use in a method for treating status epilepticus (SE) in a subject, the method comprising administering to the subject an effective amount of the compound.

The present invention, as defined in the appended claims, relates to a compound or a pharmaceutically-acceptable salt thereof, for use in a method of treating a human subject suffering from tremor, the method comprising administering a therapeutically effective amount of the compound selected from the group consisting of: 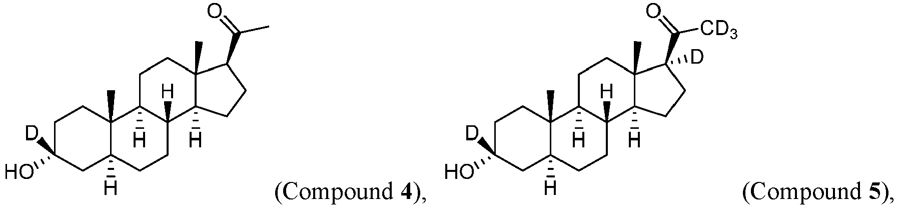 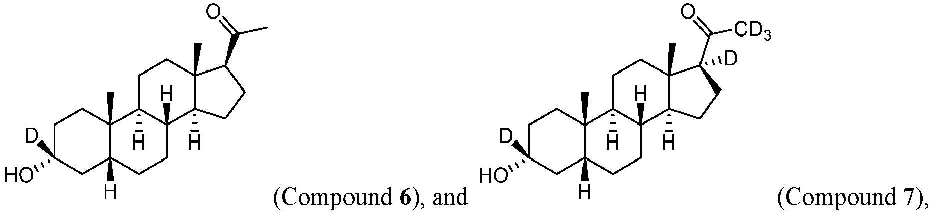 or a pharmaceutically acceptable salt thereof.
Described generally herein are compounds of Formulae **(II),** (**II-a**), **(II-b),** (**II-c**), **(I),** or a pharmaceutically-acceptable salt thereof, for use in a method of treating a human subject suffering from tremor, the method comprising administering to the subject an effective amount of the compound.

In some embodiments, the tremor is essential tremor.

Described generally herein is a deuterium-enriched compound of Formula (**II**), (**II-a**), (**II-b**), (**II-c**), or **(I),** or a pharmaceutically acceptable salt thereof, for use in a method for treating a disorder in a subject, wherein the subject has a decreased steroid level relative to a reference standard (e.g., a decreased level of allopregnanolone), comprising administering to the subject an effective amount of the deuterium-enriched compound.

Described generally herein is a deuterium-enriched compound of Formula (**II**), (**II-a**), (**II-b**), (**II-c**), or **(I),** or a pharmaceutically acceptable salt thereof, for use in a method for treating disorders related to GABA function in a subject in need thereof, the method comprising administering to the subject a therapeutically effective amount of the compound, the pharmaceutically acceptable salt thereof, or pharmaceutical composition of one of the deuterium-enriched compounds.

The present invention, as defined in the appended claims, relates to a deuterium-enriched compound or a pharmaceutically-acceptable salt thereof, for use in a method for treating a CNS-related disorder in a subject in need thereof, comprising administering to the subject an effective amount of the deuterium-enriched compound selected from the group consisting of: 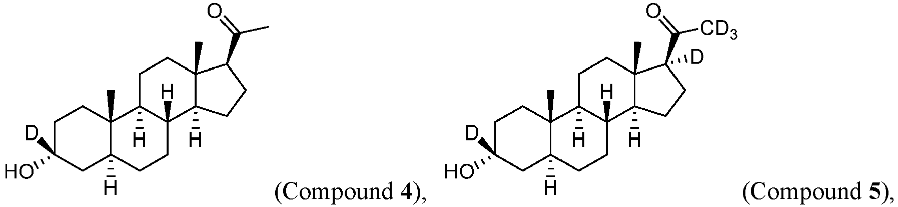 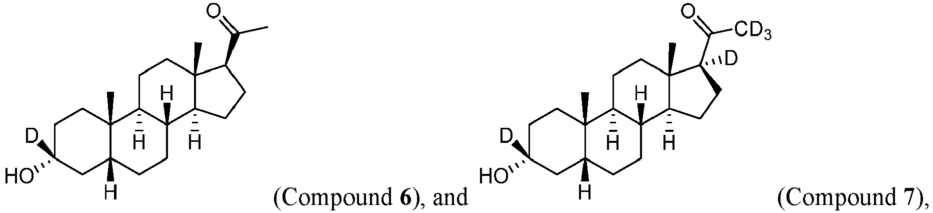 or a pharmaceutically acceptable salt thereof.

Described generally herein are deuterium-enriched compounds of Formulae (**II**), (**II-a**), (**II-b**), (**II-c**), **(I), (I-a), (I-b),** or a pharmaceutically-acceptable salt thereof, for use in a method for treating a CNS-related disorder in a subject in need thereof, the method comprising administering to the subject an effective amount of the compound:

In some embodiments, the CNS-related disorder is a sleep disorder, a mood disorder, a schizophrenia spectrum disorder, a convulsive disorder, a disorder of memory and/or cognition, a movement disorder (e.g., tremor, for example essential tremor), a personality disorder, autism spectrum disorder, pain, traumatic brain injury, a vascular disease, a substance abuse disorder and/or withdrawal syndrome, or tinnitus.

In some embodiments, the subject is a subject with Rett syndrome, Fragile X syndrome, or Angelman syndrome.

In some embodiments, the subject is a mammal. In some embodiments, the subject is a human.

In some embodiments, the administering is performed parenterally. In some embodiments, the administering is performed intravenously. In some embodiments, the administering is performed intramuscularly. In some embodiments, the administering is performed orally. In some embodiments, the the administering is performed chronically.

In some embodiments, the compound is administered in combination with another therapeutic agent.

In some embodiments in any of the foregoing, the compound is not:

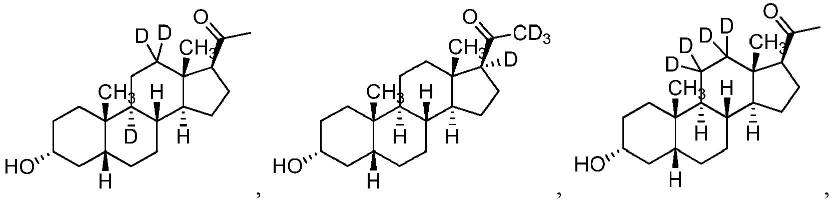

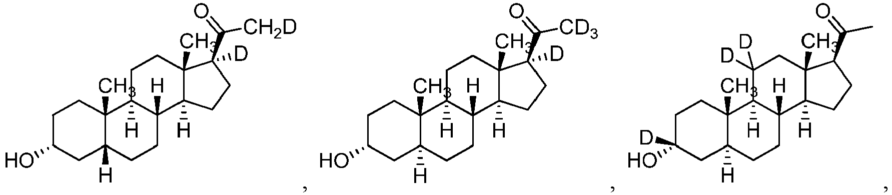

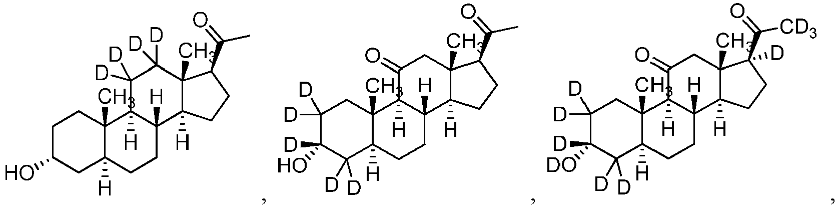

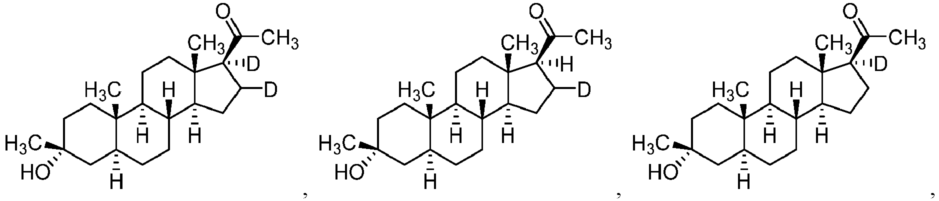

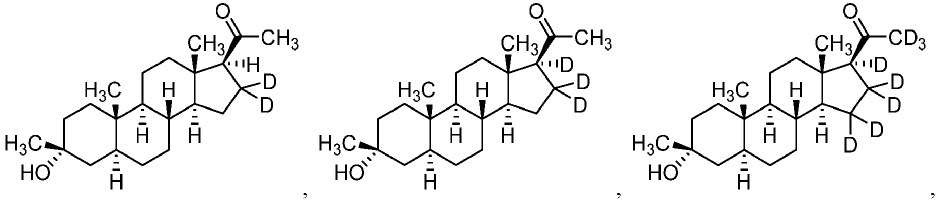

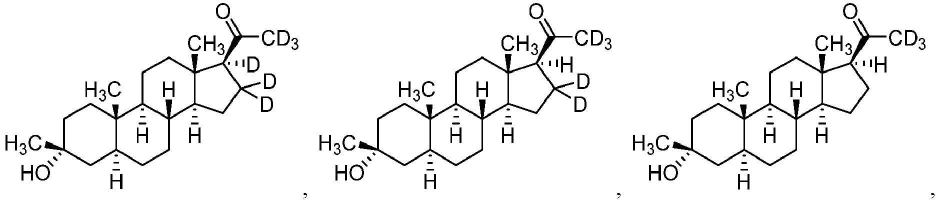

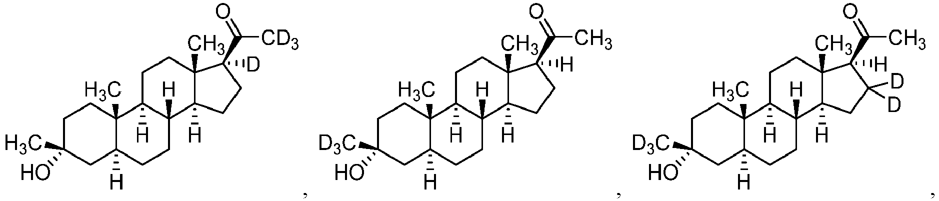

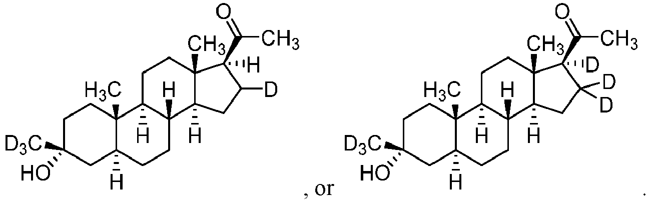

Earlier studies (see, *e.g.,* Gee et al., European Journal of Pharmacology, 136:419-423 (1987)) demonstrated that certain 3α-hydroxylated steroids are orders of magnitude more potent as modulators of the GABA receptor complex (GRC) than others had reported (see, *e.g.,* Majewska et al., Science 232:1004-1007 (1986); Harrison et al., J Pharmacol. Exp. Ther. 241:346-353 (1987)). Majewska *et al.* and Harrison *et al.* taught that 3α-hydroxylated-5-reduced steroids are only capable of much lower levels of effectiveness. *In vitro* and *in vivo* experimental data have now demonstrated that the high potency of these steroids allows them to be therapeutically useful in the modulation of brain excitability via the GRC (see, *e.g.,* Gee et al., European Journal of Pharmacology, 136:419-423 (1987); Wieland et al., Psychopharmacology 118(1):65-71 (1995)).

Various synthetic steroids have also been prepared as neuroactive steroids. See, for example, U.S. Patent 5,232,917, which discloses neuroactive steroid compounds useful in treating stress, anxiety, insomnia, seizure disorders, and mood disorders, that are amenable to GRC-active agents, such as depression, in a therapeutically beneficial manner. Furthermore, it has been previously demonstrated that these steroids interact at a unique site on the GRC which is distinct from other known sites of interaction (*e.g.,* barbiturates, benzodiazepines, and GABA) where therapeutically beneficial effects on stress, anxiety, sleep, mood disorders and seizure disorders have been previously elicited (see, *e.g.,* Gee, K.W. and Yamamura, H.I., "Benzodiazepines and Barbiturates: Drugs for the Treatment of Anxiety, Insomnia and Seizure Disorders," in Central Nervous System Disorders, Horvell, ed., Marcel-Dekker, New York (1985), pp. 123-147; Lloyd, K.G. and Morselli, P.L., "Psychopharmacology of GABAergic Drugs," in Psychopharmacology: The Third Generation of Progress, H.Y. Meltzer, ed., Raven Press, N.Y. (1987), pp. 183-195; and Gee et al., European Journal of Pharmacology, 136:419-423 (1987). These compounds are desirable for their duration, potency, and oral activity (along with other forms of administration).

Compounds of the present invention, as described herein, are generally designed to modulate GABA function, and therefore to act as neuroactive steroids for the treatment and prevention of CNS-related conditions in a subject. Modulation, as used herein, refers to the inhibition or potentiation of GABA receptor function. Accordingly, the compounds of the present invention and pharmaceutical compositions thereof are for use in preventing and/or treating CNS conditions in mammals including humans and non-human mammals. Thus, and as stated earlier, the present invention includes within its scope, and extends to, the compounds of the present invention for use in methods of treatment.

Exemplary CNS conditions related to GABA-modulation include, but are not limited to, sleep disorders [*e.g.,* insomnia], mood disorders [*e.g.,* depression, dysthymic disorder *(e.g.,* mild depression), bipolar disorder (*e.g.,* I and/or II), anxiety disorders (*e.g.,* generalized anxiety disorder (GAD), social anxiety disorder), stress, post-traumatic stress disorder (PTSD), compulsive disorders (*e.g.,* obsessive compulsive disorder (OCD))], schizophrenia spectrum disorders [*e.g.,* schizophrenia, schizoaffective disorder], convulsive disorders [*e.g.,* epilepsy (*e.g.,* status epilepticus (SE)), seizures], disorders of memory and/or cognition [*e.g.,* attention disorders (*e.g.,* attention deficit hyperactivity disorder (ADHD)), dementia (*e.g.,* Alzheimer's type dementia, Lewis body type dementia, vascular type dementia], movement disorders [*e.g*., Huntington's disease, Parkinson's disease], personality disorders [*e.g*., anti-social personality disorder, obsessive compulsive personality disorder], autism spectrum disorders (ASD) [*e.g.,* autism, monogenetic causes of autism such as synaptophathy's, *e.g.*, Rett syndrome, Fragile X syndrome, Angelman syndrome], pain [*e.g*., neuropathic pain, injury related pain syndromes, acute pain, chronic pain], traumatic brain injury (TBI), vascular diseases [*e*.*g*., stroke, ischemia, vascular malformations], substance abuse disorders and/or withdrawal syndromes [*e.g*., addition to opiates, cocaine, and/or alcohol], and tinnitus.

In yet another aspect, provided is a combination of a compound of the present invention and another pharmacologically active agent. The compounds provided herein can be administered as the sole active agent or they can be administered in combination with other agents. Administration in combination can proceed by any technique apparent to those of skill in the art including, for example, separate, sequential, concurrent and alternating administration.

In another aspect, provided is a compound of the present invention for use in a method of treating or preventing brain excitability in a subject susceptible to or afflicted with a condition associated with brain excitability.

In yet another aspect, provided is a compound, or composition thereof, of the present invention for use in a method of treating or preventing stress or anxiety in a subject.

In yet another aspect, provided is a compound of the present invention for use in a method of alleviating or preventing seizure activity in a subject.

In yet another aspect, provided is a compound of the present invention, or a composition thereof, for use in a method of alleviating or preventing insomnia in a subject.

In yet another aspect, provided is a compound of the present invention, for use in a method of inducing sleep and maintaining substantially the level of REM sleep that is found in normal sleep, wherein substantial rebound insomnia is not induced

In yet another aspect, provided is a compound of the present invention for use in a method of alleviating or preventing PMS or PND in a subject.

In yet another aspect, provided is a compound of the present invention for use in a method of inducing anesthesia in a subject.

In yet another aspect, provided is a compound of the present invention for use in a method of cognition enhancement or treating memory disorder in a subject. In certain embodiments, the disorder is Alzheimer's disease. In certain embodiments, the disorder is Rett syndrome.

In yet another aspect, provided is a compound of the present invention for use in a method of treating attention disorders in a subject. In certain embodiments, the attention disorder is ADHD.

In certain embodiments, the compound is administered to the subject chronically. In certain embodiments, the compound is administered to the subject orally, subcutaneously, intramuscularly, or intravenously.

### Neuroendocrine Disorders and Dysfunction

Provided herein are methods that can be used for treating neuroendocrine disorders and dysfunction. As used herein, "neuroendocrine disorder" or "neuroendocrine dysfunction" refers to a variety of conditions caused by imbalances in the body's hormone production directly related to the brain. Neuroendocrine disorders involve interactions between the nervous system and the endocrine system. Because the hypothalamus and the pituitary gland are two areas of the brain that regulate the production of hormones, damage to the hypothalamus or pituitary gland, e.g., by traumatic brain injury, may impact the production of hormones and other neuroendocrine functions of the brain.

Symptoms of neuroendocrine disorder include, but are not limited to, behavioral, emotional, and sleep-related symptoms, symptoms related to reproductive function, and somatic symptoms; including but not limited to fatigue, poor memory, anxiety, depression, weight gain or loss, emotional lability, lack of concentration, attention difficulties, loss of lipido, infertility, amenorrhea, loss of muscle mass, increased belly body fat, low blood pressure, reduced heart rate, hair loss, anemia, constipation, cold intolerance, and dry skin.

### Neurodegenerative Diseases and Disorders

The term "neurodegenerative disease" includes diseases and disorders that are associated with the progressive loss of structure or function of neurons, or death of neurons. Neurodegenerative diseases and disorders include, but are not limited to, Alzheimer's disease (including the associated symptoms of mild, moderate, or severe cognitive impairment); amyotrophic lateral sclerosis (ALS); anoxic and ischemic injuries; ataxia and convulsion (including for the treatment and prevention and prevention of seizures that are caused by schizoaffective disorder or by drugs used to treat schizophrenia); benign forgetfulness; brain edema; cerebellar ataxia including McLeod neuroacanthocytosis syndrome (MLS); closed head injury; coma; contusive injuries (e.g., spinal cord injury and head injury); dementias including multi-infarct dementia and senile dementia; disturbances of consciousness; Down syndrome; drug-induced or medication-induced Parkinsonism (such as neuroleptic-induced acute akathisia, acute dystonia, Parkinsonism, or tardive dyskinesia, neuroleptic malignant syndrome, or medication-induced postural tremor); epilepsy; fragile X syndrome; Gilles de la Tourette's syndrome; head trauma; hearing impairment and loss; Huntington's disease; Lennox syndrome; levodopa-induced dyskinesia; mental retardation; movement disorders including akinesias and akinetic (rigid) syndromes (including basal ganglia calcification, corticobasal degeneration, multiple system atrophy, Parkinsonism-ALS dementia complex, Parkinson's disease, postencephalitic parkinsonism, and progressively supranuclear palsy); muscular spasms and disorders associated with muscular spasticity or weakness including chorea (such as benign hereditary chorea, drug-induced chorea, hemiballism, Huntington's disease, neuroacanthocytosis, Sydenham's chorea, and symptomatic chorea), dyskinesia (including tics such as complex tics, simple tics, and symptomatic tics), myoclonus (including generalized myoclonus and focal cyloclonus), tremor (such as rest tremor, postural tremor, and intention tremor) and dystonia (including axial dystonia, dystonic writer's cramp, hemiplegic dystonia, paroxysmal dystonia, and focal dystonia such as blepharospasm, oromandibular dystonia, and spasmodic dysphonia and torticollis); neuronal damage including ocular damage, retinopathy or macular degeneration of the eye; neurotoxic injury which follows cerebral stroke, thromboembolic stroke, hemorrhagic stroke, cerebral ischemia, cerebral vasospasm, hypoglycemia, amnesia, hypoxia, anoxia, perinatal asphyxia and cardiac arrest; Parkinson's disease; seizure; status epilecticus; stroke; tinnitus; tubular sclerosis, and viral infection induced neurodegeneration (e.g., caused by acquired immunodeficiency syndrome (AIDS) and encephalopathies). Neurodegenerative diseases also include, but are not limited to, neurotoxic injury which follows cerebral stroke, thromboembolic stroke, hemorrhagic stroke, cerebral ischemia, cerebral vasospasm, hypoglycemia, amnesia, hypoxia, anoxia, perinatal asphyxia and cardiac arrest. Methods of treating or preventing a neurodegenerative disease also include treating or preventing loss of neuronal function characteristic of neurodegenerative disorder.

### Mood disorders

**Clinical depression** is also known as major depression, major depressive disorder (MDD), unipolar depression, unipolar disorder, and recurrent depression, and refers to a mental disorder characterized by pervasive and persistent low mood that is accompanied by low self-esteem and loss of interest or pleasure in normally enjoyable activities. Some people with clinical depression have trouble sleeping, lose weight, and generally feel agitated and irritable. Clinical depression affects how an individual feels, thinks, and behaves and may lead to a variety of emotional and physical problems. Individuals with clinical depression may have trouble doing day-to-day activities and make an individual feel as if life is not worth living.

**Postnatal depression (PND)** is also referred to as postpartum depression (PPD), and refers to a type of clinical depression that affects women after childbirth. Symptoms can include sadness, fatigue, changes in sleeping and eating habits, reduced sexual desire, crying episodes, anxiety, and irritability.

**Atypical depression (AD)** is characterized by mood reactivity (e.g., paradoxical anhedonia) and positivity, significant weight gain or increased appetite. Patients suffering from AD also may have excessive sleep or somnolence (hypersomnia), a sensation of limb heaviness, and significant social impairment as a consequence of hypersensitivity to perceived interpersonal rejection.

**Melancholic depression** is characterized by loss of pleasure (anhedonia) in most or all activities, failures to react to pleasurable stimuli, depressed mood more pronounced than that of grief or loss, excessive weight loss, or excessive guilt.

**Psychotic major depression (PMD)** or psychotic depression refers to a major depressive episode, in particular of melancholic nature, where the individual experiences psychotic symptoms such as delusions and hallucinations.

**Catatonic depression** refers to major depression involving disturbances of motor behavior and other symptoms. An individual may become mute and stuporose, and either is immobile or exhibits purposeless or bizarre movements.

**Seasonal affective disorder (SAD)** refers to a type of seasonal depression wherein an individual has seasonal patterns of depressive episodes coming on in the fall or winter.

**Dysthymia** refers to a condition related to unipolar depression, where the same physical and cognitive problems are evident. They are not as severe and tend to last longer (*e.g.,* at least 2 years).

**Double depression** refers to fairly depressed mood (dysthymia) that lasts for at least 2 years and is punctuated by periods of major depression.

**Depressive Personality Disorder (DPD)** refers to a personality disorder with depressive features.

**Recurrent Brief Depression (RBD)** refers to a condition in which individuals have depressive episodes about once per month, each episode lasting 2 weeks or less and typically less than 2-3 days.

**Minor depressive disorder** or minor depression refers to a depression in which at least 2 symptoms are present for 2 weeks.

**Bipolar disorder or manic depressive disorder** causes extreme mood swings that include emotional highs (mania or hypomania) and lows (depression). During periods of mania the individual may feel or act abnormally happy, energetic, or irritable. They often make poorly thought out decisions with little regard to the consequnces. The need for sleep is usually reduced. During periods of depression there may be crying, poor eye contact with others, and a negative outlook on life. The risk of suicide among those with the disorder is high at greater than 6% over 20 years, while self harm occurs in 30-40%. Other mental health issues such as anxiety disorder and substance use disorder are commonly associated with bipolar disorder.

**Depression caused by chronic medical conditions** refers to depression caused by chronic medical conditions such as cancer or chronic pain, chemotherapy, chronic stress.

**Treatment-resistant depression** refers to a condition where the individuals have been treated for depression, but the symptoms do not improve. For example, antidepressants or physchological counseling (psychotherapy) do not ease depression symptoms for individuals with treatment-resistant depression. In some cases, individuals with treatment-resistant depression improve symptoms, but come back. **Refractory depression** occurs in patients suffering from depression who are resistant to standard pharmacological treatments, including tricyclic antidepressants, MAOIs, SSRIs, and double and triple uptake inhibitors and/or anxiolytic drugs, as well as non-pharmacological treatments (e.g., psychotherapy, electroconvulsive therapy, vagus nerve stimulation and/or transcranial magnetic stimulation).

**Suicidality, suicidal ideation, suicidal behavior** refers to the tendency of an individual to commit suicide. Suicidal ideation concerns thoughts about or an unusual preoccupation with suicide. The range of suicidal ideation varies greatly, from e.g., fleeting thoughts to extensive thoughts, detailed planning, role playing, incomplete attempts. Symptoms include talking about suicide, getting the means to commit suicide, withdrawing from social contact, being preoccupied with death, feeling trapped or hopeless about a situation, increasing use of alcohol or drugs, doing risky or self-destructive things, saying goodbye to people as if they won't be seen again.

**Symptoms** of depression include persistent anxious or sad feelings, feelings of helplessness, hopelessness, pessimism, worthlessness, low energy, restlessness, difficulty sleeping, sleeplessness, irritability, fatigue, motor challenges, loss of interest in pleasurable activities or hobbies, loss of concentration, loss of energy, poor self-esteem, absence of positive thoughts or plans, excessive sleeping, overeating, appetite loss, insomnia, self-harm, thoughts of suicide, and suicide attempts. The presence, severity, frequency, and duration of symptoms may vary on a case to case basis. Symptoms of depression, and relief of the same, may be ascertained by a physician or psychologist (e.g., by a mental state examination).

### Anxiety Disorders

Described herein are compounds for use in methods for treating anxiety disorders. Anxiety disorder is a blanket term covering several different forms of abnormal and pathological fear and anxiety. Current psychiatric diagnostic criteria recognize a wide variety of anxiety disorders.

Generalized anxiety disorder is a common chronic disorder characterized by long-lasting anxiety that is not focused on any one object or situation. Those suffering from generalized anxiety experience non-specific persistent fear and worry and become overly concerned with everyday matters. Generalized anxiety disorder is the most common anxiety disorder to affect older adults.

In **panic disorder,** a person suffers from brief attacks of intense terror and apprehension, often marked by trembling, shaking, confusion, dizziness, nausea, difficulty breathing. These panic attacks, defined by the APA as fear or discomfort that abruptly arises and peaks in less than ten minutes, can last for several hours and can be triggered by stress, fear, or even exercise; although the specific cause is not always apparent. In addition to recurrent unexpected panic attacks, a diagnosis of panic disorder also requires that said attacks have chronic consequences: either worry over the attacks' potential implications, persistent fear of future attacks, or significant changes in behavior related to the attacks. Accordingly, those suffering from panic disorder experience symptoms even outside of specific panic episodes. Often, normal changes in heartbeat are noticed by a panic sufferer, leading them to think something is wrong with their heart or they are about to have another panic attack. In some cases, a heightened awareness (hypervigilance) of body functioning occurs during panic attacks, wherein any perceived physiological change is interpreted as a possible life threatening illness (i.e. extreme hypochondriasis).

**Obsessive compulsive disorder** is a type of anxiety disorder primarily characterized by repetitive obsessions (distressing, persistent, and intrusive thoughts or images) and compulsions (urges to perform specific acts or rituals). The OCD thought pattern may be likened to superstitions insofar as it involves a belief in a causative relationship where, in reality, one does not exist. Often the process is entirely illogical; for example, the compulsion of walking in a certain pattern may be employed to alleviate the obsession of impending harm. And in many cases, the compulsion is entirely inexplicable, simply an urge to complete a ritual triggered by nervousness. In a minority of cases, sufferers of OCD may only experience obsessions, with no overt compulsions; a much smaller number of sufferers experience only compulsions.

The single largest category of anxiety disorders is that of **phobia,** which includes all cases in which fear and anxiety is triggered by a specific stimulus or situation. Sufferers typically anticipate terrifying consequences from encountering the object of their fear, which can be anything from an animal to a location to a bodily fluid.

**Post-traumatic stress disorder** or **PTSD** is an anxiety disorder which results from a traumatic experience. Post-traumatic stress can result from an extreme situation, such as combat, rape, hostage situations, or even serious accident. It can also result from long term (chronic) exposure to a severe stressor, for example soldiers who endure individual battles but cannot cope with continuous combat. Common symptoms include flashbacks, avoidant behaviors, and depression.

### Epilepsy

Epilepsy is a brain disorder characterized by repeated seizures over time. Types of epilepsy can include, but are not limited to generalized epilepsy, e.g., childhood absence epilepsy, juvenile nyoclonic epilepsy, epilepsy with grand-mal seizures on awakening, West syndrome, Lennox-Gastaut syndrome, partial epilepsy, e.g., temporal lobe epilepsy, frontal lobe epilepsy, benign focal epilepsy of childhood.

### Status epilepticus (SE)

Status epilepticus (SE) can include, *e.g.,* convulsive status epilepticus, *e.g.,* early status epilepticus, established status epilepticus, refractory status epilepticus, super-refractory status epilepticus; non-convulsive status epilepticus, e.g., generalized status epilepticus, complex partial status epilepticus; generalized periodic epileptiform discharges; and periodic lateralized epileptiform discharges. Convulsive status epilepticus is characterized by the presence of convulsive status epileptic seizures, and can include early status epilepticus, established status epilepticus, refractory status epilepticus, super-refractory status epilepticus. Early status epilepticus is treated with a first line therapy. Established status epilepticus is characterized by status epileptic seizures which persist despite treatment with a first line therapy, and a second line therapy is administered. Refractory status epilepticus is characterized by status epileptic seizures which persist despite treatment with a first line and a second line therapy, and a general anesthetic is generally administered. Super refractory status epilepticus is characterized by status epileptic seizures which persist despite treatment with a first line therapy, a second line therapy, and a general anesthetic for 24 hours or more.

Non-convulsive status epilepticus can include, *e.g.,* focal non-convulsive status epilepticus, *e.g.,* complex partial non-convulsive status epilepticus, simple partial non-convulsive status epilepticus, subtle non-convulsive status epilepticus; generalized non-convulsive status epilepticus, *e.g.,* late onset absence non-convulsive status epilepticus, atypical absence non-convulsive status epilepticus, or typical absence non-convulsive status epilepticus.

Compositions described herein can also be administered as a prophylactic to a subject having a CNS disorder *e.g.,* a traumatic brain injury, status epilepticus, *e.g.,* convulsive status epilepticus, *e.g.,* early status epilepticus, established status epilepticus, refractory status epilepticus, super-refractory status epilepticus; non-convulsive status epilepticus, *e.g.,* generalized status epilepticus, complex partial status epilepticus; generalized periodic epileptiform discharges; and periodic lateralized epileptiform discharges; prior to the onset of a seizure.

### Seizure

A seizure is the physical findings or changes in behavior that occur after an episode of abnormal electrical activity in the brain. The term "seizure" is often used interchangeably with "convulsion." Convulsions are when a person's body shakes rapidly and uncontrollably. During convulsions, the person's muscles contract and relax repeatedly.

Based on the type of behavior and brain activity, seizures are divided into two broad categories: generalized and partial (also called local or focal). Classifying the type of seizure helps doctors diagnose whether or not a patient has epilepsy.

Generalized seizures are produced by electrical impulses from throughout the entire brain, whereas partial seizures are produced (at least initially) by electrical impulses in a relatively small part of the brain. The part of the brain generating the seizures is sometimes called the focus.

There are six types of generalized seizures. The most common and dramatic, and therefore the most well known, is the generalized convulsion, also called the grand-mal seizure. In this type of seizure, the patient loses consciousness and usually collapses. The loss of consciousness is followed by generalized body stiffening (called the "tonic" phase of the seizure) for 30 to 60 seconds, then by violent jerking (the "clonic" phase) for 30 to 60 seconds, after which the patient goes into a deep sleep (the "postictal" or after-seizure phase). During grand-mal seizures, injuries and accidents may occur, such as tongue biting and urinary incontinence.

Absence seizures cause a short loss of consciousness (just a few seconds) with few or no symptoms. The patient, most often a child, typically interrupts an activity and stares blankly. These seizures begin and end abruptly and may occur several times a day. Patients are usually not aware that they are having a seizure, except that they may be aware of "losing time."

Myoclonic seizures consist of sporadic jerks, usually on both sides of the body. Patients sometimes describe the jerks as brief electrical shocks. When violent, these seizures may result in dropping or involuntarily throwing objects.

Clonic seizures are repetitive, rhythmic jerks that involve both sides of the body at the same time.

Tonic seizures are characterized by stiffening of the muscles.

Atonic seizures consist of a sudden and general loss of muscle tone, particularly in the arms and legs, which often results in a fall.
Seizures described herein can include epileptic seizures; acute repetitive seizures; cluster seizures; continuous seizures; unremitting seizures; prolonged seizures; recurrent seizures; status epilepticus seizures, *e.g.,* refractory convulsive status epilepticus, non-convulsive status epilepticus seizures; refractory seizures; myoclonic seizures; tonic seizures; tonic-clonic seizures; simple partial seizures; complex partial seizures; secondarily generalized seizures; atypical absence seizures; absence seizures; atonic seizures; benign Rolandic seizures; febrile seizures; emotional seizures; focal seizures; gelastic seizures; generalized onset seizures; infantile spasms; Jacksonian seizures; massive bilateral myoclonus seizures; multifocal seizures; neonatal onset seizures; nocturnal seizures; occipital lobe seizures; post traumatic seizures; subtle seizures; Sylvan seizures; visual reflex seizures; or withdrawal seizures.

### Movement Disorders

Also described herein are compounds for use in methods for treating a movement disorder. As used herein, "movement disorders" refers to a variety of diseases and disorders that are associated with hyperkinetic movement disorders and related abnormalities in muscle control. Exemplary movement disorders include, but are not limited to, Parkinson's disease and parkinsonism (defined particularly by bradykinesia), dystonia, chorea and Huntington's disease, ataxia, tremor (e.g., essential tremor), myoclonus and startle, tics and Tourette syndrome, Restless legs syndrome, stiff person syndrome, and gait disorders.

### Tremor

The compounds described herein can be used in methods to treat tremor, for example cerebellar tremor or intention tremor, dystonic tremor, essential tremor, orthostatic tremor, parkinsonian tremor, physiological tremor, psychogenic tremor, or rubral tremor. Tremor includes hereditary, degenerative, and idiopathic disorders such as Wilson's disease, Parkinson's disease, and essential tremor, respectively; metabolic diseases (e.g., thyoidparathyroid-, liver disease and hypoglycemia); peripheral neuropathies (associated with Charcot-Marie-Tooth, Roussy-Levy, diabetes mellitus, complex regional pain syndrome); toxins (nicotine, mercury, lead, CO, Manganese, arsenic, toluene); drug-induced (narcoleptics, tricyclics, lithium, cocaine, alcohol, adrenaline, bronchodilators, theophylline, caffeine, steroids, valproate, amiodarone, thyroid hormones, vincristine); and psychogenic disorders. Clinical tremor can be classified into physiologic tremor, enhanced physiologic tremor, essential tremor syndromes (including classical essential tremor, primary orthostatic tremor, and task- and position-specific tremor), dystonic tremor, parkinsonian tremor, cerebellar tremor, Holmes' tremor (i.e., rubral tremor), palatal tremor, neuropathic tremor, toxic or drug-induced tremor, and psychogenic tremor.

**Tremor** is an involuntary, at times rhythmic, muscle contraction and relaxation that can involve oscillations or twitching of one or more body parts (*e.g*., hands, arms, eyes, face, head, vocal folds, trunk, legs).

**Cerebellar tremor** or **intention tremor** is a slow, broad tremor of the extremities that occurs after a purposeful movement. Cerebellar tremor is caused by lesions in or damage to the cerebellum resulting from, *e.g.,* tumor, stroke, disease (*e.g.,* multiple sclerosis, an inherited degenerative disorder).

**Dystonic tremor** occurs in individuals affected by dystonia, a movement disorder in which sustained involuntary muscle contractions cause twisting and repetitive motions and/or painful and abnormal postures or positions. Dystonic tremor may affect any muscle in the body. Dystonic tremors occurs irregularly and often can be relieved by complete rest.

**Essential tremor** or benign essential tremor is the most common type of tremor. Essential tremor may be mild and nonprogressive in some, and may be slowly progressive, starting on one side of the body but affect both sides within 3 years. The hands are most often affected, but the head, voice, tongue, legs, and trunk may also be involved. Tremor frequency may decrease as the person ages, but severity may increase. Heightened emotion, stress, fever, physical exhaustion, or low blood sugar may trigger tremors and/or increase their severity. Symptoms generally evolve over time and can be both visible and persistent following onset.

**Orthostatic tremor** is characterized by fast (*e.g*., greater than 12 Hz) rhythmic muscle contractions that occurs in the legs and trunk immediately after standing. Cramps are felt in the thighs and legs and the patient may shake uncontrollably when asked to stand in one spot. Orthostatic tremor may occurs in patients with essential tremor.

**Parkinsonian tremor** is caused by damage to structures within the brain that control movement. Parkinsonian tremor is often a precursor to Parkinson's disease and is typically seen as a "pill-rolling" action of the hands that may also affect the chin, lips, legs, and trunk. Onset of parkinsonian tremor typically begins after age 60. Movement starts in one limb or on one side of the body and can progress to include the other side.

**Physiological tremor** can occur in normal individuals and have no clinical significance. It can be seen in all voluntary muscle groups. Physiological tremor can be caused by certain drugs, alcohol withdrawl, or medical conditions including an overactive thyroid and hypoglycemia. The tremor classically has a frequency of about 10 Hz.

**Psychogenic tremor** or hysterical tremor can occur at rest or during postural or kinetic movement. Patient with psychogenic tremor may have a conversion disorder or another psychiatric disease.

**Rubral tremor** is characterized by coarse slow tremor which can be present at rest, at posture, and with intention. The tremor is associated with conditions that affect the red nucleus in the midbrain, classical unusual strokes.

**Parkinson's Disease** affects nerve cells in the brain that produce dopamine. Symptoms include muscle rigidity, tremors, and changes in speech and gait. **Parkinsonism** is characterized by tremor, bradykinesia, rigidity, and postural instability. Parkinsonism shares symptons found in Parkinson's Disease, but is a symptom complex rather than a progressive neurodegenerative disease.

**Dystonia** is a movement disorder characterized by sustained or intermittent muscle contractions causing abnormal, often repetitive movements or postures. Dystonic movements can be patterned, twisting, and may be tremulous. Dystonia is often initiated or worsened by voluntary action and associated with overflow muscle activation.

**Chorea** is a neurological disorder characterized by jerky involuntary movements typically affecting the shoulders, hips, and face. **Huntington's Disease** is an inherited disease that causes nerve cells in the brain to waste away. Symptoms include uncontrolled movements, clumsiness, and balance problems. Huntington's disease can hinder walk, talk, and swallowing.

**Ataxia** refers to the loss of full control of bodily movements, and may affect the fingers, hands, arms, legs, body, speech, and eye movements.

**Myloclonus and Startle** is a response to a sudden and unexpected stimulus, which can be acoustic, tactile, visual, or vestibular.

**Tics** are an involuntary movement usually onset suddenly, brief, repetitive, but nonrhythmical, typically imitating normal behavior and often occurring out of a background of normal activity. Tics can be classified as motor or vocal, motor tics associated with movements while vocal tics associated with sound. Tics can be characterized as simple or complex. For example simple motor tics involve only a few muscles restricted to a specific body part. **Tourette Syndrome** is an inherited neuropsychiatric disorder with onset in childhood, characterized by multiple motor tics and at least one vocal tic.

**Restless Legs Syndrome** is a neurologic sensorimotor disorder characterized by an overwhelming urge to move the legs when at rest.

**Stiff Person Syndrome** is a progressive movement disorder characterized by involuntary painful spasms and rigidity of muscles, usually involving the lower back and legs. Stiff-legged gait with exaggerated lumbar hyperlordosis typically results. Characteristic abnormality on EMG recordings with continuous motor unit activity of the paraspinal axial muscles is typically observed. Variants include "stiff-limb syndrome" producing focal stiffness typically affecting distal legs and feet.

**Gait disorders** refer to an abnormalitiy in the manner or style of walking, which results from neuromuscular, arthritic, or other body changes. Gait is classified according to the system responsible for abnormal locomotion, and include hemiplegic gait, diplegic gait, neuropathic gait, myopathic gait, parkinsonian gait, choreiform gait, ataxic gait, and sensory gait.

### Anesthesia /Sedation

Anesthesia is a pharmacologically induced and reversible state of amnesia, analgesia, loss of responsiveness, loss of skeletal muscle reflexes, decreased stress response, or all of these simultaneously. These effects can be obtained from a single drug which alone provides the correct combination of effects, or occasionally with a combination of drugs (*e.g*., hypnotics, sedatives, paralytics, analgesics) to achieve very specific combinations of results. Anesthesia allows patients to undergo surgery and other procedures without the distress and pain they would otherwise experience.

Sedation is the reduction of irritability or agitation by administration of a pharmacological agent, generally to facilitate a medical procedure or diagnostic procedure.

Sedation and analgesia include a continuum of states of consciousness ranging from minimal sedation (anxiolysis) to general anesthesia.

Minimal sedation is also known as anxiolysis. Minimal sedation is a drug-induced state during which the patient responds normally to verbal commands. Cognitive function and coordination may be impaired. Ventilatory and cardiovascular functions are typically unaffected.

Moderate sedation/analgesia (conscious sedation) is a drug-induced depression of consciousness during which the patient responds purposefully to verbal command, either alone or accompanied by light tactile stimulation. No interventions are usually necessary to maintain a patent airway. Spontaneous ventilation is typically adequate. Cardiovascular function is usually maintained.

Deep sedation/analgesia is a drug-induced depression of consciousness during which the patient cannot be easily aroused, but responds purposefully (not a reflex withdrawal from a painful stimulus) following repeated or painful stimulation. Independent ventilatory function may be impaired and the patient may require assistance to maintain a patent airway. Spontaneous ventilation may be inadequate. Cardiovascular function is usually maintained.

General anesthesia is a drug-induced loss of consciousness during which the patient is not arousable, even to painful stimuli. The ability to maintain independent ventilatory function is often impaired and assistance is often required to maintain a patent airway. Positive pressure ventilation may be required due to depressed spontaneous ventilation or drug-induced depression of neuromuscular function. Cardiovascular function may be impaired.

Sedation in the intensive care unit (ICU) allows the depression of patients' awareness of the environment and reduction of their response to external stimulation. It can play a role in the care of the critically ill patient, and encompasses a wide spectrum of symptom control that will vary between patients, and among individuals throughout the course of their illnesses. Heavy sedation in critical care has been used to facilitate endotracheal tube tolerance and ventilator synchronization, often with neuromuscular blocking agents.

In some embodiments, sedation (*e.g*., long-term sedation, continuous sedation) is induced and maintained in the ICU for a prolonged period of time (*e.g.*, 1 day, 2 days, 3 days, 5 days, 1 week, 2 week, 3 weeks, 1 month, 2 months). Long-term sedation agents may have long duration of action. Sedation agents in the ICU may have short elimination half-life.

Procedural sedation and analgesia, also referred to as conscious sedation, is a technique of administering sedatives or dissociative agents with or without analgesics to induce a state that allows a subject to tolerate unpleasant procedures while maintaining cardiorespiratory function.

### Examples

In order that the invention described herein may be more fully understood, the following examples are set forth. The synthetic and biological examples described in this application are offered to illustrate the compounds, pharmaceutical compositions, and methods provided herein and are not to be construed in any way as limiting their scope.

### Abbreviations

THF: tetrahydrofuran; Na₂SO₄: sodium sulfate; PE: petroleum ether; D₂O: deuterium oxide; EtOAc: ethylacetate; BHT: 2,6-di-t-butyl-p-cresol (butylated hydroxytoluene); MAD: methyl aluminum bis(2,6-di-t-butyl-4-methylphenoxide); DCM: dichloromethane; Ac₂O: acetic anhydride; TsOH: *p*-Toluenesulfonic acid; NBS: N-bromosuccinimide; Ph₃PEtBr: ethyltriphenylphosphonium bromide; tBuOK: potassium tert-butoxide; TMSOTf: trimethylsilyl trifluoromethanesulfonate; TBSOTf: tert-butyldimethylsilyl trifluoromethanesulfonate; PCC: pyridinium chlorochromate; TBAF: tetra-n-butylammonium fluoride; K-selectride: potassium tri-sec-butylborohydride; LiAlH(t-BuO)₃: Lithium tri-t-butoxy aluminum hydride; DEAD: diethyl azodicarboxylate; DIAD: diisopropyl azodicarboxylate; PPh₃: triphenylphosphine; MTBE: methyl tert-butyl ether; py: pyridine.

### Example 1. Synthesis of Compounds 1 and 2. (Reference)

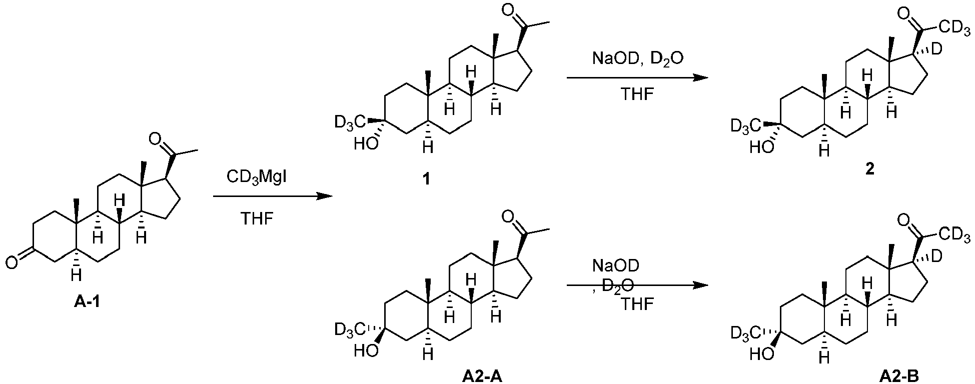

**Step 1. Synthesis of Compound 1.** To a suspension of Mg (68.7 mg, 2.83 mmol) and I₂ (1 mg) in ether (2 mL) was added a solution of CD₃I (410 mg, 2.83 mmol) in ether (5 mL) dropwise under N₂ at 25°C. The reaction temperature was raised to 35°C and stirred at 35°C for 2 hrs. To a solution of **A-1** (200 mg, 0.63 mmol) in THF (10 mL) was added fresh CD₃MgI (2.83 mmol in 7 mL of ether) dropwise at -70°C and stirred at -70°C for 1 h. The mixture was cooled to 0°C and stirred for another 1 h. NH₄Cl (20 mL, Sat. aq) was added and the mixture was extracted with EtOAc (50 mL). The organic layer was separated, dried over Na₂SO₄, concentrated under vacuum, and purified by silica gel (PE/DCM/EtOAc = 2:1:0.1) to give **Compound 1** (50 mg, 11%) and **A2-A** (10 mg, 2%) as an off white solid. **Compound 1: ¹H NMR** (400 MHz, CDCl₃) δ 2.57-2.50 (m, 1H), 2.20-2.11 (m, 1H), 2.11 (s, 3H), 2.05-1.95 (m, 1H), 1.75-1.10 (m, 19H), 1.05-0.75 (m, 2H), 0.75 (s, 3H), 0.60 (s, 3H). **LCMS** Rt = 1.264 min in 2.0 min chromatography, 30-90 AB, MS ESI calcd. for C₂₂H₃₂D₃O [M+H-H₂O]⁺ 318, found 318. **HRMS** MS ESI calcd. for C₂₂H₃₄D₃O₂ [M+H]⁺ 336.2976, found 336.2976; calcd. for C₂₂H₃₂D₃O [M+H-H₂O]⁺ 318.2871, found 318.2865. **A2-A: ¹H NMR** (400 MHz, CDCl₃) δ 2.57-2.50 (m, 1H), 2.20-2.11 (m, 1H), 2.11 (s, 3H), 2.05-1.95 (m, 1H), 1.75-0.85(m, 20H), 0.81 (s, 3H), 0.80-0.70 (m, 1H), 0.60 (s, 3H). **LCMS** Rt = 1.189 min in 2.0 min chromatography, 30-90 AB, MS ESI calcd. for C₂₂H₃₂D₃O [M+H-H₂O]⁺ 318, found 318. **HRMS** ESI calcd. for C₂₂H₃₄D₃O₂ [M+H]⁺ 336.2976, found 336.2973; calcd. for C₂₂H₃₂D₃O [M+H-H₂O]⁺ 318.2871, found 318.2859.

**Synthesis of Compound 2.** To a solution of **Compound 1** (48 mg) in THF (2 mL) was added D₂O (0.5 g) and NaOD (0.125 M in D₂O, 0.5 mL). The mixture was stirred at 60°C for 2 days. The pH was adjusted to 7 with a solution of CF₃COOD/D₂O. The mixture was extracted with EtOAc (3 x 2 mL). The combined organic layers were dried over Na₂SO₄, filtered, and concentrated to give 70 mg crude **Compound 2** as an off white solid. The reaction was repeated to convert the small remained 17-H undeuterated material. To a solution of crude **Compound 2** (70 mg, some 17-H still remained) in THF (2 mL) was added D₂O (0.5 g) and NaOD (0.125 M in D₂O, 0.1 mL). The mixture was stirred at 60°C for 16 hrs. The pH was adjusted to 7 with a solution of CF₃COOD/D₂O. The mixture was extracted with EtOAc (3 x 2 mL). The combined organic layers were dried over Na₂SO₄, filtered, and concentrated to give crude product, which was triturated form MeCN (1 mL) to give **Compound 2** (27.1 mg, 56%) as white solid. **¹H NMR** (400 MHz, CDCl₃) δ 2.20-2.11 (m, 1H), 2.05-1.95 (m, 1H), 1.75-1.10 (m, 19H), 1.05-0.75 (m, 2H), 0.75 (s, 3H), 0.60 (s, 3H). **LCMS** Rt = 1.253 min in 2.0 min chromatography, 30-90 AB, MS ESI calcd. for C₂₂H₂₈D₇O [M+H-H₂O]⁺ 322, found 322. **HRMS** ESI calcd. for C₂₂H₃₀D₇O₂ [M+H]⁺ 340.3227, found 340.3221; calcd. for C₂₂H₂₈D₇O [M+H-H₂O]⁺ 322.3122, found 322.3113.

**Synthesis of A2-B.** To a solution **of A2-A** (8 mg) in THF (2 mL) was added D₂O (0.5 g) and NaOD (0.125 M in D₂O, 0.5 mL). The mixture was stirred at 60°C for 2 days. The pH was adjusted to 7 with a solution of CF₃COOD/D₂O. The mixture was extracted with EtOAc (3 x 2 mL).The combined organic layers were dried over Na₂SO₄, filtered, and concentrated to give 20 mg crude **A2-B** as white solid. The reaction was then repeated to convert the small remained 17-H undeuterated material. To a solution of crude **A2-B** (20 mg, some 17-H still remained) in THF (2 mL) was added D₂O (0.5 g) and NaOD (0.125 M in D₂O, 0.1 mL). The mixture was stirred at 60°C for 16 hrs. The pH was adjusted to 7 with a solution of CF₃COOD/D₂O. The mixture was extracted with EtOAc (3 x 2 mL). The combined organic layers were dried over Na₂SO₄, filtered, and concentrated to give crude product, which was triturated form MeCN (1 mL) to give **A2-B** (1.3 mg, 16%) as an off white solid. **¹H NMR** (400 MHz, CDCl₃) δ 2.20-2.11 (m, 1H), 2.05-1.95 (m, 1H), 1.75-0.85(m, 20H), 0.81 (s, 3H), 0.80-0.70 (m, 1H), 0.60 (s, 3H). **LCMS** Rt = 1.181 min in 2.0 min chromatography, 30-90 AB, MS ESI calcd. for C₂₂H₂₈D₇O [M+H-H₂O]⁺ 322, found 322. **HRMS** ESI calcd. for C₂₂H₃₀D₇O₂ [M+H]⁺ 340.3227, found 340.3226; calcd. for C₂₂H₂₈D₇O [M+H-H₂O]⁺ 322.3122, found 322.3112.

### Example 2. Synthesis of Compound 3. (Reference)

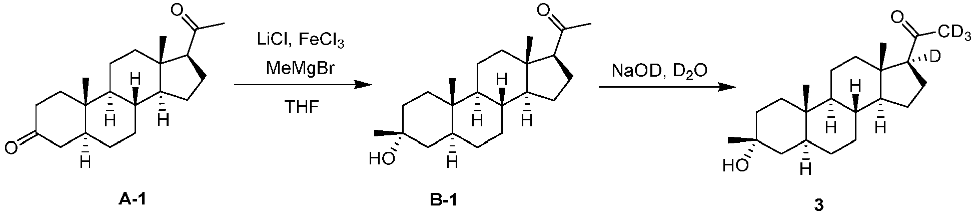

**Step 1. Synthesis of Compound B-1.** Anhydrous THF (900 mL) was cooled to 10°C and anhydrous LiCl (16.7 g, 396 mmol) was added in one portion. The mixture was stirred for 30 mins to obtain a clear solution. Anhydrous FeCl₃ (33.5 g, 207 mmol) was added in one portion. The resulting mixture was stirred for additional 30 mins. The reaction mixture was cooled to - 35°C and MeMgBr (3 M in diethyl ether, 252 mL, 756 mmol) was added dropwise maintaining the internal temperature between -35°C and -30°C. The mixture was stirred for 30 mins at -30°C. **A-1** (60 g, 189 mmol) was added in one portion. The internal temperature was allowed to -20°C and kept between -15°C and -20°C for 2 hrs. The reaction mixture was poured into ice-cooled aqueous HCl (1 M, 1000 mL). The mixture was extracted with EtOAc (2 x 800 mL). The combined organic layer was washed with water (1000 mL), aqueous NaOH (10%, 2 x 500 mL) and brine (800 mL), dried over anhydrous Na₂SO₄, filtered and concentrated. The residue was triturated from EtOAc and PE to afford crude product, which was re-crystallized from DCM and PE to give **B-1** (51 g, 81%) as an off white solid. **¹H NMR** (400 MHz, CDCl₃) δ 2.53 (t, *J* = 8.8 Hz, 1H), 2.20-2.08 (m, 4H), 2.04-1.96 (m, 1H), 1.71-1.61 (m, 4H), 1.55-1.34 (m, 7H), 1.32-1.12 (m, 11H), 1.02-0.89 (m, 1H), 0.84-0.72 (m, 4H), 0.60 (s, 3H).

**Step 2. Synthesis of Compound 3.** To a solution of **B-1** (300 µmol, 100 mg) in THF (1 mL) was added D₂O (1 mL) and NaOD (0.125 M, 62.5 µmol, 0.5 mL). The reaction was stirred at 60°C for 24 hrs. After cooling, the pH was adjusted to 7 with a solution of CF₃COOD/D₂O. The mixture was extracted with EtOAc (3 x 2 mL). The combined organic layers were dried over Na₂SO₄, filtered, and concentrated. The crude residue was triturated from CH₃CN (3 mL), filtered, and dried to afford give **Compound 3** (50 mg, 48%) as an off white powder. **¹H NMR** (400 MHz, CDCl₃) δ 2.14 (t, *J* = 11.2 Hz, 1H), 1.99 (dt, *J* = 12.0, 3.6 Hz, 1H), 1.70-1.10 (m, 22H), 1.02-0.86 (m, 1H), 0.84-0.70 (m, 4H), 0.60 (s, 3H). **LCMS** Rt = 1.056 min in 2.0 min chromatography, 30-90 AB, MS ESI calcd. for C₂₂H₃₁D₄O₁ [M+H-H₂O]⁺ 319, found 319. **HRMS** MS calcd. for C₂₂H₃₃D₄O₂ [M+H]⁺ 337.3045, found 337.3028.

### Example 3. Synthesis of Compound 4.

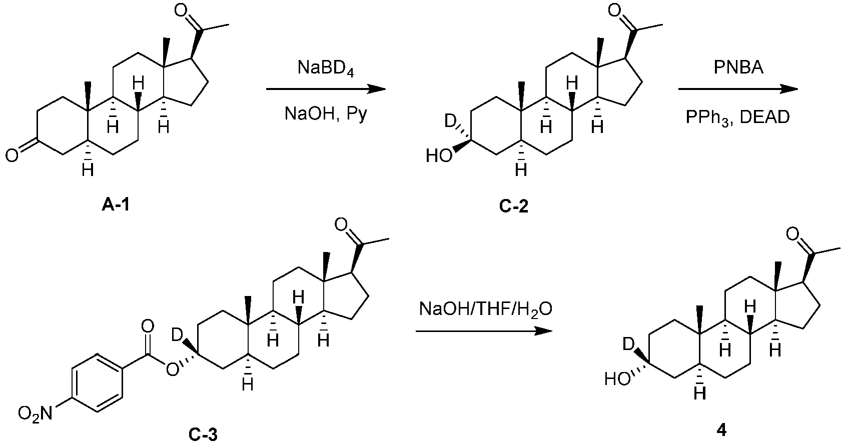

**Step 1. Synthesis of C-2.** To a solution **of A-1** (1 g, 3.15 mmol) in MeOH (74 mL) was added an ice-cold solution of NaOH (88 mg, 2.19 mmol) in water (0.8 mL) and MeOH (24 mL) at 0°C, followed by addition of an ice-cold solution of NaBD₄ (0.2 g, 4.77 mmol) in pyridine (22 mL) at 0°C. The reaction was stirred for 30 min at 5-10°C. The reaction was quenched with 5% HCl to adjust the pH to 5. The resulting mixture was extracted with EtOAc (2 x 10 mL). The combined organic layers were washed with brine (10 mL), dried over Na₂SO₄, filtered and concentrated to give the crude product which was purified on silica gel (PE/EtOAc=10/1) to give **C-2** (350 mg, 35%) as an off white solid. **¹H NMR** (400 MHz, CDCl₃) δ 2.52 (t, *J* = 8.8Hz, 1H), 2.20-2.15 (m, 1H), 2.11 (s, 3H), 2.05-1.95 (m, 1H), 1.70-1.58 (m, 7H), 1.40-1.14 (m, 11H), 1.05-0.85 (m, 2H), 0.80 (s, 3H), 0.70-0.65 (m, 1H), 0.60 (s, 3H).

**Step 2. Synthesis of C-3.** To a solution of **C-2** (350 mg, 1.09 mmol) in THF (10 mL) was added 4-nitrobenzoic acid (272mg, 1.63 mmol) and PPh₃ (857 mg, 3.27mmol) at 20°C under N₂. A solution of DEAD (569 mg, 3.27 mmol) in THF (2 mL) was added and the mixture was stirred at 0°C under N₂ for 2 hrs. The reaction mixture was warmed to 20°C and stirred for an additional 15 hrs. The reaction was quenched with H₂O (5 mL), extracted with EtOAc (2 x 10 mL). The combined organic layers were washed with brine (5 mL), dried over Na₂SO₄, filtered, and concentrated under vacuum. The residue was purified by silica gel chromatography (PE/EtOAc = 20/1) to give **C-3** (300 mg, 58%) as an off white solid. **¹H NMR** (400 MHz, CDCl₃) δ 8.31 (d, *J* = 8.8 Hz, 2H), 8.22 (d, *J* = 8.8 Hz, 2H), 2.53 (t, *J* = 8.8 Hz, 2H), 2.25-2.15 (m, 1H), 2.12 (s, 3H), 2.05-2.00 (m, 1H), 1.73-1.61 (m, 8H), 1.44-1.22 (m, 9H), 1.00-0.88 (m, 1H), 0.85-0.70 (m, 4H), 0.62 (s, 3H).

**Step 3. Synthesis of Compound 4.** To a solution of **C-3** (500 mg, 1.06 mmol) in THF (5 mL) was added aq. NaOH (3 mL 15%) at 20°C. The reaction mixture was stirred at 60°C for 15 hrs. The mixture was cooled to 0°C.The mixture was acidified to pH = 8 by HCl (1 M). The mixture was extracted with EtOAc (2 x 10 mL).The combined organic layer was washed with brine (5 mL), dried over Na₂SO₄, filtered, and concentrated. The residue was purified by silica gel chromatography (PE/EtOAc = 20/1) to give **Compound 4** (330 mg, 97%) as an off white solid. **¹H NMR** (400 MHz, CDCl₃) δ 2.53 (t, *J* = 9.2 Hz, 1H), 2.17-2.16 (m, 1H), 2.11 (s, 3H), 2.00-1.95 (m, 1H), 1.65-1.60 (m, 6H), 1.52-1.35 (m, 13H), 1.00-0.90 (m, 1H), 0.85-0.80 (m, 1H), 0.77 (s, 3H), 0.60 (s, 3H). **LCMS** Rt = 1.093 min in 2.0 min chromatography, 30-90 AB,MS ESI calcd. for C₂₁H₃₂DO [M+H-H₂O]⁺ 302, found 302. **HRMS** ESI calcd. for C₂₁H₃₄DO₂ [M+H]⁺ 320.2694, found 320.2680.

### Example 4. Synthesis of Compound 5.

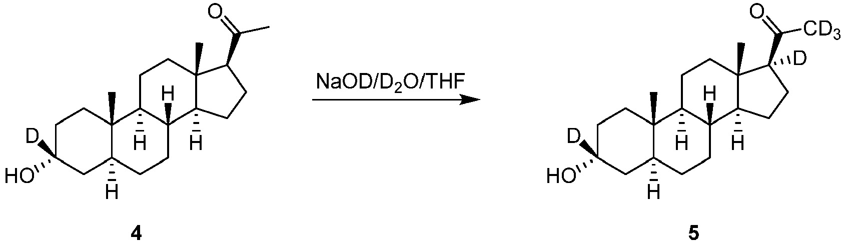

**Synthesis of Compound 5.** To a solution of **Compound 4** (50 mg, 156 µmol) in THF (0.5 mL) and D₂O (1 mL) was added NaOD (0.2 mL, 15% in D₂O) at 20°C. The reaction mixture was stirred at 60°C for 15 hrs. The mixture was neutralized by CF₃COOD (5% in D₂O) at 20°C. The mixture was extracted with EtOAc (2 x 2 mL). The combined organic layer was dried over Na₂SO₄, filtered, and concentrated to give the product (50 mg) as an off white solid. The reaction was repeated to convert the small remained 17-H undeuterated material. To the crude mixture of Compound 4 and 5 (50 mg) in THF (0.5 mL) and D₂O (1.0 mL) was added NaOD (0.2 mL, 15% in D₂O) at 20°C. The reaction mixture was stirred at 60°C for 15 hrs. The mixture was neutralized and worked up was conducted as described previously to give the desired **Compound 5** (48 mg, 96%) as an off white solid. **¹H NMR** (400 MHz, CDCl₃) δ 2.17-2.07 (m, 1H), 2.00-1.97 (m, 1H), 1.69-1.62 (m, 6H), 1.52-1.21 (m, 13H), 1.00-0.90 (m, 1H), 0.85-0.80 (m, 1H), 0.79 (s, 3H), 0.77 (s, 3H). **LCMS** Rt = 1.098 min in 2.0 min chromatography, 30-90 AB, MS ESI calcd. for C₂₁H₂₈D₅O [M+H-H₂O]⁺ 306, found 306. **HRMS** MS ESI calcd. for C₂₁H₃₀D₅O₂ [M+H]⁺ 324.2945, found 324.2936.

### Example 5. Synthesis of Compound 6.

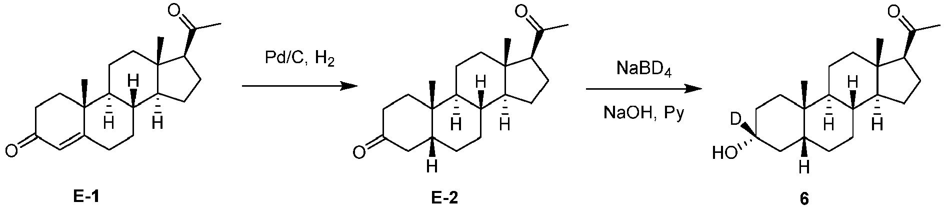

**Step 1. Synthesis of E-2.** To a solution **of E-1,** (20 g, 63.6 mmol) in EtOAc (200mL) was added Pd/C (10%, wet, 2 g) under N₂. The suspension was degassed under vacuum and purged with H₂ several times. The mixture was stirred under H₂ (103 kPa (15 psi)) at 15°C for 1 hr. The reaction mixture was filtered and the filtrate was concentrated to provide the crude product, which was purified by trituration to give **E-2** (15 g, 75%) as an off white solid. **¹H** (400 MHz, CDCl₃) δ 2.74-2.68 (m, 1H), 2.49-2.53 (m, 1H), 2.35-1.09 (m, 24H), 1.02 (s, 3H), 0.64 (s, 3H).

**Step 2. Synthesis of Compound 6.** To an ice cold solution of NaOH (189 mg, 4.73 mmol) in H₂O (1.6 mL) and MeOH (47.5 mL) was added an ice cold solution of **E-2** (2 g, 6.31 mmol) in MeOH (150 mL), followed by addition of an ice cold solution of NaBD₄ (343 mg, 8.20 mmol) in pyridine (43.5 mL). The reaction mixture was stirred at 0°C for 10 mins. The mixture was acidified to pH = 6 with HCl solution (5%). The mixture was extracted with EtOAc (3 x 100 mL). The combined organic layers were washed with saturated NaHCO₃ (60 mL), brine (60 mL), dried over Na₂SO₄, filtered, and concentrated under vacuum to provide a residue, which was purified by silica gel chromatography with (PE/EtOAc = 10:1 - 3:1) to afford **Compound 6** (0.9 g, 45%) as an off white solid. **¹H NMR** (400 MHz, CDCl₃) δ 2.55-2.51 (m, 1H), 2.18-2.09 (m, 4H), 2.03-1.97 (m, 1H), 1.91-1.09 (m, 20H), 1.05-0.86 (m, 4H), 0.59 (s, 3H). **LCMS** Rt = 1.282 min in 2 min chromatography, 10-80 AB, MS ESI calcd. for C₂₁H₃₂DO [M+H-H₂O]⁺ 302, found 302. **HRMS** MS ESI calcd. for C₂₁H₃₄DO₂ [M+H]⁺ 320.2694, found 320.2678.

### Example 6. Synthesis of Compound 7.

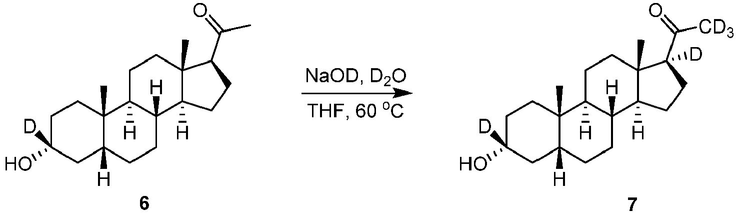

**Synthesis of Compound 7.** To a solution of **Compound 6** (0.1 g, 312 µmol) in THF (1 mL) was added D₂O (1 mL) and NaOD (0.2 M in D₂O, 1.55 mL). The reaction mixture was stirred at 60°C for 12 hrs. CF₃COOD (0.1 M in D₂O) was added until the pH to 7. The mixture was extracted with EtOAc (3 x 1.5 mL). The combined organic layers were dried over Na₂SO₄. The mixture was filtered and concentrated under vacuum. The reaction was repeated twice with the same procedure to provide **Compound** 7 (46.6 mg, 47%) as an off white solid. **¹H NMR** (400 MHz, CDCl₃) δ 2.17-2.12 (m, 1H), 2.03-1.95 (m, 1H), 1.92-1.08 (m, 20H), 1.06-0.91 (m, 4H), 0.59 (s, 3H). **LCMS** Rt = 1.275 min in 2 min chromatography, 10-80 AB, MS ESI calcd. for C₂₁H₂₈D₅O [M+H-H₂O]⁺ 306, found 306. **HRMS** MS ESI calcd. for C₂₁H₃₀D₅O₂ [M+H]⁺ 324.2945, found 324.2949.

### Example 7. Synthesis of Compounds 9 and 10. (Reference)

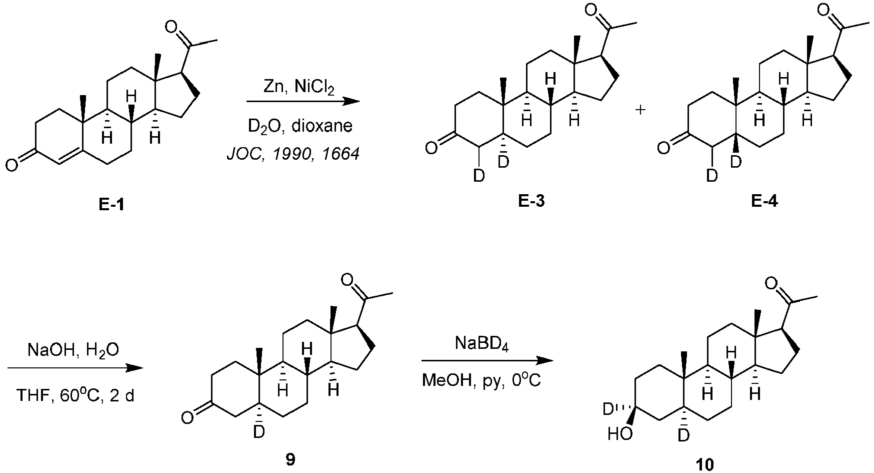

**Step 1.** Zinc dust (3.04 g, 47.7 mmol) and anhydrous nickel chloride (1.53 g, 12.0 mmol) were placed in an argon-flushed flask equipped with a magnetic bar. **E-1** (10 g, 31.8 mmol) in dioxane (50 mL) and D₂O (12.8 mL) were added. The flask was firmly stirred and heated to 40°C. An excess of deuterium was developed during the early stage. The reaction was stirred at 40°C for 16 hrs. The reaction was diluted with ethyl acetate, filtered through Celite, dried over Na₂SO₄, filtered and evaporated. The reaction was performed twice on 10 g scale. The product was combined and purified by combi-flash (0-10% of EtOAc in PE) twice and triturated from MTBE to give **E-3** (1.65 g) and **E-4** (1.8 g). **E-3: ¹H NMR** (400 MHz, CDCl₃) δ 2.60-2.47 (m, 1H), 2.45-2.13 (m, 4H), 2.11 (s, 3H), 2.08-1.94 (m, 2H), 1.79-1.55 (m, 4H), 1.50-1.09 (m, 8H), 1.01 (s, 3H), 1.00-0.71 (m, 2H), 0.63 (s, 3H). **E-4: ¹H NMR** (400 MHz, CDCl₃) δ 2.65 (s, 1H), 2.59-2.48 (m, 1H), 2.40-2.17 (m, 1H), 2.23-2.14 (m, 2H), 2.12 (s, 3H), 2.10-1.96 (m, 2H), 1.94-1.79 (m, 1H), 1.75-1.63 (m, 2H), 1.59-1.31 (m, 7H), 1.30-1.06 (m, 4H), 1.02 (s, 3H), 0.63 (s, 3H). **Step 2.** To a solution of **E-3** (900 mg, 2.82 mmol) in THF (36 mL) and water (18 mL) was added NaOH (1.8 mL, 1 M in water) and the mixture was stirred at 60°C for 24 hrs. The reaction was combined with another batch and acidified pH = 3-5 with 1 N HCl (5 mL), extracted with EtOAc (3 x 30 mL), dried over Na₂SO₄, filtered and concentrated in vacuum to give a white solid. The solid was triturated with MTBE (40 mL) to give 900 mg **Compound 9** and 400 mg of impure solid from mother liquid. The deuterated ratio was 94%. **¹H NMR** (400 MHz, CDCl₃) δ 2.57-2.47 (m, 1H), 2.46-2.22 (m, 3H), 2.21-2.13 (m, 1H), 2.12 (s, 3H), 2.11-1.94 (m, 1H), 1.77-1.60 (m, 4H), 1.48-1.09 (m, 8H), 1.02 (s, 3H), 0.99-0.86 (m, 1H), 083-0.70 (m, 1H), 0.63 (s, 3H). **LCMS** Rt = 1.072 min in 2.0 min chromatography, 30-90AB_2MIN_ELSD, MS ESI calcd. for C₂₁H₃₂DO₂ [M+H]⁺ 318, found 318. **HRMS**: Acq Method: 0-95_1_4min_REF.m; Rt = 3.215 min in 4 min chromatography; DA Method: HRMS-ZN.m. MS ESI calcd. for C₂₁H₃₂DO₂ [M+H]⁺ 318.2543, found 318.2534.

**Step 3.** To a solution of **Compound 9** (80 mg, 0.251 mmol) in MeOH (5.5 mL) was added an ice-cold solution of NaOH (5.99 mg, 0.015 mmol) in water (0.04 mL) and MeOH (2 mL) at 0°C. An ice-cold solution of NaBD₄ (13.6 mg, 0.326 mmol) in pyridine (1.6 mL) was added at 0°C. The reaction was stirred for 20 mins. The reaction was combined with another batch and quenched with 5% of HCl to pH = 5. The resulting mixture was extracted with EtOAc (2 x 10 mL). The combined organic layers were washed with brine (10 mL), dried over Na₂SO₄, filtered and concentrated in vacuum to give the crude product, which was purified by combi-flash (10 % of EtOAc in PE) to give the desired product **Compound 10** as a white solid (20 mg of pure product and 80 mg of impure product). *Note:* Deuterated ratio was 91%. **¹H NMR** (400 MHz, CDCl₃) δ 2.51 (t, *J* = 8.8 Hz, 1H), 2.22-2.12 (m, 1H), 2.11 (s, 3H), 2.04-1.91 (m, 1H), 1.87-1.50 (m, 6H), 1.48-1.07 (m, 11H), 1.04-0.84 (m, 2H), 0.80 (s, 3H), 0.78-0.63 (m, 1H), 0.60 (s, 3H). **LCMS** Rt = 0.998 min in 2.0 min chromatography, 30-90AB_2MIN_E, MS ESI calcd. for C₂₁H₃₁D₂O [M+H-H₂O]⁺ 303, found 303. **HRMS** Acq Method: 0-95_1_4min_REF.m; Rt = 3.132-3.234 min in 4 min chromatography; DA Method: HRMS-ZN.m. MS ESI calcd. for C₂₁H₃₃D₂O₂ [M+H]⁺ 321.2684, found 321.2738.

### Example 8. Synthesis of Compound 11. (Reference)

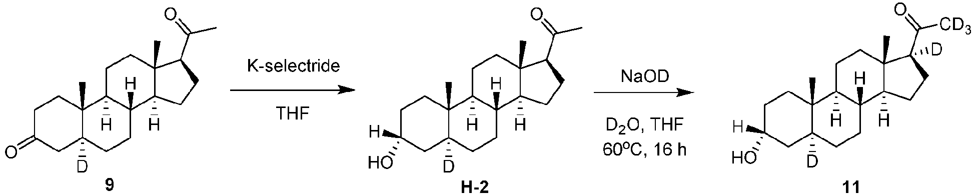

**Step 1.** To a solution of **Compound 9** (100 mg, 0.314 mmol) in anhydrous THF (2 mL) was added dropwise K-selectride (0.439 mL, 0.439 mmol, 1 M in THF) at -78°C under N₂. After the addition was completed, the reaction mixture was stirred for 3 hrs at -78°C. The reaction mixture was slowly quenched with H₂O₂ (0.2 mL, 10% aq.) at -78°C and warmed to 0°C. NH₄Cl (Sat., 2 mL) and Na₂S₂O₃ (Sat. aq., 2 mL) was added. The organic layer was separated. The aqueous phase was extracted with EtOAc (2 x 10 mL). The combined organic layers were washed with brine and dried over Na₂SO₄, filtered and concentrated under vacuum to give the crude product, which was purified by combi-flash (15-20% of EtOAc in PE) to afford **H-2** (30 mg, 30%) as an off white solid. Note: The deuterated ratio was 94%. **¹H NMR** (400 MHz, CDCl₃) δ 4.09-4.00 (m, 1H), 2.58-2.48 (m, 1H), 2.23-2.07 (m, 4H), 2.05-1.91 (m, 1H), 1.77-1.09 (m, 18H), 1.04-0.89 (m, 1H), 0.86-0.70 (m, 4H), 0.60 (s, 3H). **LCMS** Rt = 1.038 min in 2.0 min chromatography, 30-90AB_2MIN_E, MS ESI calcd. for C₂₁H₃₂DO [M+H-H₂O]⁺ 302, found 302. **HRMS** MS ESI calcd. for C₂₁H₃₄DO₂ [M+H]⁺ 320.2694, found 320.2676.

**Step 2.** To a solution of **H-2** (150 mg, crude) in THF (6 mL) and D₂O (3 mL) was added NaOD (0.0615 mL, 0.1M/L in D₂O). The mixture was stirred at 60°C for 12 hrs. The reaction was acidified to pH = 7 with 1 mL of CH₃COOD (0.1 mL CH₃COOD in 1 mL D₂O). The mixture was extracted with EtOAc (3 x 5 mL), dried over Na₂SO₄, filtered and concentrated in vacuum to give an off white solid. This reaction was conducted 5 times. The solid was triturated from hexane (2 x 0.5 mL) to give **Compound 11** (34.8 mg). The deuterated ratio was 90%.

**¹H NMR** (400 MHz, CDCl₃) δ 4.12-3.95 (m, 1H), 2.20-2.07 (m, 1H), 2.05-1.92 (m, 1H), 1.77-1.07 (m, 18H), 1.04-0.87 (m, 1H), 0.85-0.69 (m, 4H), 0.60 (s, 3H). **LCMS** Rt = 1.026 min in 2.0 min chromatography, 30-90AB_2MIN_E, MS ESI calcd. for C₂₁H₂₈D₅O [M+H-H₂O]⁺ 306, found 306. **HRMS** ESI calcd. for C₂₁H₃₀D₅O₂ [M+H]⁺ 324.2945, found 324.2942.

### Example 9. Synthesis of Compounds 12 and 13. (Reference)

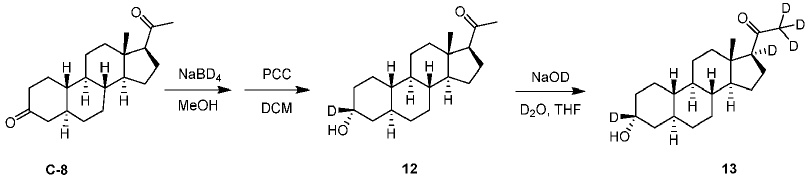

**Step 1.** To a solution **of C-8** (2 g, 6.61 mmol) in MeOH (50 mL) was added an ice-cold solution of NaBD₄ (138 mg, 3.30 mmol) in MeOH (10 mL) at 0°C. The reaction was stirred for 30 mins at 5°C. The resulting mixture was concentrated to give a residue, which was diluted with water (200 mL) and extracted with EtOAc (2 x 200 mL). The combined organic parts were washed with brine (200 mL), dried over Na₂SO₄, filtered and concentrated in vacuum to give the crude product, which was purified by silica gel chromatography (PE/EtOAc = 20/1) to give 800 mg of a mixture containing **Compound 12.** To the crude **Compound 12** (800 mg) in DCM (50 mL), silica gel (800 mg) and PCC (840 mg, 3.91 mmol) was added. The reaction mixture was stirred was stirred at 15°C for 2 hrs. The solution was filtered and the filter cake was washed with DCM (50 mL). The combined filtrate was concentrated in vacuum. The crude product was purified by silica gel column eluted with PE/EtOAc = 15/1 to **Compound 12** (80 mg, 10%). This step was conducted for purification. **¹H NMR** (400 MHz, CDCl₃) δ 2.53-2.51 (m, 1H), 2.19-2.10 (m, 4H), 1.99-1.97(m, 3H), 1.85-1.70 (m, 2H), 1.65-1.57 (m, 4H), 1.55-0.75 (m, 12H), 0.74-0.61 (m, 5H). **LCMS** Rt =2.411 min in 7.0 min chromatography, 30-90 AB, MS ESI calcd. for C₂₀H₃₀DO [M+H-H₂O]⁺ 288, found 288. **HRMS** MS ESI calcd. for C₂₀H₃₁DO₂ [M+H]⁺ 306.2538, found: 306.2538.

**Step 3.** To a solution of **Compound 12** (60 mg) in THF (1.5 mL) was added NaOD/D₂O (1.5 mL, 0.1 M) and CD₃OD (0.2 mL), the mixture was stirred at 60°C for 16 h. To the mixture was added CH₃COOD/D₂O (0.5 M) till pH=7 without monitored at 25°C. The mixture was extracted with EtOAc (2 x 3 mL). The combined organic layer was concentrated to give white solid, which was triturated form hexane (3 x 2 mL) to give **Compound 13** (7 mg) as white solid. **¹H NMR** (400 MHz, CDCl₃) δ 2.15-2.14 (m, 1H), 2.01-1.89 (m, 3H), 1.85-1.73(m, 2H), 1.68-1.60 (m, 3H), 1.42-0.75 (m, 13H), 0.70-0.55 (m, 5H). **LCMS** Rt = 0.964 min in 2.0 min chromatography, 30-90 AB, MS ESI calcd. for C₂₀H₂₆D₅O [M+H-H₂O]⁺ 292, found 292. **HRMS,** MS ESI calcd. for C₂₀H₂₈D₅O₂ [M+H]⁺ 310.2789, found 310.2776.

### Example 10. Synthesis of Compounds 14, 15, and 16. (Reference)

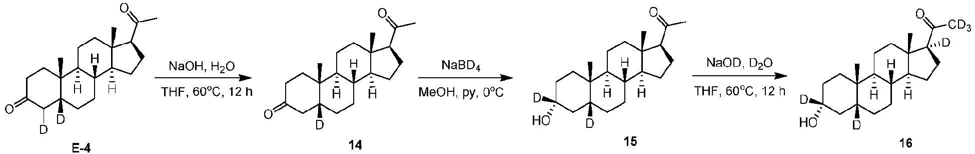

**Step 1.** To a solution of **E-4** (1.0 g, 3.13 mmol) in THF (40 mL) and H₂O (20 mL) was added NaOH (2.0 mL, 1M/L in water).The mixture was stirred at 60°C for 24 hrs. The reaction was acidified to pH = 3-5 with 1 N HCl (5 mL), extracted with EtOAc (3 x 30 mL). The organic layers were dried over Na₂SO₄, filtered and concentrated in vacuum to give the product **Compound 14** as an off white solid (900 mg). *Note*: Deuterated ratio was 95% **¹H NMR** (400 MHz, CDCl₃) δ 2.68 (d, *J* = 15 Hz, 1H), 2.60-2.49 (m, 1H), 2.40-2.26 (m, 1H), 2.24-2.15 (m, 2H), 2.12 (s, 3H), 2.10-1.98 (m, 3H), 1.94-1.80 (m, 1H), 1.76-1.62 (m, 2H), 1.57-1.33 (m, 7H), 1.31-1.07 (m, 4H), 1.02 (s, 3H), 0.64 (s, 3H). **LCMS** Rt = 1.046 min in 2.0 min chromatography, 30-90AB_2MIN_E, MS ESI calcd. for C₂₁H₃₂DO₂ [M+H]⁺ 318, found 318. **HRMS** ESI calcd. for C₂₁H₃₂DO₂ [M+H]⁺ 318.2538, found 318.2534.

**Step 2.** To a solution of **14** (400 mg, 1.25 mmol) in MeOH (27.5 mL) was added an ice-cold solution of NaOH (30.0 mg, 7.5 mmol) in water (0.2 mL) and MeOH (10 mL) at 0°C. An ice-cold solution of NaBD₄ (36.6 mg, 875 µmol) in pyridine (8 mL) was added at 0°C. The reaction was stirred for 20 mins. The reaction was quenched with 5% HCl to pH = 5. The resulting mixture was extracted with EtOAc (2 x 50 mL). The combined organic layrers were washed with brine (50 mL), dried over Na₂SO₄, filtered and concentrated in vacuum to give the crude product, which was purified by combi-flash (10 % of EtOAc in PE) to give **Compound 15** as an off white solid (240 mg). *Note:* Deuterated ratio was 94%

**¹H NMR** (400 MHz, CDCl₃) δ 2.53 (t, *J* = 8.8 Hz, 1H), 2.23-2.12 (m, 1H), 2.11 (s, 3H), 2.05-1.95 (m, 1H), 1.90-1.62 (m, 6H), 1.55-1.05 (m, 13H), 1.04-0.94 (m, 1H), 0.92 (s, 3H), 0.59 (s, 3H). **LCMS** Rt = 1.031 min in 2.0 min chromatography, 30-90AB_2MIN_E, MS ESI calcd. for C₂₁H₃₁D₂O [M+H-H₂O]⁺ 303, found 303. **HRMS** ESI calcd. for C₂₁H₃₃D₂O₂ [M+H]⁺ 321.2757, found 321.2773.

**Step 3.** To a solution of **Compound 15** (80 mg, 249 µmol, 1.0 eq.) in THF (2 mL) and D₂O (1 mL) was added NaOD (0.1 mL, 1M/L in water). The mixture was stirred at 60°C for 12 hrs. The reaction was adjusted pH to 7 with a solution of CD₃COOD (0.1 mL of CD₃COOD in 1 mL of D₂O) and extracted with EtOAc (3 x 5 mL). The organic layers were dried over Na₂SO₄, filtered and concentrated in vacuum to give an off white solid. The reaction was re-conducted 3 times to give 25 mg of the product as an off white powder. *Note:* Deuterated ratio was 92%. **¹H NMR** (400 MHz, CDCl₃) δ 2.21-2.09 (m, 1H), 2.05-1.95 (m, 1H), 1.91-1.57 (m, 6H), 1.55-1.05 (m, 8H), 1.04-0.87 (m, 6H), 0.92 (s, 3H), 0.59 (s, 3H). **LCMS** Rt = 1.035 min in 2.0 min chromatography, 30-90AB_2MIN_E, MS ESI calcd. for C₂₁H₂₇D₆O [M+H-H₂O]⁺ 307, found 307. **HRMS** ESI calcd. for C₂₁H₂₉D₆O₂ [M+H]⁺ 325.3008, found 325.3000.

### Example 11. Synthesis of Compounds 17 and 18. (Reference)

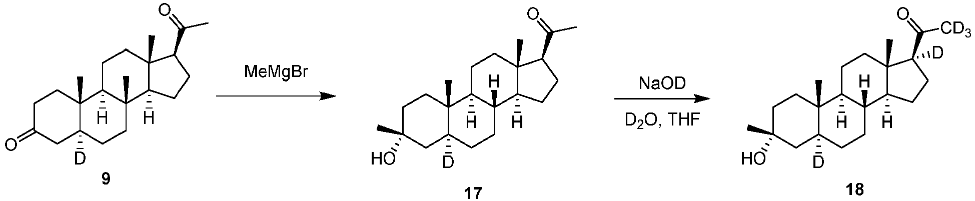

**Step 1.** To a solution of **Compound 9** (300 mg, 0.944 mmol) in THF (5 mL) was added MeMgBr (0.6 mL, 3 M in ether, 1.88 mmol) dropwise at -70°C. The mixture was stirred at -70°C for 0.5 h and a lot of solid was formed. The mixture was treated with NH₄Cl (1 mL, sat. aq.). The mixture was extracted with EtOAc (2 × 5 mL), dried over Na₂SO₄, concentrated in vacuum, and purified by column chromatography on silica gel (0-10% of EtOAc in PE/DCM (2/1)) to give **Compound 17**as an off white solid (70 mg, 23%). *Note:* The deuterated ratio was 94%. **¹H NMR** (400 MHz, CDCl₃) δ 2.58-2.45 (m, 1H), 2.20-2.04 (m, 4H), 2.03-1.92 (m, 1H), 1.72-1.48 (m, 4H), 1.47-1.05 (m, 16H), 1.04-0.87 (m, 1H), 0.85-0.78 (m, 2H), 0.75 (s, 3H), 0.60 (s, 3H). **LCMS** Rt = 1.102 min in 2.0 min chromatography, 30-90AB_2MIN_E, MS ESI calcd. for C₂₂H₃₄DO [M+H-H₂O]⁺ 316, found 316. **HRMS** ESI calcd. for C₂₂H₃₆DO₂ [M+H]⁺ 334.2851, found 334.2851.

**Step 2.** To a solution of **Compound 17** (45 mg, crude) in THF (2 mL) was added NaOD/D₂O (1 mL, 0.1 M) and CD₃OD (0.2 mL) and the mixture was stirred at 60°C for 72 h. The reaction was adjusted to pH 7 with CH₃COOD (1 mL, 0.1 mL CH₃COOD in 1 mL D₂O), extracted with EtOAc (3 x 5 mL), dried over Na₂SO₄, filtered and concentrated in vacuum to give an off white solid, which was further triturated with hexane (2 x 0.5 mL) to give **Compound 18** as white solid (42.2 mg, 93%). Note: deuterated ratio was 91%. **¹H NMR** (400 MHz, CDCl₃) δ 2.19-2.09 (m, 1H), 2.03-1.92 (m, 1H), 1.80-1.45 (m, 4H), 1.44-1.03 (m, 16H), 1.02-0.85 (m, 2H), 0.83-0.77 (m, 1H), 0.75(s, 3H) 0.60 (s, 3H). **LCMS** Rt = 1.095 min in 2.0 min chromatography, 30-90AB_2MIN_E, MS ESI calcd. for C₂₂H₃₀D₅O [M+H-H₂O]⁺ 320, found 320. **HRMS** ESI calcd. for C₂₂H₃₂D₅O₂ [M+H]⁺ 338.3102, found 338.3090.

### Example 12. Synthesis of Compound 21. (Reference)

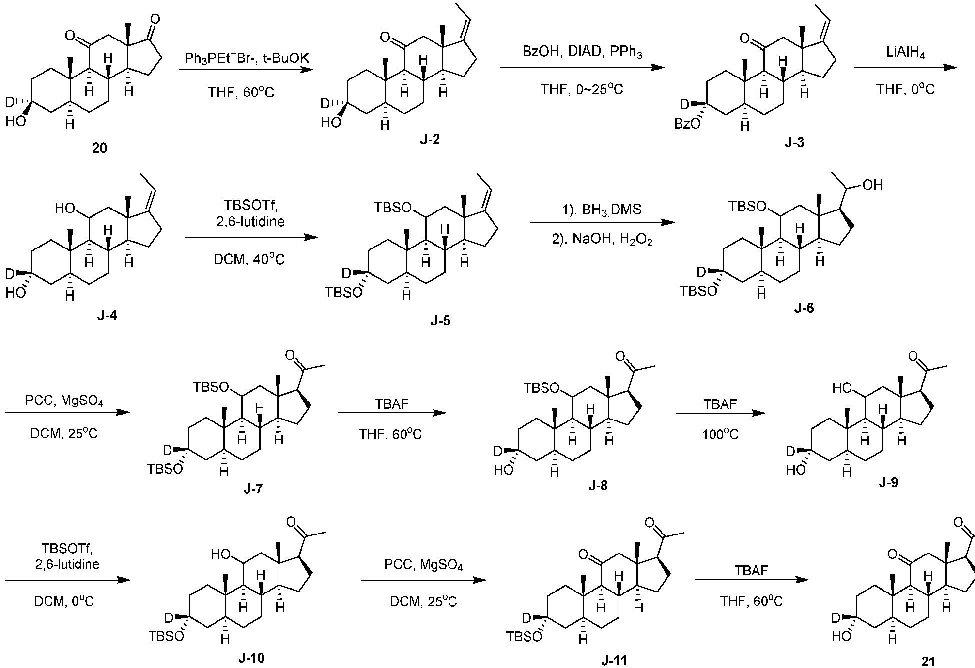

**Step 1.** To a slurry of bromo(ethyl)triphenylphosphorane (42.2 g, 114 mmol) in THF (70 mL) was added t-BuOK (12.7 g, 114 mmol) under N₂. After addition, the mixture was stirred at 60°C for 30 minutes. A solution of **Compound 20** (5 g, 16.3 mmol) in THF (10 mL) was added. The mixture was stirred at 60°C for 1 hour. The reaction was quenched by saturated NH₄Cl solution (100 mL) and extracted with EtOAc (2 × 50 mL). The combined organic phase was dried, filtered, concentrated and purified by combi-flash (0-20% of EtOAc in PE) to give J-2 (4.3 g, 83%) as white solid. **¹H NMR** (400 MHz, CDCl₃) δ 5.29-5.15 (m, 1H), 2.87-2.84 (m, 1H), 2.55-2.34 (m, 5H), 1.86-1.30 (m, 7H), 1.28-1.06 (m, 12H), 0.83-0.81 (m, 4H), 0.70-0.68 (m, 1H). **Step 2.** To a solution of **J-2** (2 g, 6.29 mmol) in THF (20 mL) was added PhCO₂H (0.92 g, 7.54 mmol), PPh₃ (3.27 g, 12.5 mmol) and DIAD (2.52 g, 12.5 mmol) at 0°C. The mixture was stirred at 25°C for 16 h. The mixture was concentrated and purified by column chromatography on silica gel (PE/EtOAc = 15/1) directly to give **J-3** (2.1 g, 79%) as white solid. **¹H NMR** (400 MHz, CDCl₃) δ 8.10-8.00 (m, 2H), 7.60-7.50 (m, 1H), 7.50-7.40 (m, 2H), 5.20-5.10 (m, 1H), 2.87 (d, *J* = 12.4 Hz, 1H), 2.59 (d, *J* = 12.0 Hz, 1H), 2.50-2.25 (m, 3H), 1.95-1.65 (m, 7H), 1.70-1.45 (m, 6H), 1.35-1.15 (m, 5H), 1.07 (s, 3H), 0.84 (s, 3H).

**Step 3.** To a suspension of LiAlH₄ (565 mg, 14.9 mmol) in THF (20 mL) was added a solution of **J-3** (2.1 g, 4.98 mmol) in THF (5 mL) at 0°C and the mixture was stirred at 0°C for 1 h. The mixture was quenched by adding water (0.56 mL), NaOH (0.56 mL, 15 % aq.) and water (1.68 mL) at 0°C. The mixture was stirred and warmed to 25°C within 10 mins. The mixture was dried over MgSO₄ and filtered. The filtrate was concentrated in vacuum to give **J-4** (2.0 g, crude) as a colorless oil. **¹H NMR** (400 MHz, CDCl₃) δ 5.15-5.05 (m, 1H), 4.40-4.30 (m, 1H), 2.45-2.30 (m, 2H), 2.25-2.10 (m, 1H), 1.90-0.80 (m, 28H).

**Step 4.** To a solution of **J-4** (1.8 g, 5.63 mmol) in DCM (20 mL) was added 2,6-lutidine (3.01 g, 28.1 mmol) and TBSOTf (7.42 g, 28.1 mmol) at 0°C. The mixture was stirred at 40°C for 16 hrs. To the mixture was added water (10 mL). The organic solvent was removed in vacuum. The residue was extracted with PE (2 x 20 mL). The combined organic layer was dire over Na₂SO₄, filtered, concentrated in vacuum and purified by column chromatography on silica gel (PE) to give **J-5** (4.6 g, crude) as white solid. **¹H NMR** (400 MHz, CDCl₃) δ 5.10-5.00 (m, 1H), 4.40-4.35 (m, 1H), 2.55-2.45 (m, 1H), 2.40-2.30 (m, 1H), 2.25-2.10 (m, 1H), 1.90-1.75 (m, 2H), 1.70-0.80 (m, 41H), 0.80-0.70 (m, 1H), 0.17 (s, 3H), 0.09 (s, 3H), 0.01 (s, 6H).

**Step 5.** To a solution of **J-5** (4.6 g, crude) in THF (20 mL) was added BH₃-Me₂S (8.39 mL, 83.9 mmol) at 0°C dropwise. The solution was stirred at 25°C for 16 h. To the mixture was added NaOH (40 mL, 3 M) and H₂O₂ (10 mL, 10 M) at 0°C. The mixture was stirred at 25°C for 1 h. The mixture was extracted with EtOAc (2 x 100 mL). The combined organic layer was washed with Na₂S₂O₃ (100 mL, sat. aq.), dried over Na₂SO₄, filtered, purified by silica gel column (PE/EtOAc = 50/1 to 20/1) to give 0.3 g of an isomer of **J-6R** and 0.7 g of another isomer **J-6S,** stereochemistry was randomly assigned at C-20. **J-6R: ¹H NMR** (400 MHz, CDCl₃) δ 4.32-4.28 (m, 1H), 3.75-3.65 (m, 1H), 2.38-2.30 (m, 1H), 1.85-0.91 (m, 47H), 0.77-0.69 (m, 1H), 0.12 (s, 3H), 0.06 (s, 3H), 0.01 (s, 6H). **J-6S: ¹H NMR** (400 MHz, CDCl₃) δ 4.38-4.30 (m, 1H), 3.70-3.60 (m, 1H), 2.15-2.09 (m, 1H), 1.96-0.95 (m, 47H), 0.77-0.69 (m, 1H), 0.11 (s, 3H), 0.07 (s, 3H), 0.01 (s, 6H).

**Step 6.** To a solution of **J-6S** (0.7 g, 1.23 mmol) and **J-6R** (0.3 g, 0.53 mmol) in DCM (15 mL) was added MgSO₄ (1 g) and PCC (792 mg, 3.69 mmol). The mixture was stirred at 25°C for 2 hours. The mixture was filtered. The solid was washed with DCM. The combined filtrate was concentrated, purified by silica gel column (PE/EtOAc = 50:1) to give **J-7** (1 g, 100%) as white solid. **¹H NMR** (400 MHz, CDCl₃) δ 4.42-4.39 (m, 1H), 2.50-2.40 (m, 1H), 2.32-2.25 (m, 1H), 2.22-2.12 (m, 1H), 2.10 (s, 3H), 1.85-0.71 (m, 42H), 0.11 (s, 3H), 0.08 (s, 3H), 0.01 (s, 6H). **Step 7. J-7** (1 g, 1.77 mmol) was added to TBAF (5 mL, 1 M in THF). The mixture was stirred at 60°C for 20 hrs. The mixture was treated with water (20 mL) and the organic solvent was removed in vacuum. The residue was treated with PE (20 mL) and stirred for 10 mins. The mixture was filtered. The filtrate cake was washed with water (20 mL) and PE (20 mL), dried in vacuum to give pure **J-8** (490 mg, 82%) as an off white solid. **¹H NMR** (400 MHz, CDCl₃) δ 4.41-4.37 (m, 1H), 2.43 (t, *J* = 8.8 Hz, 1H), 2.35-2.28 (m, 1H), 2.22-2.12 (m, 1H), 2.09 (s, 3H), 1.88-0.75 (m, 34H), 0.11 (s, 3H), 0.08 (s, 3H).

**Step 8.** 450 mg of **J-8** (1 mmol) was added to TBAF (5 mL, 1 M in THF). The mixture was heated to 100°C and the solvent was evaporated. The residue was stirred at 100°C for 3 h. The mixture was combined with another batch from 320 mg of impure **J-8.** To the residue was added water (20 mL), extracted with EtOAc (2 × 20 mL). The combined organic layer was concentrated and purified by silica gel column (PE/EtOAc = 3/1 to 2/1) directly to give **J-9** (350 mg, 61%) as white solid. **¹H NMR** (400 MHz, CDCl₃) δ 4.43-4.38 (m, 1H), 2.45 (t, *J* = 8.8 Hz, 1H), 2.25-2.12 (m, 2H), 2.11 (s, 3H), 1.90-0.95 (m, 22H), 0.90-0.78 (m, 4H). **HRMS** MS ESI calcd. for C₂₁H₃₄DO₃ [M+H]⁺ 336.2643, found 336.2632.

**Step 9.** To a solution of **J-9** (170 mg, 0.506 mmol) in DCM (3 mL) was added 2,6-lutidine (542 mg, 5.06 mmol) and TBSOTf (668 mg, 2.53 mmol) at 0°C. The mixture was stirred at 0°C for 5 mins. The mixture was treated with water (5 mL) and extracted with EtOAc (10 mL). The organic layer was separated, dried over Na₂SO₄, filtered, concentrated and purified by silica gel column (PE/EtOAc = 30/1 to 20/1) to give 110 mg of **J-10** (110 mg, 48%) as an off white solid. **¹H NMR** (400 MHz, CDCl₃) δ 4.43-4.38 (m, 1H), 2.45 (t, *J* = 8.8 Hz, 1H), 2.25-2.14 (m, 2H), 2.12 (s, 3H), 1.88-0.80 (m, 34H), 0.02 (s, 6H).

**Step 10.** To a solution of **J-10** (50 mg, 0.11 mmol) in DCM (1 mL) was added MgSO₄ (66.8 mg) and PCC (35.6 mg, 0.16 mmol). The mixture was stirred at 25°C for 1 h. The mixture was treated with PE (3 mL). The mixture was filtered and the filtrate was concentrated in vacuum to give **J-11** (50 mg, 100%) as light red oil. **¹H NMR** (400 MHz, CDCl₃) δ 2.72 (t, *J* = 8.8 Hz, 1H), 2.60-2.48 (m, 2H), 2.30-2.27 (m, 1H), 2.26-2.03 (m, 4H), 1.89-0.80 (m, 28H), 0.56 (s, 3H), 0.01 (s, 6H).

**Step 11.** A solution of **J-11** (50 mg, 0.11 mmol) in 1 mL of TBAF (1 mL, 1M in THF) stirred at 60°C for 16 hrs. The mixture was concentrated in vacuum and purified by silica gel column directly (PE/EtOAc = 3/1) to give 30 mg of impure **Compound 21.** The impure **Compound 21** (30 mg) was purified by SFC (Instrument: SFC-14, Column: AD(250mm*30mm,5um), Condition: Base-ETOH, Begin B: 30%, End B: , Gradient Time(min): 100%B Hold Time(min): , FlowRate(ml/min): 60ML/MIN, Injections: 100) to give 20 mg of **Compound 21** (NMR and LCMS showed 21-H was partly deuterated). To a solution of **Compound 21** (19 mg, 21-H partly deuterated) in THF (2 mL) and MeOH (0.5 mL) was added NaOH (0.5 mL, 0.1 M aq.) and the mixture was stirred for 16 h at 60°C. Crude LCMS showed 21-D was exchanged with the proton. The mixture was concentrated and purified by silica gel column (PE/EtOAc = 3/1) directly to give 2.9 mg of white solid (deuterated ratio: 98.0%). **¹H NMR** (400 MHz, CDCl₃) δ 2.73 (t, *J* = 8.8 Hz, 1H), 2.62-2.45 (m, 2H), 2.30-2.17 (m, 2H), 2.09 (s, 3H), 1.88-1.69 (m, 7H), 1.59-1.49 (m, 3H), 1.40-1.11 (m, 7H), 1.00 (s, 3H), 0.57 (s, 3H). **LCMS** Rt = 1.089 min in 2.0 min chromatography, 10-80AB_2MIN_E, MS ESI calcd. for C₂₁H₃₂DO₃ [M+H]⁺ 334, found 334. **HRMS** MS ESI calcd. for C₂₁H₃₂DO₃ [M+H]⁺ 334.2487, found 334.2478.

### Example 13. Synthesis of Compounds 22 and 23. (Reference)

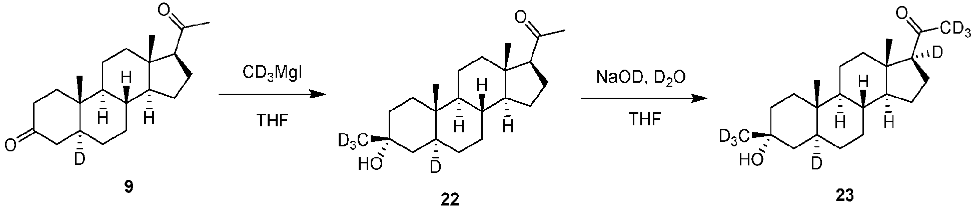

**Step 1.** To a suspension of Mg (91.1 mg, 3.75 mmol) and I₂ (1 mg) in ether (3 mL) was added solution of CD₃I (543 mg, 3.75 mmol) in ether (6 mL) dropwise under N₂ at 25°C and inner temperature was raised to 35°C. The mixture was then stirred at 35°C for 0.5 hour. To a solution of **Compound 9** (200 mg, 0.63 mmol) in THF (10 mL) was added fresh prepared CD₃MgI (1.25 mmol in 3 mL of ether) dropwise at 0°C. The mixture was stirred at 0°C for 1 h. The mixture was quenched by adding NH₄Cl (10 mL, sat. aq.) and extracted with EtOAc (2 × 20 mL). The organic layer was separated, dried over Na₂SO₄, filtered, concentrated in vacuum and purified by silica gel column (PE/DCM/EtOAc = 2/1/0.02) to give **Compound 22** (7.5 mg, deuterated ratio: 96%) as white solid. **¹H NMR** (400 MHz, CDCl₃) δ 2.53 (t, *J* = 8.8 Hz, 1H), 2.20-2.12 (m, 1H), 2.11 (s, 3H), 2.02-1.95 (m, 1H), 1.72-1.58 (m, 4H), 1.55-1.10 (m, 14H), 1.00-0.91 (m, 1H), 0.85-0.77 (m, 1H), 0.75 (s, 3H), 0.60 (s, 3H). **LCMS** Rt = 1.069 min in 2.0 min chromatography, 30-90AB_2MIN_E, (ELSD), MS ESI calcd. for C₂₂H₃₁D₄O [M+H-H₂O]⁺ 319, found 319. **HRMS** MS ESI calcd. for C₂₂H₃₃D₄O₂ [M+H]⁺ 337.3039, found 337.3033.

**Step 2.** To a solution of **Compound 22** (19 mg, 56.4 umol) in THF (2 mL) was added and CD₃OD (0.2 mL) and NaOD (1 mL, 0.1 M in D₂O) and the mixture was stirred for 16 h at 60°C. The mixture was neutralized by CH₃COOD/D₂O till pH = 7. The mixture was extracted with EtOAc (2 x 3 mL). The combined organic layer was dried over Na₂SO₄, filtered, concentrated in vacuum and triturated form PE (2 × 2 mL) to give **Compound 23** (7.1 mg, deuterated ratio: 92%) as white solid. **¹H NMR** (400 MHz, CDCl₃) δ 2.10-2.10 (m, 1H), 2.02-1.95 (m, 1H), 1.72-1.60 (m, 4H), 1.55-1.10 (m, 14H), 1.01-0.89 (m, 1H), 0.85-0.77 (m, 1H), 0.75 (s, 3H), 0.60 (s, 3H). **LCMS** Rt = 1.087 min in 2.0 min chromatography, 30-90AB_2MIN_E, MS ESI calcd. for C₂₂H₂₇D₈O [M+H-H₂O]⁺ 323, found 323. **HRMS** MS ESI calcd. for C₂₂H₂₉D₈O₂ [M+H]⁺ 341.3290, found 341.3281.

### Example 14. Synthesis of Compound 24. (Reference)

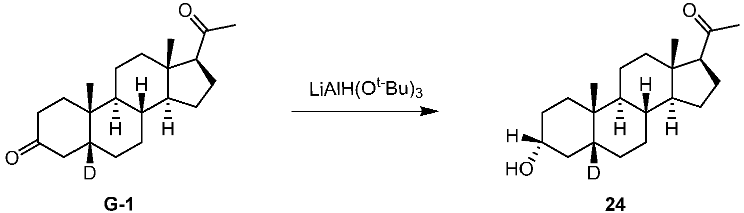

**Step 1.** To a solution of **G-1** (250 mg, 0.787 mmol) in THF (3 mL) was slowly added a solution of LiAlH(t-BuO)₃ (223 mg, 0.865 mmol) in THF (2 mL) under N₂ at -40°C. Then the reaction solution was stirred at -40°C for 15 minutes. The mixture was quenched with saturated NH₄Cl solution (3 mL) .The reaction mixture was extracted with EtOAc (3 x 15 mL). The organic layers were combined, dried over Na₂SO₄, concentrated and purified by silica gel column (PE/EtOAc = 8/1, 6/1, 5/1, 4/1) to give **Compound 24** (38 mg, 15%) as white solid. **¹H NMR** (400 MHz, CDCl₃) δ 3.70-3.60 (m, 1H), 2.53 (t, *J* = 8.8 Hz, 1H), 2.22-2.12 (m, 1H), 2.11 (s, 3H), 2.06-1.98 (m, 1H), 1.88-0.95 (m, 20H), 0.92 (s, 3H), 0.59 (s, 3H). **LCMS** Rt = 1.008 min in 2 min chromatography, 30-90AB ELSD, MS ESI calcd. for C₂₁H₃₂DO [M+H-H₂O]⁺ 302, found 302. **HRMS** ESI calcd. for C₂₁H₃₄DO₂ [M+H]⁺ 320.2694, found 320.2629.

### Example 15. Synthesis of Compound 25. (Reference)

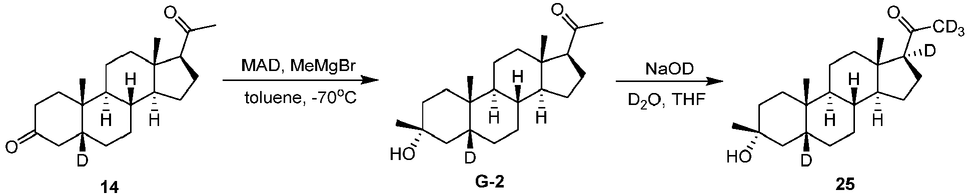

**Step 1.** To a solution of BHT (832 mg, 3.78 mmol) in toluene (10 mL) was added AlMe₃ (0.95 mL, 2M in toluene) at 0°C. The mixture was stirred for 1 h at 0°C. To the previous fresh prepared MAD solution was added a solution of **Compound 14** (200 mg, 629 µmol) in toluene (2 mL) dropwise at -70°C. The mixture was stirred at -70°C for 1 h. MeMgBr (0.63 mL, 3 M in ether, 1.88 mmol) was added dropwise at -70°C. The mixture was stirred at -70°C for another 1 h. To the mixture was added citric acid (10 mL, 20%, aq.) and warmed to 25°C. The mixture was extracted with EtOAc (2 x 20 mL). The combined organic layer was dried over Na₂SO₄, filtered, concentrated and purified by silica gel column (PE/DCM/EtOAc = 2/1/0.03) to give **G-2** (150 mg, deuterated ratio: 98%) as an off white solid.

**¹H NMR** (400 MHz, CDCl₃) δ 2.52 (t, *J* = 8.8 Hz, 1H), 2.22-2.12 (m, 1H), 2.11 (s, 3H), 2.03-1.33 (m, 14H), 1.31-0.99 (m, 10H), 0.94 (s, 3H), 0.59 (s, 3H). **LCMS** Rt = 1.063 min in 2.0 min chromatography, 30-90AB_2MIN_E, MS ESI calcd. for C₂₂H₃₄DO [M+H-H₂O]⁺ 316, found 316. **HRMS** MS ESI calcd. for C₂₂H₃₄DO [M+H-H₂O]⁺ 316.2745, found 316.2751.

**Step 2.** To a solution of **G-2** (50 mg, 149 µmol) in THF (2 mL) was added CD₃OD (0.2 mL) and NaOD (1 mL, 0.1 M in D₂O). The mixture was stirred for 16 hrs at 60°C. The mixture was neutralized by CH₃COOD/D₂O. The mixture was extracted with EtOAc (2 x 3 mL). The combined organic layer was dried over Na₂SO₄, filtered, concentrated in vacuum to give **Compound 25** (20.5 mg, deuterated ratio: 91%) as an off white solid. **¹H NMR** (400 MHz, CDCl₃) δ 2.19-2.10 (m, 1H), 2.03-1.92 (m, 1H), 1.90-1.80 (m, 1H), 1.79-1.70 (m, 1H), 1.69-1.59 (m, 3H), 1.53-1.33 (m, 8H), 1.30-1.01 (m, 10H), 0.94 (s, 3H), 0.59 (s, 3H). **LCMS** Rt = 1.061 min in 2.0 min chromatography, 30-90AB_2MIN_E, MS ESI calcd. for C₂₂H₃₀D₅O [M+H-H₂O]⁺ 320, found 320. **HRMS** MS ESI calcd. for C₂₂H₃₀D₅O [M+H-H₂O]⁺ 320.2996, found 320.3008.

### Example 16. Synthesis of Compound 26. (Reference)

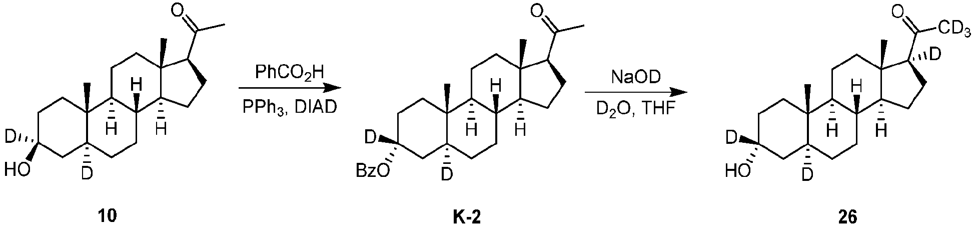

**Step 1.** To a solution of **Compound 10** (200 mg, 624 µmol) in THF (3 mL) was added benzoic acid (91 mg, 748 µmol) and PPh₃ (490 mg, 1.87 mmol), followed by DIAD (377 mg, 1.87 mmol) slowly at 0°C. After that, the reaction was stirred at 28°C for 12 hrs. The reaction was concentrated and purified by combi-flash (5% of EtOAc in PE) to give **K-2** (130 mg) as an off white solid. **¹H NMR** (400 MHz, CDCl₃) δ 8.11-8.00 (m, 2H), 7.61-7.52 (m, 1H), 7.50-7.39 (m, 2H), 2.53 (t, *J* = 8.8 Hz, 1H), 2.27-2.08 (m, 4H), 2.06-1.96 (m, 1H), 1.93-1.52 (m, 13H), 1.49-1.10 (m, 4H), 1.06-0.92 (m, 1H), 0.89-0.73 (m, 4H), 0.62 (s, 3H).

**Step 2.** To a solution of **K-2** (130 mg, 306 µmol, 1.0 eq.) in THF (2 mL) was added NaOD solution (1 mL, 40% in D₂O) and the mixture was stirred at 80°C for 12 hrs. The reaction was acidified to pH = 7 with a solution of CD₃CO₂D (1 mL of CD₃CO₂D in 1 mL of D₂O). The mixture was extracted with EtOAc (3 x 20 mL), dried over Na₂SO₄, filtered and concentrated. This reaction was re-conducted for 3 times. The residue after 3^{rd} run was triturated from hexane to give 25 mg of **Compound 26** as an off white powder. The deuterated ratio was 90%. **¹H NMR** (400 MHz, CDCl₃) δ 2.20-2.06 (m, 1H), 2.04-1.92 (m, 1H), 1.80-1.54 (m, 13H), 1.53-1.08 (m, 5H), 1.04-0.87 (m, 1H), 0.85-0.71 (m, 4H), 0.60 (s, 3H). **LCMS** Rt = 1.121 min in 2.0 min chromatography, 30-90AB_2MIN_E, MS ESI calcd. for C₂₁H₂₇D₆O [M+H-H₂O]⁺ 307, found 307. **HRMS** ESI calcd. for C₂₁H₂₉D₆O₂ [M+H]⁺ 325.3014, found 325.2999.

### Example 17. Synthesis of Compounds 27 and 28. (Reference)

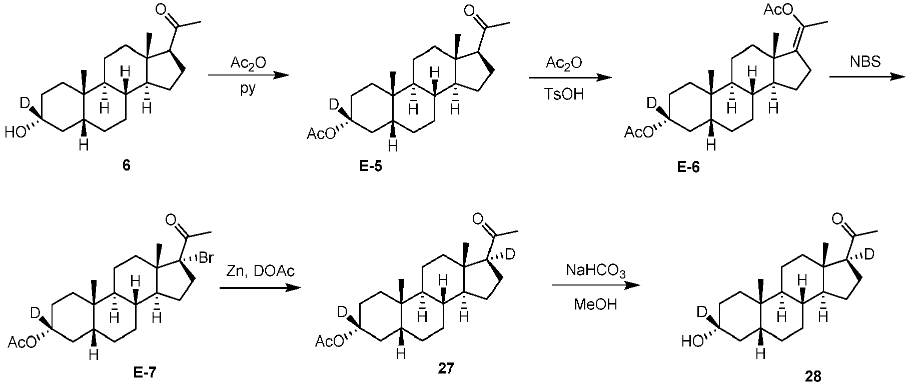

**Step 1.** To a solution of **Compound 6** (1.6 g, 5 mmol) in pyridine (15 mL) was added acetic anhydride (4.7 mL, 50 mmol). The reaction was stirred at 25°C for 4 hrs. The mixture was diluted with MTBE (100 mL) and added 10% HCl (50 mL) by an ice bath to keep the maximum temperature at 35°C. The organic layer was dried over Na₂SO₄, filtered, concentrated under vacuum and purified by combi-flash (0-10% of EtOAc in PE) to give the product **E-5** as an off white solid (1.6 g, 89%). **¹H NMR** (400 MHz, CDCl₃) δ 2.52 (t, *J* = 8.8 Hz, 1H), 2.11 (s, 3H), 2.03 (s, 3H), 2.01-1.98 (m, 1H), 1.91-1.76 (m, 3H), 1.74-1.60 (m, 4H), 1.55-1.35 (m, 8H), 1.31-0.99 (m, 6H), 0.93 (s, 3H), 0.59 (s, 3H).

**Step 2.** To a solution **of E-5** (1.6 g, 4.42 mmol) in acetic anhydride (60 mL) was added TsOH (1.44 g, 8.36 mmol) and the mixture was heated to 150°C for 5 hrs. The reaction was concentrated under vacuum to remove the solvent. The residue was dissolved in DCM (300 mL) and adjusted to pH 7 with 1M NaOH. The organic layer was washed with water and brine (50 mL), dried over Na₂SO₄, filtered, and concentrated to give the product **E-6** as yellow oil (2.0 g, crude). **¹H NMR** (400 MHz, CDCl₃) δ 2.03 (s, 6H), 1.91-0.96 (m, 25H), 0.92 (s, 3H), 0.81 (s, 3H).

**Step 3.** To a solution **of E-6** (2.0 g, crude) in DCM (120 mL) was added NBS (850 mg, 4.81 mmol). The reaction was heated to 55°C for 2 hrs. The reaction was concentrated and purified by combi-flash (5% of EtOAc in PE) to give **E-7** (1.2 g) as a yellow solid.

**¹H NMR** (400 MHz, CDCl₃) δ 3.11-2.96 (m, 1H), 2.38 (s, 3H), 2.35-2.18 (m, 1H), 2.11 (s, 3H), 2.08-1.94 (m, 4H), 1.93-1.78 (m, 6H), 1.76-1.66 (m, 1H), 1.57-1.35 (m, 5H), 1.33-0.99 (m, 4H), 0.94 (s, 3H), 0.74 (s, 3H).

**Step 4.** A solution of **E-7** (600 mg, 1.36 mmol) in diethyl ether (12 mL) was added to a solution of D₂O (27.2 g, 1.36 mol) and acetic anhydride (2.77 g, 27.2 mmol) in diethyl ether (6 mL). Zinc dust (1.33 g, 20.4 mmol) was added, and the reaction was stirred at 20°C for 1 h. The reaction was diluted with diethyl ether (150 mL) and washed with D₂O (12 mL). The organic layer was concentrated under reduced pressure and quickly purified to avoid any deuterium-hydrogen exchange on the silica gel column chromatography (10% of EtOAc in PE) to afford **Compound 27** as an off white solid (400 mg, 81%). The deuterated ratio was 93%. **¹H NMR** (400 MHz, CDCl₃) δ 2.20-2.07 (m, 4H), 2.06-1.95 (m, 4H), 1.91-1.75 (m, 3H), 1.74-1.58 (m, 4H), 1.55-1.34 (m, 7H), 1.33-0.98 (m, 6H), 0.93 (s, 3H), 0.60 (s, 3H).

**LCMS** Rt = 1.209 min in 2.0 min chromatography, 30-90AB_2MIN_EMS ESI calcd. for C₂₁H₃₁D₂O [M+H-HOAc]⁺ 303, found 303. **HRMS** ESI calcd. for C₂₃H₃₅D₂O₃ [M+H]⁺ 363.2863, found 363.2853.

**Step 5.** To a solution of **Compound 27** (150 mg, 413 µmol) in MeOH (7.5 mL) was added NaHCO₃ (103 mg, 1.23 mmol) at 25°C. The mixture was stirred at 25°C for 12 hrs. The solvent was removed under vacuum. The residue was diluted with 3 mL of D₂O, extracted with EtOAc (10 mL), dried over Na₂SO₄, filtered, concentrated in vacuum, and purified by combi-flash (15% of EtOAc in PE) to give **Compound 28** as white solid (29.3 mg, 22%). The deuterated ratio was 92%.

**¹H NMR** (400 MHz, CDCl₃) δ 2.20-2.07 (m, 4H), 2.05-1.95 (m, 1H), 1.91-1.60 (m, 6H), 1.58-1.05 (m, 14H), 1.04-0.94 (m, 1H), 0.92 (s, 3H), 0.59 (s, 3H). **LCMS** Rt = 1.016 min in 2.0 min chromatography, 30-90AB_2MIN_E, MS ESI calcd. for C₂₁H₃₁D₂O [M+H-H₂O]⁺ 303, found 303. **HRMS** ESI calcd. for C₂₁H₃₃D₂O₂ [M+H]⁺ 321.2757, found 321.2685.

### Example 18. Synthesis of Compound 29. (Reference)

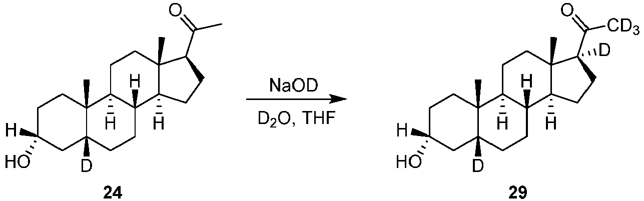

To a solution of **Compound 24** (45 mg, crude) in THF (2 mL) was added NaOD/D₂O (1 mL, 0.1 M) and CD₃OD (0.2 mL) and the mixture was stirred at 60°C for 72 h. The reaction was adjusted the pH to 7 with 1 mL of CH₃COOD (0.1 mL CH₃COOD in 1 mL D₂O), extracted with EtOAc (3 x 5 mL), dried over Na₂SO₄, filtered and concentrated in vacuum to give an off white solid, which was further triturated from hexane (2 x 0.5 mL) to give **Compound 29** as an off white solid (13.2 mg). The deuterated ratio was 90%. **¹H NMR** (400 MHz, CDCl₃) δ 3.70-3.60 (m, 1H), 2.19-2.11 (m, 1H), 2.03-1.92 (m, 1H), 1.91-1.62 (m, 5H), 1.61-1.37 (m, 3H), 1.36-1.06 (m, 10H), 1.04-0.93 (m, 1H), 0.92-0.80 (m, 4H), 0.59 (s, 3H). **LCMS** Rt = 0.992 min in 2.0 min chromatography, 30-90AB_2MIN_E, MS ESI calcd. for C₂₁H₂₈D₅O [M+H-H₂O]⁺ 306, found 306. **HRMS** ESI calcd. for C₂₁H₃₀D₅O₂ [M+H]⁺ 324.2945, found 324.2933.

### Example 19. Synthesis of Compound 30. (Reference)

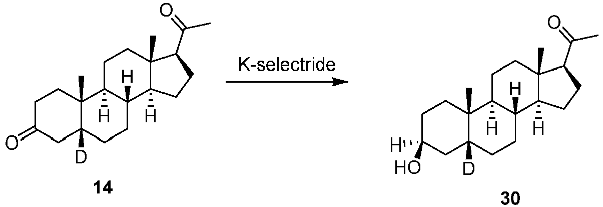

To a solution of **Compound 14** (200 mg, 0.629 mmol) in anhydrous THF (4 mL) was added dropwise K-selectride (0.880 ml, 0.880 mmol, 1 M in THF) at -78°C under N₂. After the addition was completed, the reaction mixture was stirred for 3 hours at -78°C. The reaction mixture was slowly quenched with H₂O₂ (0.2 mL, 10% aq.) at -78°C and warmed to 0°C and NH₄Cl (aq., 2 mL) and Na₂S₂O₃ (aq., 2 mL) were added. The organic layer was separated. The aqueous phase was extracted with ethyl acetate (3 x 10 mL), the combined organic layers were washed with brine and dried over anhydrous Na₂SO₄, filtered, concentrated under vacuum to give the crude product. The product was triturated by acetonitrile (3-5 mL), filtered and dried under vacuum to give **Compound 30** (70 mg) as white solid. **¹H NMR** (400 MHz, CDCl₃) δ 4.17-4.07 (m, 1H), 2.53 (t, *J* = 8.8 Hz, 1H), 2.24-2.14 (m, 1H), 2.11 (s, 3H), 2.05-1.82 (m, 3H), 1.74-1.02 (m, 17H), 0.96 (s, 3H), 0.90-0.73 (m, 1H), 0.60 (s, 3H). **LCMS** Rt = 1.011 min in 2 min chromatography, 30-90AB_ELSD, MS ESI calcd. for C₂₁H₃₂DO [M+H-H₂O]⁺ 302, found 302. **HRMS** MS ESI calcd. for C₂₁H₃₄DO₂ [M+H]⁺ 320.2694, found 320.229.

### Example 20. Synthesis of Compounds 31 and 32. (Reference)

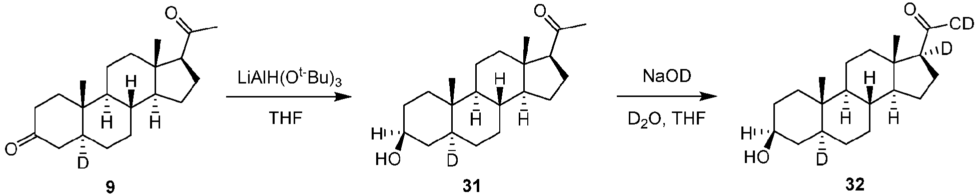

**Step 1.** To a solution of **Compound 9** (250 mg, 0.787 mmol) in THF (2 mL) was slowly added a solution of LiAlH(t-BuO)₃ (219 mg, 0.865 mmol) in THF (1 mL) under N₂ at -40°C. Then the reaction solution was stirred at -40°C for 1 h. The mixture was quenched with saturated NH₄Cl solution (5 mL). The reaction mixture was extracted with EtOAc (2 x 10 mL). The organic layers were combined, dried over Na₂SO₄, concentrated and purified by combi-flash (PE/DCM = 60%-0%) to afford product as a white solid (25.6 mg). *Note:* Deuterated ratio was 95%. **¹H NMR** (400 MHz, CDCl₃) δ 3.68-3.52 (m, 1H), 2.57-2.44 (m, 1H), 2.23-2.07 (m, 4H), 2.04-1.92 (m, 1H), 1.88-1.50 (m, 6H), 1.47-1.07 (m, 11H), 1.05-0.85(m, 2H), 0.80 (s, 3H), 0.73-0.63(m, 1H), 0.60 (s, 3H). **LCMS** Rt = 0.992 min in 2.0 min chromatography, 30-90AB_2MIN_E, MS ESI calcd. for C₂₁H₃₂DO [M+H-H₂O]⁺ 302, found 302. **HRMS** Acq Method: 0-95_1_4min_REF.m; Rt = 3.178 min in 4 min chromatography; DA Method: HRMS-ZN.m. MS ESI calcd. for C₂₁H₃₄DO₂ [M+H]⁺ 320.2694, found 320.2707.

**Step 2.** To a solution of **Compound 31** (45 mg, crude) in THF (2 mL) was added NaOD/D₂O (1 mL, 0.1 M) and CD₃OD (0.2 mL) and the mixture was stirred at 60°C for 72 h. The reaction was adjusted the pH to 7 with CH₃COOD (1 mL, 0.1 mL CH₃COOD in 1 mL D₂O) , extracted with EtOAc (3 x 5 mL), dried over Na₂SO₄, filtered and concentrated in vacuum to give a white solid, which was further triturated with hexane (2 x 0.5 mL) to give **Compound 32** as an off white solid (36.4 mg, 80%). *Note:* Deuterated ratio was 91%. **¹H NMR** (400 MHz, CDCl₃) δ 3.65-3.54 (m, 1H), 2.19-2.09 (m, 1H), 2.03-1.95 (m, 1H), 1.86-1.77 (m, 1H), 1.75-1.53 (m, 6H), 1.50-1.05 (m, 10H), 1.02-0.85 (m, 2H), 0.80 (s, 3H), 0.73-0.63 (m, 1H), 0.60 (s, 3H). **LCMS** Rt = 0.981 min in 2.0 min chromatography, 30-90AB_2MIN_E, MS ESI calcd. for C₂₁H₂₈D₅O [M+H-H₂O]⁺ 306, found 306. **HRMS** Acq Method: 0-95_1_4min_REF.m; Rt = 3.196 min in 4 min chromatography; DA Method: HRMS-ZN.m. MS ESI calcd. for C₂₁H₃₀D₅O₂ [M+H]⁺ 324.2945, found 324.2929.

### Example 21. Synthesis of Compounds 33 and 34. (Reference)

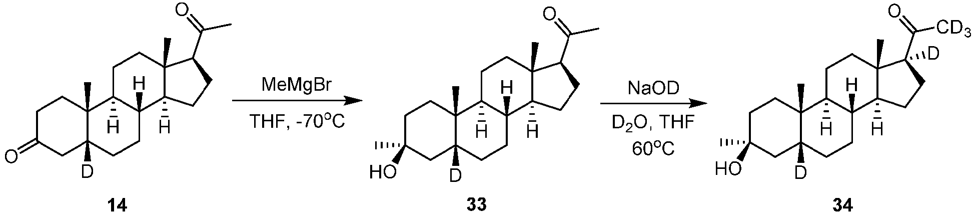

**Step 1.** To a solution of **Compound 14** (120 mg, 0.38 mmol) in THF (2 mL) was added MeMgBr (0.25 mL, 3 M in ether, 0.75 mmol) dropwise at -70°C. The mixture was stirred at - 70°C for 0.5 h. Then NH₄Cl (1 mL, sat. aq.) was added and the mixture was extracted with EtOAc (2 x 5 mL). The combined organic layer was separated, combined with another batch from 120 mg of **Compound 33,** dried over Na₂SO₄, concentrated in vacuum, purified by column chromatography on silica gel (PE/DCM=2:1 to DCM to DCM/acetone = 100:1) to give **Compound 33** (70 mg, 56%, deuterated ratio: 92%) as a white solid. **¹H NMR** (400 MHz, CDCl₃) δ 2.53 (t, *J* = 8.8 Hz, 1H), 2.21-2.13 (m, 1H), 2.11 (s, 3H), 2.08-1.98 (m, 1H), 1.95-1.78 (m, 2H), 1.72-1.12 (m, 20H), 1.11-1.00 (m, 1H), 0.96 (s, 3H), 0.60 (s, 3H). **LCMS** Rt = 1.069 min in 2.0 min chromatography, 30-90AB_2MIN_E, (ELSD), MS ESI calcd. for C₂₂H₃₄DO [M+H-H₂O]⁺ 316, found 316. **HRMS** MS ESI calcd. for C₂₂H₃₆DO₂ [M+H]⁺ 334.2851, found 334.2859.

**Step 2.** To a solution of **Compound 33** (48 mg) in THF (1 mL) was added NaOD/D₂O (1 mL, 0.2 M) and the mixture was stirred at 60°C for 16 h. To the mixture was added CH₃COOD/D₂O (0.5 M) till pH = 7 without monitored at 25°C. The mixture was extracted with EtOAc (2 x 3 mL). The combined organic layer was concentrated to give 48 mg of white solid. NMR showed there was partly 17-H undeuterated remained. The previous partly 17-H undeuterated mixture was recycled and dissolved in THF (1 mL) was added NaOD/D₂O (1 mL, 0.1 M) and CD₃OD (0.2 mL), the mixture was stirred at 60°C for 16 hrs. This reaction was worked-up with the same condition and checked by NMR. The partly 17-H undeuterated mixture was recycled for another three times and NMR showed all of the 17-H was deuterated completed. The crude was trituration form hexane (3 x 2 mL) to give **Compound 34** (20.6 mg, 42%) as white solid. *Note:* Deuterated ratio: 92%. **¹H NMR** (400 MHz, CDCl₃) δ 2.21-2.13 (m, 1H), 2.08-1.98 (m, 1H), 1.95-1.78 (m, 2H), 1.72-1.12 (m, 20H), 1.11-1.00 (m, 1H), 0.96 (s, 3H), 0.60 (s, 3H). **LCMS** Rt = 1.101 min in 2.0 min chromatography, 30-90AB_2MIN_E, MS ESI calcd. for C₂₂H₃₀D₅O [M+H-H₂O]⁺ 320, found 320. **HRMS** MS ESI calcd. for C₂₂H₃₂D₅O₂ [M+H]⁺ 338.3102, found 338.3103.

### Example 22. Synthesis of Compound 35. (Reference)

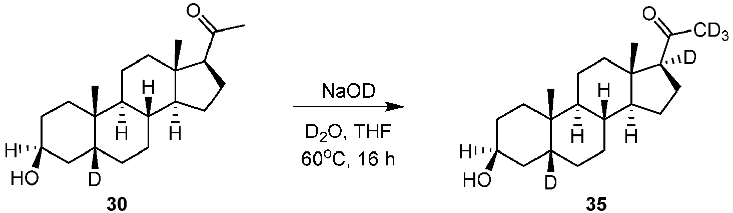

To a solution of **Compound 30** (50 mg, crude) in THF (2 mL) and D₂O (1 mL) was added NaOD (0.03 mL, 0.1 M in D₂O) and the mixture was stirred at 60°C for 18 h. The reaction was adjusted the pH to 7 with CH₃COOD (1 mL, 0.1 mL CH₃COOD in 1 mL D₂O), extracted with EtOAc (3 x 5 mL), dried over Na₂SO₄, filtered and concentrated in vacuum to give a white solid, which was further triturated with hexane (2 x 0.5 mL) to give **Compound 35** as a white solid (12.5 mg). *Note:* Deuterated ratio was 93%. **¹H NMR** (400 MHz, CDCl₃) δ 4.16-4.09 (m, 1H), 2.18-2.11 (m, 1H), 2.05-1.83 (m, 3H), 1.71-1.00 (m, 17H), 0.99-0.79 (m, 4H), 0.59 (s, 3H). **LCMS** Rt = 0.983 min in 2.0 min chromatography, 30-90AB_2MIN_E, MS ESI calcd. for C₂₁H₂₈D₅O [M+H-H₂O]⁺ 306, found 306. **HRMS** Acq Method: 0-95_1_4min_REF.m; Rt = 3.211 min in 4 min chromatography; DA Method: HRMS-ZN.m. MS ESI calcd. for C₂₁H₃₀D₅O₂ [M+H]⁺ 324.2945, found 324.2931.

### Example 23. Metabolic Stability

Metabolic stability was assessed at 10 µM with human, mouse, and rat liver microsomes (1, 0.25, and 0.5 mg/mL, respectively). Co-factor NADPH was supplied by a commercial regeneration solution for the non-cytosol reactions. Compound and protein were equilibrated at 37°C for 5 min prior to reaction initiation with co-factor. Time points were taken at 0, 5, 20, 35, and 65 min. Aliquots were added to isopropyl alcohol, mixed for 5 min, and incubated at room temperature for 2 hours for extraction. Following centrifugation, supernatants were analyzed by HPLC-MS/MS with methods specific to compounds tested, and response areas were normalized to the initial T0 time point responses to obtain percent of T0. The natural log of the percents of T0 were plotted against time-point min and the slope was used to calculate T ½ (LN(2)/-slope).

Microsomal clearance (Clint) and hepatic clearance (CLhep) are derived from t½ using the following scaling factors:

| Species | Microsomal Protein (mg/g Liver) | Liver Size (g/kg body weight) | Hepatic Blood Flow (mL/min/kg) |
|---|---|---|---|
| Human | 32.0 | 25.7 | 20.7 |
| Mouse | 45.0 | 87.5 | 90.0 |
| Rat | 61.0 | 40.0 | 55.2 |

**Table 1. Metabolic Stablity of Exemplary Compounds**

| **Compound** | **Mouse** | **Rat** | **Dog** | **Monkey** | **Human** |
|---|---|---|---|---|---|
| **Allopregnanolone** | 452.7 | 216.1 | 24.0 | 59.9 | 14.7 |
| **Ganaxolone** | 266.5 | 86.1 | 26.6 | 49.1 | 12.3 |
| 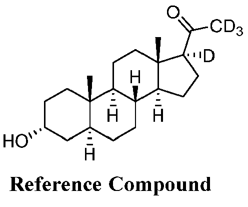 | 183.0 | 59.2 | - | - | 18.7 |
| **22*** | - | 30.5 | 21.5 | 84.7 | 7.7 |
| **23*** | - | 43.8 | 22.0 | 88.5 | 8.3 |
| **18*** | - | 41.7 | 18.7 | 59.6 | 9.7 |
| **2*** | 260.5 | 74.7 | 14.0 | 91.9 | 11.2 |
| **3*** | 266.4 | 61.4 | 15.9 | 106.9 | 12.3 |
| **1*** | - | 44.4 | 15.2 | 93.2 | 12.4 |
| **7** | 372.6 | 286.9 | - | - | 19.2 |
| **4** | 552.5 | 21.0 | - | - | 22.2 |
| **6** | 346.7 | 323.3 | - | - | 22.6 |
| **5** | 738.5 | 429.3 | - | - | 34.4 |

| | | | | | |
|---|---|---|---|---|---|
| * Reference compound. | | | | | |

## Claims

1. A deuterium-enriched compound or a pharmaceutically-acceptable salt thereof, for use in a method of treating a mood disorder, depression, post-partum depression, or anxiety disorder, in a subject, comprising administering to the subject an effective amount of the compound selected from the group consisting of: 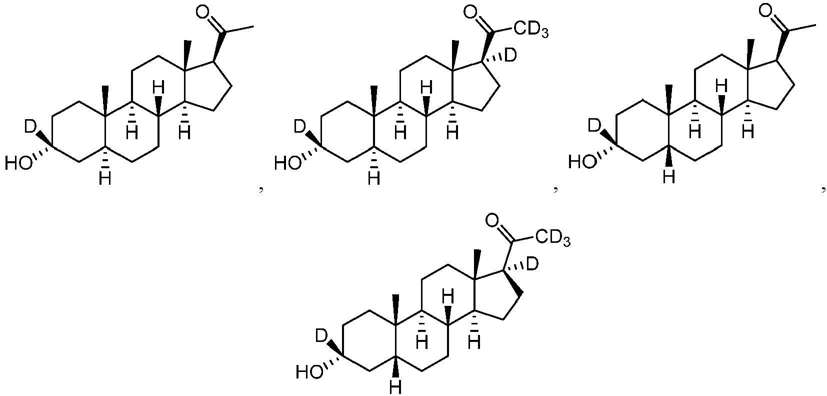 and
or a pharmaceutically-acceptable salt thereof.

2. The compound for use according to claim 1, wherein the compound is for oral, parenteral, or intravenous administration, or continuous intravenous infusion.

3. The compound for use according to claim 1, wherein the subject is selected from a mammal, a female, an adult; a human, or a human from 18 to 45 years of age.

4. The compound for use according to claim 1, wherein the subject is suffering from postpartum depression or severe postpartum depression; or wherein the subject has experienced a Major Depressive Episode in the postpartum period, wherein the period begins within the first 4 weeks following delivery of a baby.

5. A compound or a pharmaceutically-acceptable salt thereof, for use in a method of inducing sedation and/or anesthesia in a subject, comprising administering to the subject an effective amount of the compound selected from the group consisting of: 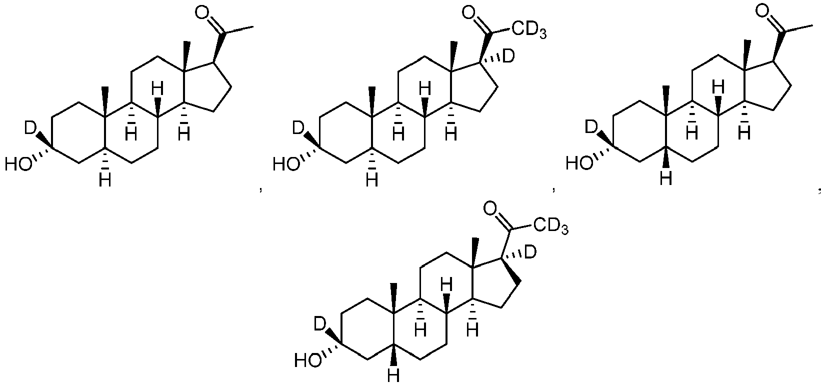 and
or a pharmaceutically-acceptable salt thereof.

6. A compound or a pharmaceutically-acceptable salt thereof, or a pharmaceutical composition of the compound or the pharmaceutically-acceptable salt thereof, for use as a medicament, the compound selected from the group consisting of: 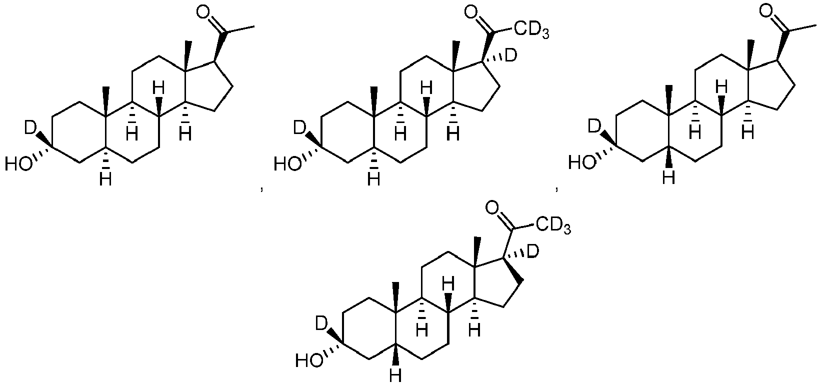 , and
or a pharmaceutically-acceptable salt thereof.

7. The compound for use according to claim 5 or claim 6, wherein the compound is for intravenous administration, or wherein the compound is for chronic administration.

8. A compound or a pharmaceutically-acceptable salt thereof, for use in a method for treating seizure in a subject, comprising administering to the subject an effective amount of the compound selected from the group consisting of: 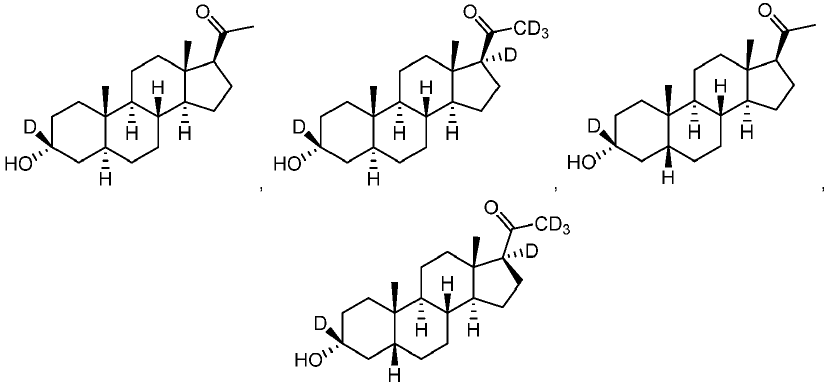 and
or a pharmaceutically-acceptable salt thereof.

9. A compound or a pharmaceutically-acceptable salt thereof, for use in a method for treating epilepsy in a subject, the method comprising administering to the subject an effective amount of the compound selected from the group consisting of: 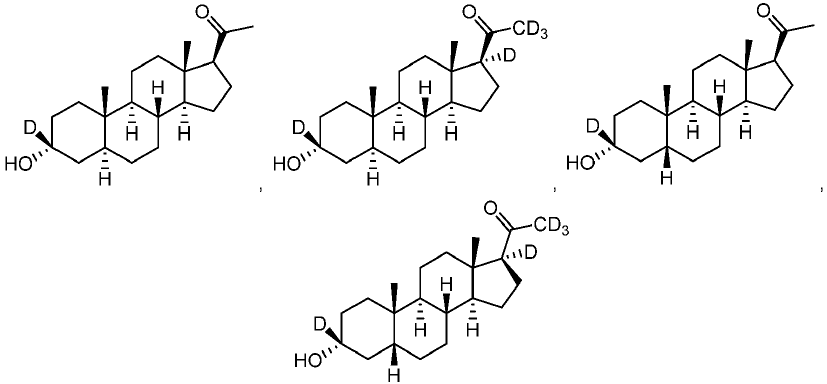 and
or a pharmaceutically-acceptable salt thereof.

10. A compound or a pharmaceutically-acceptable salt thereof, for use in a method for treating status epilepticus (SE), convulsive status epilepticus, early status epilepticus, established status epilepticus, refractory status epilepticus, super-refractory status epilepticus, non-convulsive status epilepticus, generalized status epilepticus, or complex partial status epilepticus, in a subject, the method comprising administering to the subject an effective amount of the compound selected from the group consisting of: 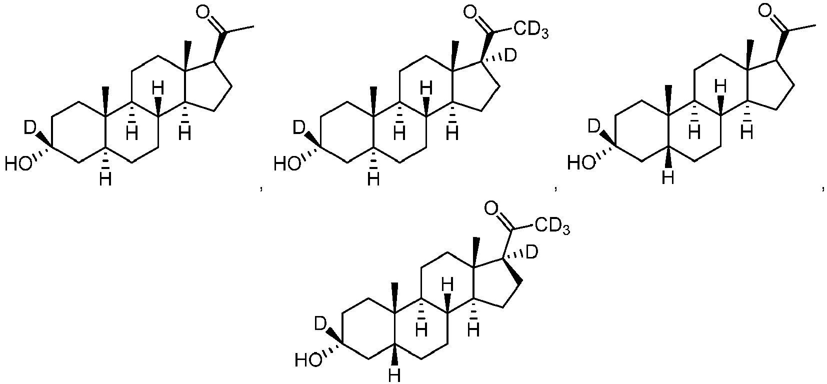 and
or a pharmaceutically-acceptable salt thereof.

11. A compound or a pharmaceutically-acceptable salt thereof, for use in a method of treating a human subject suffering from tremor, or essential tremor, the method comprising administering a therapeutically effective amount of the compound selected from the group consisting of: 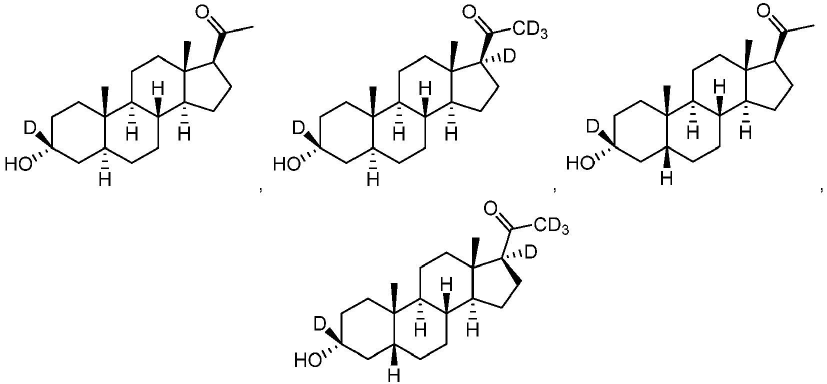 and
or a pharmaceutically-acceptable salt thereof.

12. A deuterium-enriched compound or a pharmaceutically-acceptable salt thereof, for use in a method for treating a CNS-related disorder in a subject in need thereof, comprising administering to the subject an effective amount of the deuterium-enriched compound selected from the group consisting of: 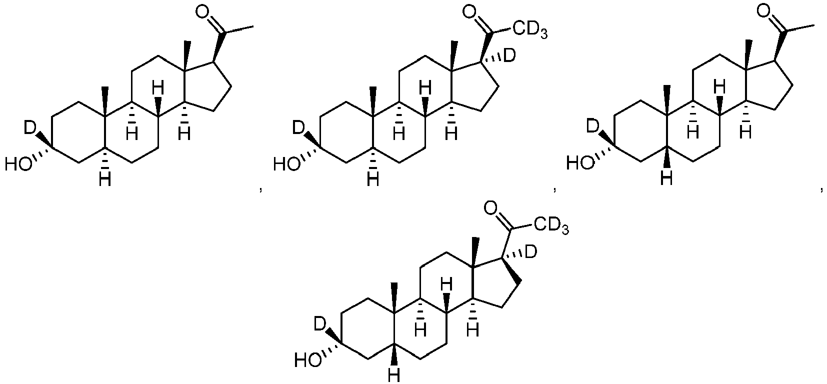 and
or a pharmaceutically-acceptable salt thereof,
wherein the CNS-related disorder is a sleep disorder, a mood disorder, a schizophrenia spectrum disorder, a convulsive disorder, a disorder of memory and/or cognition, a movement disorder, tremor, essential tremor, a personality disorder, autism spectrum disorder, pain, traumatic brain injury, a vascular disease, a substance abuse disorder and/or withdrawal syndrome, or tinnitus.

13. The compound for use according to claim 12, wherein the subject is a subject with Rett syndrome, Fragile X syndrome, or Angelman syndrome.

14. The compound for use according to any one of claims 5 to 13, wherein the subject is a mammal or a human.

15. The compound for use according to any one of claims 5 to 12, wherein the administering is performed parenterally, intravenously, intramuscularly, orally, or performed chronically.

16. The compound for use according to any one of claims 5 to 12, wherein the compound is administered in combination with another therapeutic agent.

17. A pharmaceutical composition comprising a deuterium-enriched compound selected from the group consisting of: 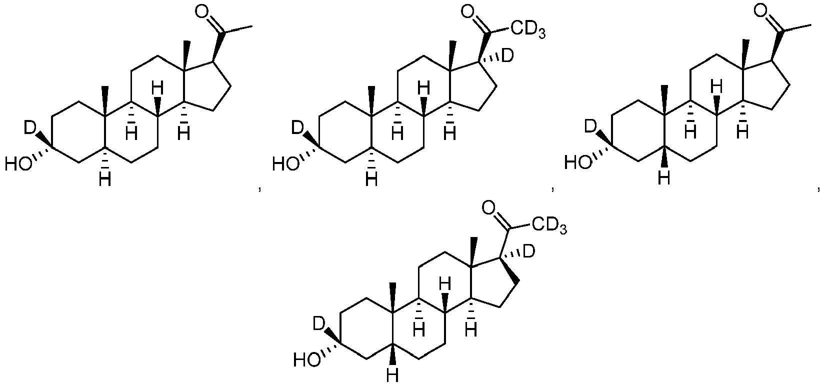 and
or a pharmaceutically-acceptable salt thereof,
the composition optionally further comprising a pharmaceutically-acceptable excipient.

18. A deuterium-enriched compound selected from the group consisting of: 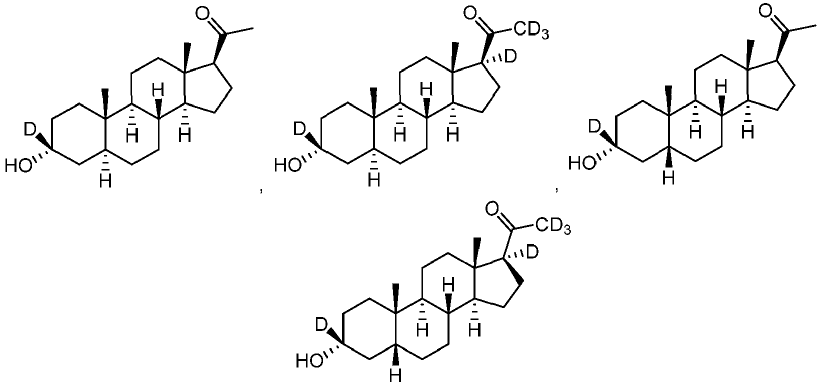 , and
or a pharmaceutically-acceptable salt thereof.

## Patentansprüche

1. Deuteriumangereicherte Verbindung oder pharmazeutisch unbedenkliches Salz davon zur Verwendung bei einem Verfahren zur Behandlung einer affektiven Störung, Depression, postpartalen Depression oder Angststörung bei einem Individuum, bei dem man dem Individuum eine wirksame Menge der aus der Gruppe bestehend aus 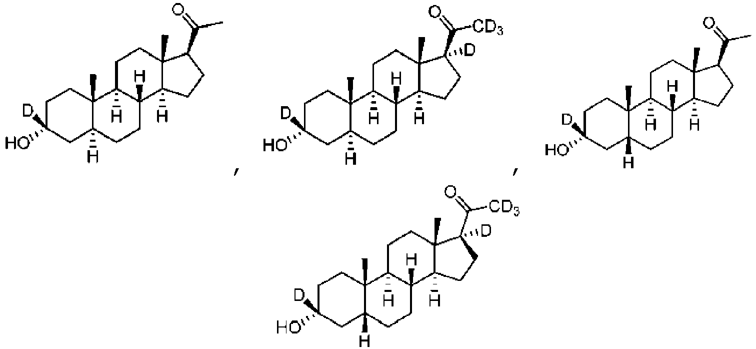 und
ausgewählten Verbindung oder eines pharmazeutisch unbedenklichen Salzes davon verabreicht.

2. Verbindung zur Verwendung nach Anspruch 1, wobei die Verbindung für die orale, parenterale oder intravenöse Verabreichung oder kontinuierliche intravenöse Infusion bestimmt ist.

3. Verbindung zur Verwendung nach Anspruch 1, wobei es sich bei dem Individuum um ein Säugetier, ein Weibchen, ein Adulttier; einen Menschen oder einen Menschen im Alter von 18 bis 45 Jahren handelt.

4. Verbindung zur Verwendung nach Anspruch 1, wobei das Individuum an postpartaler Depression oder schwerer postpartaler Depression leidet oder wobei das Individuum in der Nachgeburtsperiode eine schwere depressive Episode durchlebt hat, wobei die Periode innerhalb der ersten 4 Wochen nach der Geburt eines Babys beginnt.

5. Verbindung oder pharmazeutisch unbedenkliches Salz davon zur Verwendung bei einem Verfahren zur Induzierung von Sedierung und/oder Anästhesie bei einem Individuum, bei dem man dem Individuum eine wirksame Menge der aus der Gruppe bestehend aus 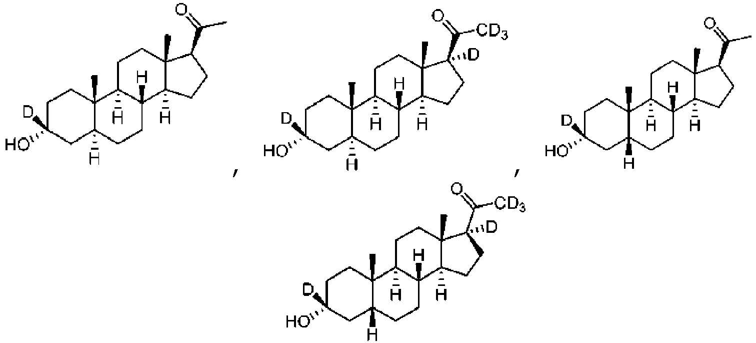 und
ausgewählten Verbindung oder eines pharmazeutisch unbedenklichen Salzes davon verabreicht.

6. Verbindung oder pharmazeutisch unbedenkliches Salz davon oder pharmazeutische Zusammensetzung der Verbindung oder des pharmazeutisch unbedenklichen Salzes davon zur Verwendung als Medikament, wobei die Verbindung aus der Gruppe bestehend aus 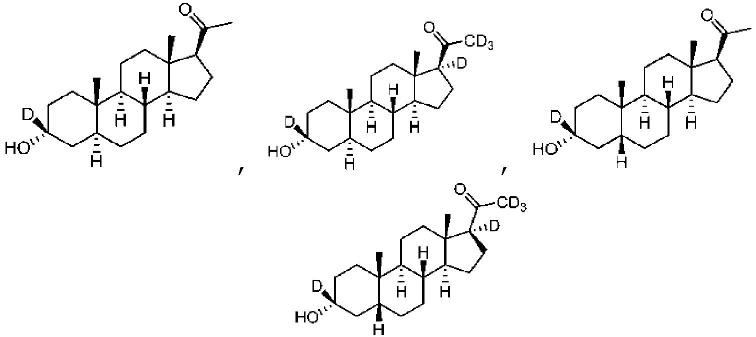 und
ausgewählt ist oder ein pharmazeutisch unbedenkliches Salzes davon.

7. Verbindung zur Verwendung nach Anspruch 5 oder Anspruch 6, wobei die Verbindung für die intravenöse Verabreichung bestimmt ist oder wobei die Verbindung für die chronische Verabreichung bestimmt ist.

8. Verbindung oder pharmazeutisch unbedenkliches Salz davon zur Verwendung bei einem Verfahren zur Behandlung von Anfällen bei einem Individuum, bei dem man dem Individuum eine wirksame Menge der aus der Gruppe bestehend aus 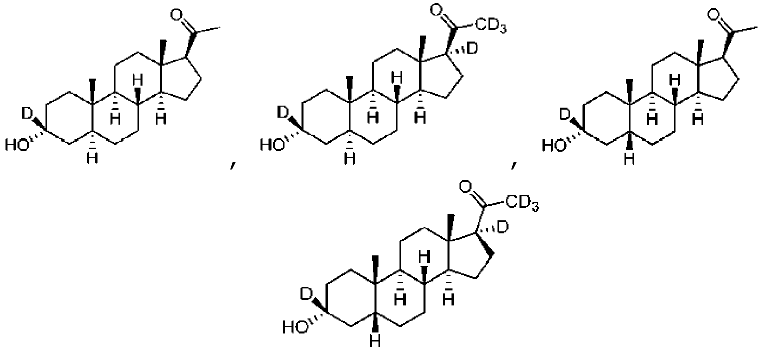 und
ausgewählten Verbindung oder eines pharmazeutisch unbedenklichen Salzes davon verabreicht.

9. Verbindung oder pharmazeutisch unbedenkliches Salz davon zur Verwendung bei einem Verfahren zur Behandlung von Epilepsie bei einem Individuum, bei dem man dem Individuum eine wirksame Menge der aus der Gruppe bestehend aus 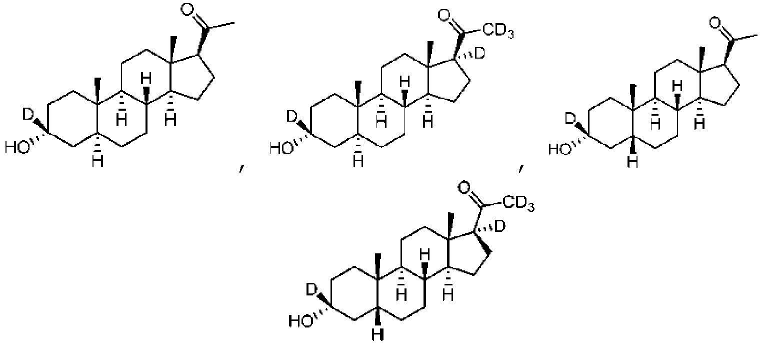 und
ausgewählten Verbindung oder eines pharmazeutisch unbedenklichen Salzes davon verabreicht.

10. Verbindung oder pharmazeutisch unbedenkliches Salz davon zur Verwendung bei einem Verfahren zur Behandlung von Status epilepticus (SE), konvulsivem Status epilepticus, frühem Status epilepticus, etabliertem Status epilepticus, refraktärem Status epilepticus, superrefraktärem Status epilepticus, nichtkonvulsivem Status epilepticus, generalisiertem Status epilepticus oder komplexem partiellem Status epilepticus bei einem Individuum, bei dem man dem Individuum eine wirksame Menge der aus der Gruppe bestehend aus 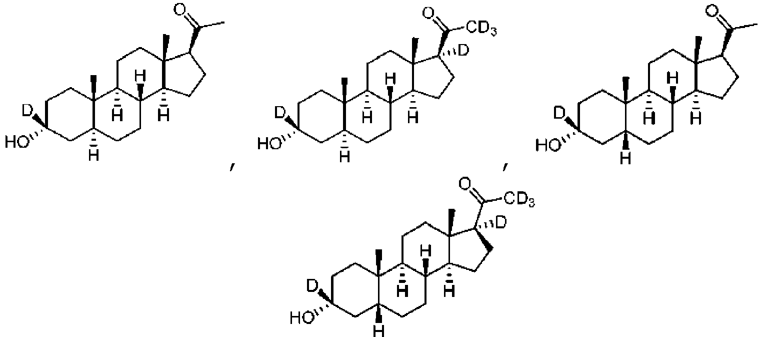 und
ausgewählten Verbindung oder eines pharmazeutisch unbedenklichen Salzes davon verabreicht.

11. Verbindung oder pharmazeutisch unbedenkliches Salz davon zur Verwendung bei einem Verfahren zur Behandlung eines menschlichen Individuums, das an Tremor oder essentiellem Tremor leidet, bei dem man eine therapeutisch wirksame Menge der aus der Gruppe bestehend aus 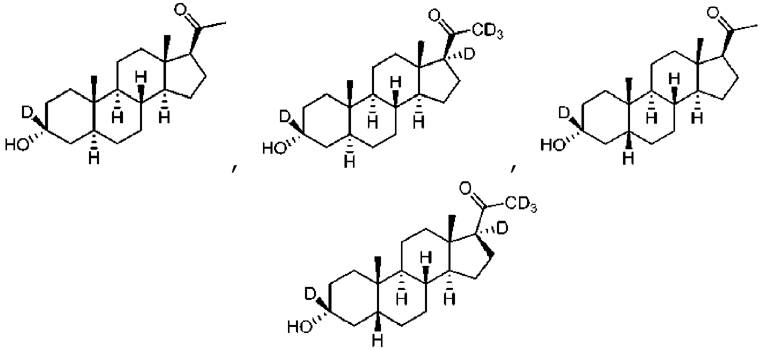 und
ausgewählten Verbindung oder eines pharmazeutisch unbedenklichen Salzes davon verabreicht.

12. Deuteriumangereicherte Verbindung oder pharmazeutisch unbedenkliches Salz davon zur Verwendung bei einem Verfahren zur Behandlung einer mit dem ZNS in Zusammenhang stehenden Störung bei einem Individuum, bei dem diesbezüglicher Bedarf besteht, bei dem man dem Individuum eine wirksame Menge der aus der Gruppe bestehend aus 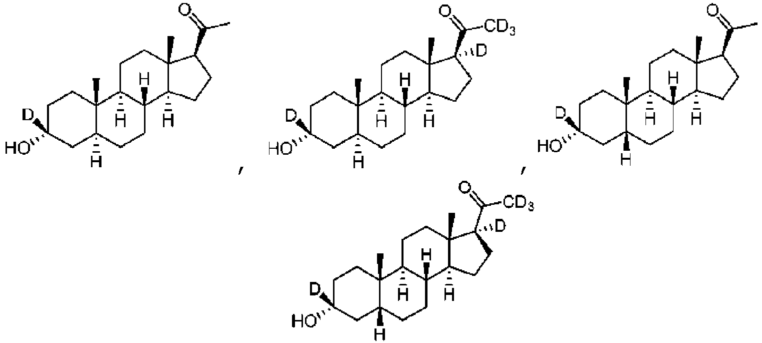 und
ausgewählten deuteriumangereicherten Verbindung oder eines pharmazeutisch unbedenklichen Salzes davon verabreicht,
wobei es sich bei der mit dem ZNS in Zusammenhang stehenden Störung um eine Schlafstörung, eine affektive Psychose, eine Störung des Schizophrenie-Spektrums, eine konvulsive Störung, eine Gedächtnis- und/oder Kognitionsstörung, eine Bewegungsstörung, Tremor, essentiellen Tremor, eine Persönlichkeitsstörung, eine Störung des Autismus-Spektrums, Schmerzen, traumatische Hirnverletzung, eine Gefäßerkrankung, eine Substanzmissbrauchsstörung und/oder ein Entzugssyndrom oder Tinnitus handelt.

13. Verbindung zur Verwendung nach Anspruch 12, wobei es sich bei dem Individuum um ein Individuum mit Rett-Syndrom, Fragile-X-Syndrom oder Angelman-Syndrom handelt.

14. Verbindung zur Verwendung nach einem der Ansprüche 5 bis 13, wobei es sich bei dem Individuum um ein Säugetier oder einen Menschen handelt.

15. Verbindung zur Verwendung nach einem der Ansprüche 5 bis 12, wobei die Verabreichung parenteral, intravenös, intramuskulär, oral oder chronisch durchgeführt wird.

16. Verbindung zur Verwendung nach einem der Ansprüche 5 bis 12, wobei die Verbindung in Kombination mit einem anderen therapeutischen Mittel verabreicht wird.

17. Pharmazeutische Zusammensetzung, umfassend eine aus der Gruppe bestehend aus 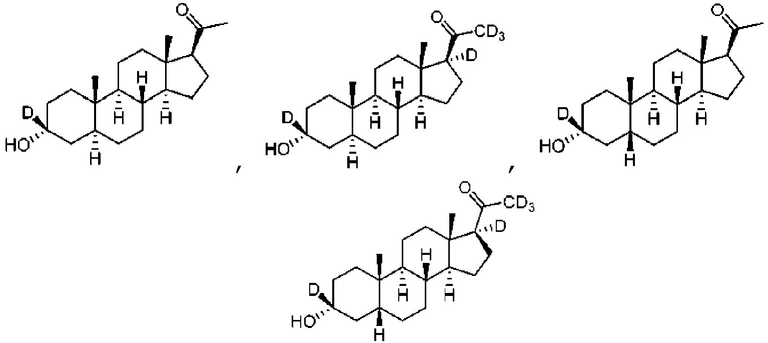 und
ausgewählte deuteriumangereicherte Verbindung oder ein pharmazeutisch unbedenkliches Salz davon,
wobei die Zusammensetzung gegebenenfalls ferner einen pharmazeutisch unbedenklichen Hilfsstoff umfasst.

18. Deuteriumangereicherte Verbindung, ausgewählt aus der Gruppe bestehend aus 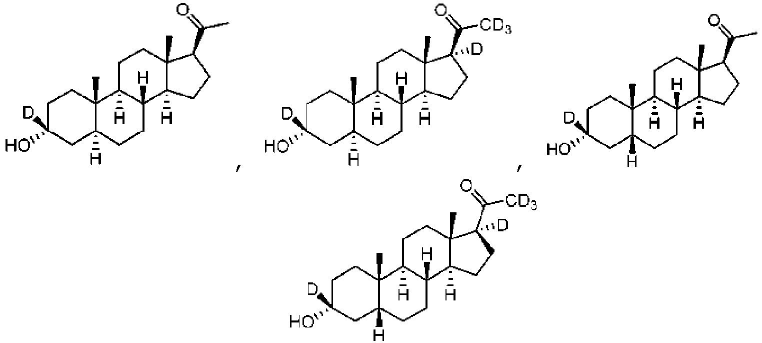 und
oder ein pharmazeutisch unbedenkliches Salz davon.

## Revendications

1. Composé enrichi en deutérium ou sel pharmaceutiquement acceptable de celui-ci, pour utilisation dans un procédé de traitement d'un trouble de l'humeur, de la dépression, de la dépression post-partum ou d'un trouble anxieux, chez un sujet, comprenant l'administration au sujet d'une quantité efficace du composé choisi dans le groupe constitué de : 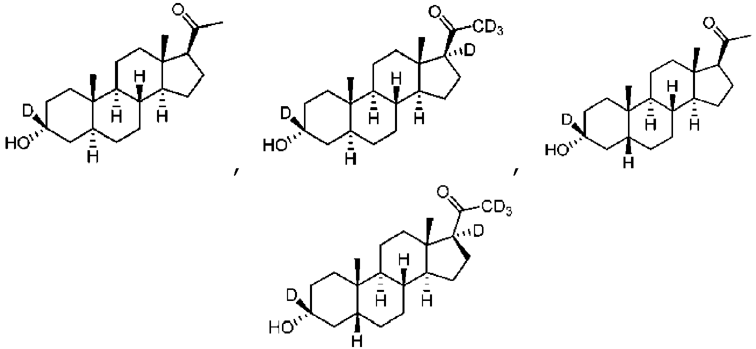 et
ou un sel pharmaceutiquement acceptable de celui-ci.

2. Composé pour utilisation selon la revendication 1, dans lequel le composé est destiné à une administration orale, parentérale, ou intraveineuse, ou une perfusion intraveineuse continue.

3. Composé pour utilisation selon la revendication 1, le sujet étant choisi parmi un mammifère, une femelle, un adulte ; un humain, ou un humain âgé de 18 à 45 ans.

4. Composé pour utilisation selon la revendication 1, le sujet souffrant de dépression post-partum ou de dépression post-partum sévère ; ou le sujet ayant subi un épisode dépressif majeur dans la période post-partum, la période commençant dans les 4 premières semaines suivant l'accouchement d'un enfant.

5. Composé ou sel pharmaceutiquement acceptable de celui-ci, pour utilisation dans un procédé d'induction d'une sédation et/ou d'une anesthésie chez un sujet, comprenant l'administration au sujet d'une quantité efficace du composé choisi dans le groupe constitué de : 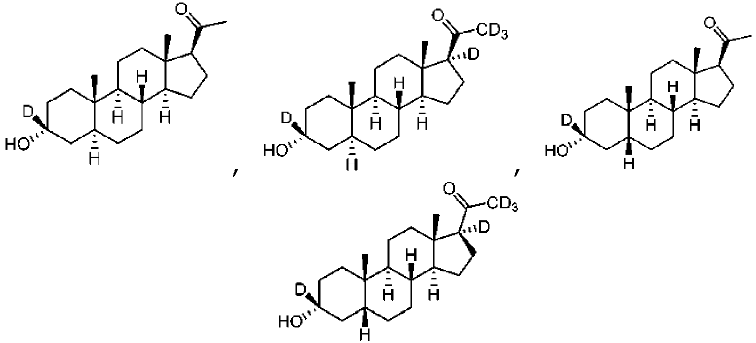 et
ou un sel pharmaceutiquement acceptable de celui-ci.

6. Composé ou sel pharmaceutiquement acceptable de celui-ci, ou composition pharmaceutique du composé ou du sel pharmaceutiquement acceptable de celui-ci, pour utilisation en tant que médicament, le composé étant choisi dans le groupe constitué de : 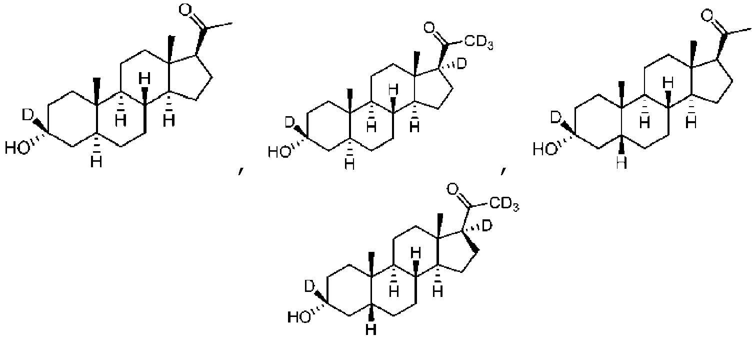 et
ou un sel pharmaceutiquement acceptable de celui-ci.

7. Composé pour utilisation selon la revendication 5 ou la revendication 6, le composé étant destiné à une administration intraveineuse, ou le composé étant destiné à une administration chronique.

8. Composé ou sel pharmaceutiquement acceptable de celui-ci, pour utilisation dans un procédé de traitement des crises d'épilepsie chez un sujet, comprenant l'administration au sujet d'une quantité efficace du composé choisi dans le groupe constitué de : 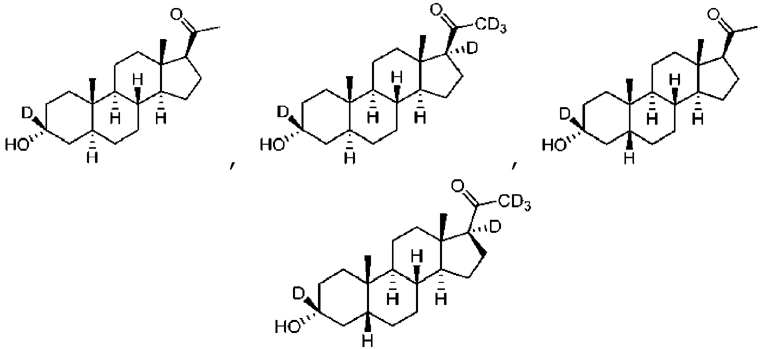 et
ou un sel pharmaceutiquement acceptable de celui-ci.

9. Composé ou sel pharmaceutiquement acceptable de celui-ci, pour utilisation dans un procédé de traitement de l'épilepsie chez un sujet, le procédé comprenant l'administration au sujet d'une quantité efficace du composé choisi dans le groupe constitué de : 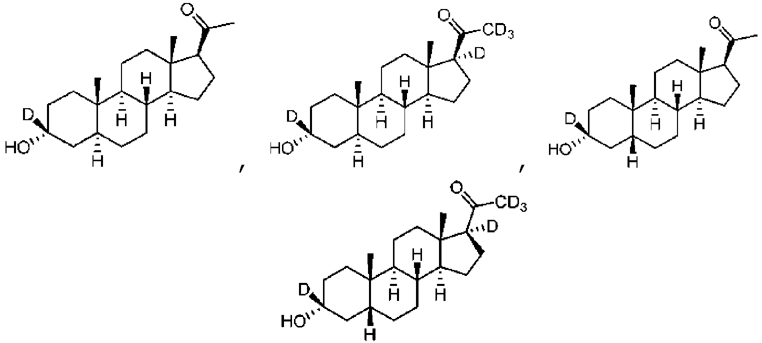 et
ou un sel pharmaceutiquement acceptable de celui-ci.

10. Composé ou sel pharmaceutiquement acceptable de celui-ci, pour utilisation dans un procédé de traitement d'un état de mal épileptique (SE), un état de mal épileptique convulsif, un état de mal épileptique précoce, un état de mal épileptique établi, un état de mal épileptique réfractaire, un état de mal épileptique hyperréfractaire, un état de mal épileptique non convulsif, un état de mal épileptique généralisé ou un état de mal épileptique partiel complexe, chez un sujet, le procédé comprenant l'administration au sujet d'une quantité efficace du composé choisi dans le groupe constitué de : 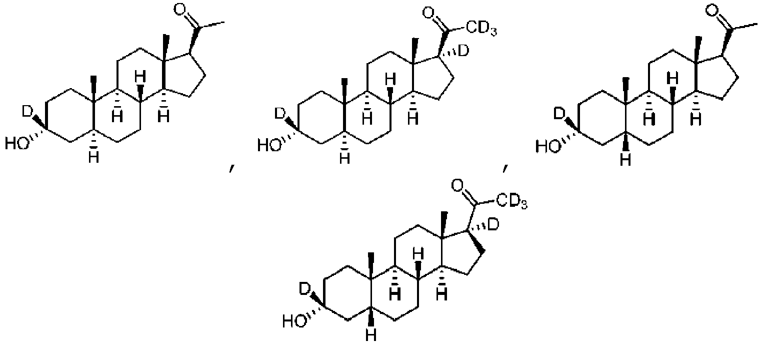 et
ou un sel pharmaceutiquement acceptable de celui-ci.

11. Composé ou sel pharmaceutiquement acceptable de celui-ci, pour utilisation dans un procédé de traitement d'un sujet humain souffrant de tremblement ou de tremblement essentiel, le procédé comprenant l'administration d'une quantité thérapeutiquement efficace du composé choisi dans le groupe constitué de : 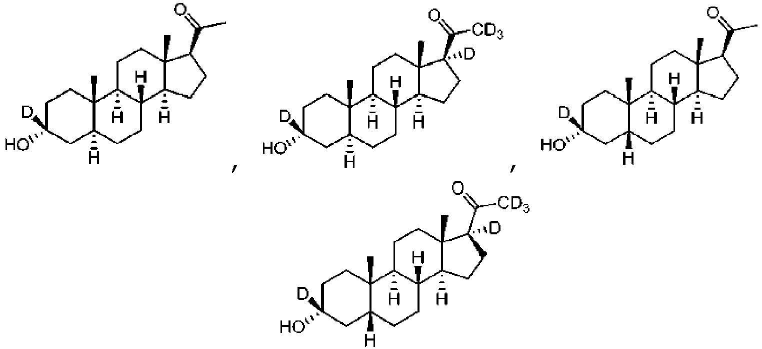 et
ou un sel pharmaceutiquement acceptable de celui-ci.

12. Composé enrichi en deutérium ou sel pharmaceutiquement acceptable de celui-ci, pour utilisation dans un procédé de traitement d'un trouble lié au SNC chez un sujet en ayant besoin, comprenant l'administration au sujet d'une quantité efficace du composé enrichi en deutérium choisi dans le groupe constitué de : 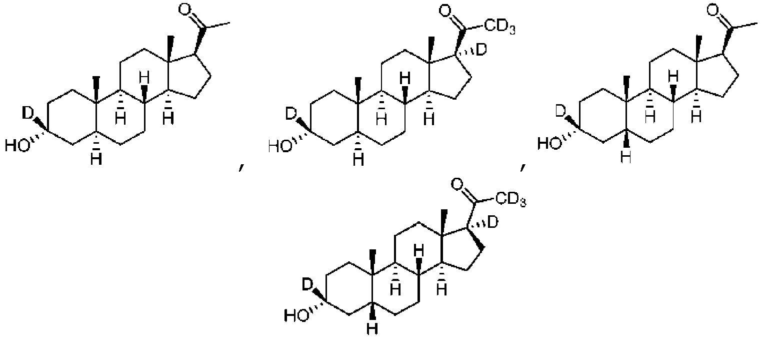 et
ou un sel pharmaceutiquement acceptable de celui-ci,
le trouble lié au SNC étant un trouble du sommeil, un trouble de l'humeur, un trouble du spectre de la schizophrénie, un trouble convulsif, un trouble de la mémoire et/ou cognitif, un trouble du mouvement, un tremblement, un tremblement essentiel, un trouble de la personnalité, un trouble du spectre de l'autisme, une douleur, une lésion cérébrale atraumatique, une maladie vasculaire, un trouble de toxicomanie et/ou un syndrome de sevrage ou des acouphènes.

13. Composé pour utilisation selon la revendication 12, le sujet étant un sujet souffrant du syndrome de Rett, du syndrome de l'X fragile ou du syndrome d'Angelman.

14. Composé pour utilisation selon l'une quelconque des revendications 5 à 13, le sujet étant un mammifère ou un humain.

15. Composé pour utilisation selon l'une quelconque des revendications 5 à 12, l'administration étant effectuée par voie parentérale, par voie intraveineuse, par voie intramusculaire, par voie orale, ou effectuée de façon chronique.

16. Composé pour utilisation selon l'une quelconque des revendications 5 à 12, le composé étant administré en combinaison avec un autre agent thérapeutique.

17. Composition pharmaceutique comprenant un composé enrichi en deutérium choisi dans le groupe constitué de : 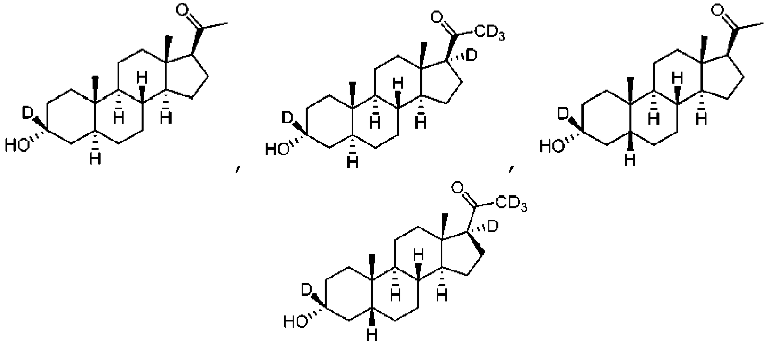 et
ou un sel pharmaceutiquement acceptable de celui-ci,
la composition comprenant facultativement en outre un excipient pharmaceutiquement acceptable.

18. Composé enrichi en deutérium choisi dans le groupe constitué de : 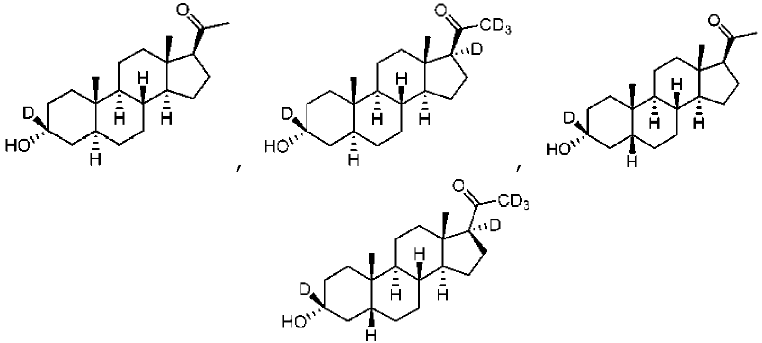 et
ou un sel pharmaceutiquement acceptable de celui-ci.
